# FASCICULE DE BREVET EUROPEEN

(11) **EP 4 032 985 B1**
(45) Date de publication et mention de la délivrance du brevet: **13.03.2024**
(21) Numéro de dépôt: 21215711.9
(22) Date de dépôt: 31.10.2014
(51) Int. Cl.: C12Q 1/6895

(54) **MICROPEPTIDES ET LEUR UTILISATION POUR MODULER L'EXPRESSION DE GENES**
MIKROPEPTIDE UND IHRE VERWENDUNG ZUR MODULATION VON GENEXPRESSIONEN
MICROPEPTIDES AND USE THEREOF FOR MODULATING GENE EXPRESSION

(30) Priorité: 31.10.2013 FR 1360727; 03.06.2014 FR 1455044
(43) Date de publication de la demande: 27.07.2022
(62) Demande divisionnaire de: 19152673.0
(73) Titulaire: Université Paul Sabatier Toulouse III, 31062 Toulouse Cedex 9 (FR); Centre National de la Recherche Scientifique, 75794 Paris (FR)
(72) Inventeur: COMBIER, Jean-Philippe, 31320 Castanet Tolosan (FR); LAURESSERGUES, Dominique, 31000 Toulouse (FR); BECARD, Guillaume, 31450 Odars (FR); PAYRE, François, 31450 Pompertuzat (FR); PLAZA, Serge, 31520 Ramonville Saint Agne (FR); CAVAILLE, Jérôme, 31520 Ramonville Saint Agne (FR)
(74) Mandataire: Grosset-Fournier, Chantal Catherine

(56) Documents cités:
- AXEL BRENNICKE: "Micropeptide als Gen-Regulatoren", BIOL. UNSERER ZEIT, vol. 40, 1 juin 2010 (2010-06-01), page 371, XP055168530, DOI: 10.1002/biuz.201090090
- JEROEN CRAPPÉ ET AL: "Micro peptides as a new class of bio-active peptides in Eukaryotes", BENELUX BIOINFORMATICS CONFERENCE: PROCEEDINGS OF BBC11, 9 décembre 2011 (2011-12-09), page 77, XP055168531,
- MÁXIMO IBO GALINDO ET AL: "Peptides Encoded by Short ORFs Control Development and Define a New Eukaryotic Gene Family", CURRENT SCIENCE, vol. 83, no. 5, 1 janvier 2007 (2007-01-01), page 426, XP055168533, ISSN: 0011-3891, DOI: 10.1371/journal.pbio.0050106

## Description

La présente invention concerne des micropeptides (peptides codés par des microARNs ou « miPEPs ») et leur utilisation pour moduler l'expression de gènes.

Les microARNs (miRs) sont des petits ARNs non codants, d'environ 21 nucléotides après maturation, qui contrôlent l'expression de gènes cibles au niveau post-transcriptionnel, en dégradant l'ARNm cible ou en inhibant sa traduction. Les miRs sont rencontrés chez les plantes et les animaux.

Les gènes cibles sont souvent des gènes clés de processus développementaux. Ils codent par exemple des facteurs de transcription ou des protéines du protéasome.

La régulation de l'expression des miRs est très peu connue, mais on sait notamment que celle-ci fait intervenir, à l'instar de la plupart des gènes codants, une ARN polymerase II : cette enzyme produit un transcrit primaire, appelé « *pri-miR* », qui est ensuite maturé par un complexe protéique contenant notamment les enzymes type Dicer. Cette maturation conduit tout d'abord à la formation d'un précurseur de miR appelé « *pre-miR* », ayant une structure secondaire en forme tige-boucle contenant le *miR* et sa séquence *miR** complémentaire. Puis le précurseur est maturé, ce qui conduit à la formation d'un ARN double brin plus court contenant le miR et le miR*. Le miR est ensuite pris en charge par le complexe RISC qui clive l'ARNm du gène cible ou bien inhibe sa traduction.

Par ailleurs, il a été montré que la présence d'introns dans le transcrit primaire du microARN augmente l'expression du microARN mature (Schwab et al., EMBO Rep., 14(7) :615-21, 2013). Cependant, du fait de difficultés expérimentales, les transcrits primaires de microARNs, ou pri-miRs, sont très peu étudiés.

Environ 50% des gènes eucaryotes possèdent, au sein de leur région 5'UTR (5' UnTranslated Région) en amont de la séquence codante, de petits cadres ouverts de lecture. Ces petits cadres ouverts de lecture (ou « uORFs » pour upstream ORFs) peuvent jouer un rôle de régulateur de la traduction, principalement en *cis,* en modulant la fixation et la vitesse des ribosomes sur l'ARNm, mais également en *trans* selon un mécanisme encore inconnu, par l'intermédiaire de peptides codés par lesdits uORFs (Combier et al., Gene Dev, 22 :1549-1559, 2008). Par définition, les uORFS sont présents en amont de gènes codants. Récemment, il a également été découvert de petits ORFs dans de longs ARN non codants intergéniques (lincRNAs) dont la fonction putative, si elle existe, n'est pas connue (Ingolia et al., Cell, 147(4) :789-802, 2011 ; Guttman & Rinn, Nature, 482(7385) :339-46, 2012). Toutefois, aucun exemple n'a encore été rapporté concernant l'existence d'ORFs codant des peptides au sein de microARNs non codants. Jusqu'à présent, les microARNs, et par extension leur transcrit primaire, ont toujours été considérés, de par leur mode d'action particulier, comme des ARNs régulateurs non codants ne produisant aucun peptide.

Brennincke (2010), Crappé et al. (2011) et Galindo et al. (2007) ont décrit l'identification de micropeptides, lesquels ont une activité régulatrice de la fonction de certains gènes. Ces micropeptides sont codés par des microORFs.

L'invention est définie dans les revendications.

L'invention concerne ainsi un procédé de détection et d'identification d'un micropeptide (miPEP) codé par une séquence nucléotidique contenue dans la séquence du transcrit primaire d'un microARN, comprenant :
- a) une étape de détection d'un cadre ouvert de lecture de 12 à 303 nucléotides contenu dans la séquence du transcrit primaire dudit microARN, puis
- b) une étape de comparaison entre :
- le phénotype d'une plante exprimant ledit miR, en présence d'un peptide codé par une séquence nucléotidique identique ou dégénérée par rapport à celle dudit cadre ouvert de lecture, ledit peptide étant présent dans la plante indépendamment de la transcription du transcrit primaire dudit miR, et
- le phénotype d'une même plante exprimant ledit miR en l'absence dudit peptide, dans lequel, un changement de phénotype de la plante en présence dudit peptide par rapport au phénotype de la plante en absence dudit peptide indique l'existence d'un miPEP codé par ledit cadre ouvert de lecture.

Dans une première étape, le procédé de détection et d'identification d'un micropeptide consiste donc à détecter sur le transcrit primaire d'un microARN l'existence d'un cadre ouvert de lecture codant potentiellement un peptide.

La deuxième étape permet, quant à elle, de caractériser ledit peptide, c'est-à-dire de déterminer si ledit peptide correspond a un peptide réellement produit dans la cellule, en recherchant un effet dudit peptide sur l'accumulation dudit microARN.

Pour mettre en évidence un effet du peptide sur l'accumulation du microARN, une quantité importante de peptide est introduite dans une première cellule exprimant ledit microARN. L'accumulation du microARN dans cette première cellule est ensuite mesurée et comparée avec l'accumulation du microARN dans une deuxième cellule identique à la première, mais ne contenant pas ledit peptide.

L'observation d'une variation des quantités de microARN entre les cellules en présence et en absence du peptide indique ainsi (i) qu'il existe un peptide codé sur le transcrit primaire dudit microARN, (ii) que la séquence de ce peptide est codée par le cadre ouvert de lecture identifié sur le transcrit primaire dudit microARN, et (iii) que ledit peptide agit sur l'accumulation dudit microARN.

L'invention repose donc sur la double observation inattendue faite par les Inventeurs que d'une part, il existe des cadres ouverts de lecture susceptibles de coder des micropeptides présents sur les transcrits primaires de microARNs, et d'autre part que lesdits micropeptides sont capables de moduler l'accumulation desdits microARNs.

Dans l'invention, les termes « *microARN », « microARN non codant »* et *« miR »* sont équivalents et peuvent être utilisés l'un pour l'autre. Ils définissent des petites molécules d'ARN d'environ 21 nucléotides, qui ne sont pas traduites et ne conduisent pas à un peptide ou une protéine.

Cependant, sous cette forme mature, les microARNs assurent une fonction de régulation de certains gènes *via* des mécanismes post-transcriptionnels, comme par exemple par l'intermédiaire du complexe RISC.

Le transcrit primaire du microARN ou *« pri-miR »* correspond lui à la molécule d'ARN directement obtenue à partir de la transcription de la molécule d'ADN. Généralement, ce transcrit primaire subit une ou plusieurs modifications post-transcriptionnelles, qui entraînent par exemple une structure particulière de l'ARN ou un clivage de certaines parties de l'ARN par des phénomènes d'épissage, et qui conduisent à la forme précurseur du microARN ou *« pre-miR »,* puis à la forme mature du microARN ou *« miR ».*

Les termes *« micropeptides »* et *« miPEPs »* (*microRNA encoded PEPtides)* sont équivalents et peuvent être utilisés l'un pour l'autre. Ils définissent un peptide qui est codé par un cadre ouvert de lecture présent sur le transcrit primaire d'un microARN, et qui est capable de moduler l'accumulation dudit microARN. Les micropeptides au sens de la présente invention ne doivent être entendus comme étant nécessairement des peptides de petite taille, dans la mesure où « micro » ne correspond pas à la taille du peptide.

Selon l'invention, un miPEP peut être également assimilé à un modulateur de transcription, et notamment un activateur transcriptionnel. Un tel modulateur de transcription peut agir au niveau de la transcription pour moduler l'accumulation du pri-miR, du pre-miR et du miR. Compte tenu de la dégénérescence du code génétique, un même micropeptide peut être codé par plusieurs séquences nucléotidiques. De telles séquences nucléotidiques, différentes entre elles d'au moins un nucléotide mais codant un même peptide, sont appelées « *séquences dégénérées ».*

Les termes « *cadre ouvert de lecture »* ou « *ORF »* (*open reading frame*) sont équivalents et peuvent être utilisés l'un pour l'autre. Ils correspondent à une séquence de nucléotides dans une molécule d'ADN ou d'ARN pouvant potentiellement coder un peptide ou une protéine : ledit cadre ouvert de lecture débute par un codon start (le codon start codant généralement une méthionine), suivi d'une série de codons (chaque codon codant un acide aminé), et se termine par un codon stop (le codon stop n'étant pas traduit).

Dans l'invention, les ORFs peuvent être dénommés de manière spécifique *« miORFs »* lorsque ceux-ci sont présents sur les transcrits primaires de microARN.

Les miORFs tels que définis dans l'invention particulier peuvent avoir une taille de 12 à 303 nucléotides et coder des peptides de 3 à 100 acides aminés.

En particulier, les miORFs tels que définis dans l'invention peuvent avoir une taille de 15 à 303 nucléotides. Un acide aminé étant codé par un codon de 3 nucléotides, les miORFs de 15 à 303 nucléotides codent des miPEPS de 4 à 100 acides aminés.

En particulier, les miORFs ont une taille de :
15, 18, 21, 24, 27, 30, 33, 36, 39, 42, 45, 47, 51, 54, 57, 60, 63, 66, 69, 72, 75, 78, 81, 84, 87, 90, 93, 96, 99, 102, 105, 108, 111, 114, 117, 120, 123, 126, 129, 132, 135, 138, 141, 144, 147, 150, 153, 156, 159, 162, 165, 168, 171, 174, 177, 180, 183, 186, 189, 192, 195, 198, 201, 204, 207, 210, 213, 216, 219, 222, 225, 228, 231, 234, 237, 240, 243, 246, 249, 252, 255, 258, 261, 264, 267, 270, 273, 276, 279, 282, 285, 288, 291, 294, 297, 300 ou 303 nucléotides, et codent respectivement des miPEPs ayant une taille de :
4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 ou 100 acides aminés.

Dans l'invention, par « *accumulation »,* on entend la production d'une molécule, telle qu'un microARN ou un micropeptide, dans la cellule.

Ainsi, la *« modulation »* de l'accumulation d'une molécule dans une cellule correspond à une modification de la quantité de cette molécule présente dans la cellule.

Par ailleurs, l'effet d'un miPEP peut être observé via la modulation de l'accumulation du miR, mais également à travers la modulation de l'accumulation du pri-miR ou du pre-miR correspondant.

Dans un mode de réalisation, l'invention concerne un procédé de détection et d'identification d'un miPEP tel que défini ci-dessus, dans lequel la modulation de l'accumulation dudit microARN est une diminution ou une augmentation de l'accumulation dudit microARN, en particulier une augmentation.

Une *« diminution de l'accumulation »* correspond à une baisse de la quantité de ladite molécule dans la cellule.

A l'inverse, une « *augmentation de l'accumulation »* correspond à une hausse de la quantité de ladite molécule dans la cellule.

Dans un mode de réalisation avantageux, l'invention concerne un procédé de détection et d'identification d'un miPEP tel que défini ci-dessus, dans lequel la modulation de l'accumulation dudit microARN est une augmentation de l'accumulation dudit microARN. Dans un mode de réalisation, l'invention concerne un procédé de détection et d'identification d'un miPEP tel que défini ci-dessus, dans lequel la présence dudit peptide dans la cellule résulte :
- de l'introduction dans la cellule d'un acide nucléique codant ledit peptide, ou
- de l'introduction dans la cellule dudit peptide.

De manière à caractériser un miPEP, il est nécessaire de disposer d'un modèle cellulaire exprimant un microARN et dans lequel ledit peptide à tester est présent. Pour cela, il est possible d'introduire un peptide dans la cellule, soit en mettant la cellule en contact avec le dit peptide, soit en introduisant dans la cellule un acide nucléique codant ledit peptide, lequel acide nucléique sera alors traduit en peptide à l'intérieur de la cellule.

Dans un mode de réalisation, l'invention concerne un procédé de détection et d'identification d'un miPEP tel que défini ci-dessus, dans lequel le dit cadre ouvert de lecture de l'étape a) est contenu dans la partie 5' ou 3' dudit transcrit primaire du microARN, de préférence dans la partie 5'.

Les parties 5' ou 3' du transcrit primaire du microARN correspondent aux parties terminales de la molécule de l'ARN qui sont clivées au cours de la maturation du microARN.

Dans un mode de réalisation, l'invention concerne un procédé de détection et d'identification d'un miPEP tel que défini ci-dessus, dans lequel ledit microARN est présent dans une cellule végétale sauvage.

Dans l'invention, une cellule végétale sauvage correspond à une cellule végétale qui n'a pas été modifiée génétiquement par l'homme.

Dans un mode de réalisation, l'invention concerne un procédé de détection et d'identification d'un miPEP tel que défini ci-dessus, dans lequel ladite cellule eucaryote déterminée et ladite cellule eucaryote de même type que la susdite cellule eucaryote déterminée, utilisées à l'étape b, sont des cellules végétales d'une plante crucifère telle *qu'Arabidopsis thaliana,* d'une plante légumineuse telle que *Glycine max* (soja), *Medicago truncatula* et *Medicago sativa* (luzerne) ou d'une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne un procédé de détection et d'identification d'un miPEP tel que défini ci-dessus, dans lequel ladite cellule eucaryote déterminée et ladite cellule eucaryote de même type que la susdite cellule eucaryote déterminée, utilisées à l'étape b, sont des cellules végétales, de préférence des cellules de *Medicago truncatula, de Nicotiana benthamiana* ou *d'Arabidopsis thaliana.*

Dans un mode de réalisation, l'invention concerne un procédé de détection et d'identification d'un miPEP tel que défini ci-dessus, dans lequel ladite cellule eucaryote déterminée et ladite cellule eucaryote de même type que la susdite cellule eucaryote déterminée, utilisées à l'étape b, sont des cellules animales, de préférence des cellules humaines ou de drosophile. Dans le procédé de détection et d'identification d'un micropeptide tel que défini ci-dessus, après avoir identifié un ORF susceptible de coder un peptide sur le transcrit primaire d'un microARN, il est nécessaire de disposer d'un modèle cellulaire possédant ledit microARN et ledit peptide, pour pouvoir démontrer un effet éventuel du peptide sur ledit microARN. Deux options sont donc envisageables :
- le modèle cellulaire dans lequel a été identifié le miORF et celui dans lequel est mis en évidence l'effet du peptide sur le miR sont identiques, ou
- le modèle cellulaire dans lequel a été identifié le miORF et celui dans lequel est mis en évidence l'effet du peptide sur le miR sont différents.

Dans la première option, le modèle cellulaire utilisé pour observer un effet du peptide est le même que celui dans lequel le transcrit primaire dudit microARN a été isolé. Dans ce modèle cellulaire, les cellules eucaryotes déterminées contiennent naturellement ledit microARN et seul le peptide à tester doit être introduit dans ces cellules. Dans ce contexte, ledit microARN est qualifié « d'origine endogène » car celui-ci existe naturellement dans les cellules. Néanmoins, dans une cellule, d'autres copies d'un microARN d'origine endogène peuvent être ajoutées, comme par exemple en introduisant dans la cellule un vecteur codant ledit microARN d'origine endogène.

Dans la deuxième option, le modèle cellulaire utilisé pour observer un effet du peptide est différent de celui dans lequel le transcrit primaire dudit microARN a été isolé. Dans ce modèle cellulaire, les cellules eucaryotes déterminées ne contiennent ni le microARN, ni le peptide à tester. Ces deux éléments doivent donc être introduits dans ces cellules. Dans ce contexte, ledit microARN est qualifié « d'origine exogène » car celui-ci n'existe pas naturellement dans les cellules.

Dans un mode de réalisation, l'invention concerne un procédé de détection et d'identification d'un miPEP tel que défini ci-dessus, dans lequel ledit microARN est d'origine endogène dans ladite cellule eucaryote et dans ladite cellule eucaryote de même type que la susdite cellule eucaryote déterminée, utilisées à l'étape b).

Dans un mode de réalisation, l'invention concerne un procédé de détection et d'identification d'un miPEP tel que défini ci-dessus dans lequel ledit microARN est d'origine exogène dans ladite cellule eucaryote et dans ladite cellule eucaryote de même type que la susdite cellule eucaryote déterminée, utilisées à l'étape b), lesdites cellules eucaryotes contenant un vecteur permettant l'expression dudit microARN.

Dans un mode de réalisation, l'invention concerne un procédé de détection et d'identification d'un miPEP tel que défini ci-dessus, dans lequel l'accumulation dudit microARN est déterminée en mettant en oeuvre une RT-PCR quantitative ou un Northern blot.

Dans un mode de réalisation, l'invention concerne un procédé de détection et d'identification d'un miPEP tel que défini ci-dessus, dans lequel l'accumulation dudit microARN est déterminée en mettant en oeuvre une puce à ADN ou à ARN.

L'accumulation dudit microARN peut être déterminée à l'aide des techniques de biologie moléculaire permettant le dosage de molécules d'acides nucléiques spécifiques.

Dans un mode de réalisation, l'invention concerne un procédé de détection et d'identification d'un microARN tel que défini ci-dessus, dans lequel la modulation de l'accumulation dudit microARN est une diminution ou une augmentation de l'accumulation dudit microARN, en particulier une augmentation.

Dans un mode de réalisation, l'invention concerne un procédé de détection et d'identification d'un microARN tel que défini ci-dessus, dans lequel la présence dudit peptide dans la cellule résulte :
- de l'introduction dans la cellule d'un acide nucléique codant ledit peptide, ou
- de l'introduction dans la cellule de ledit peptide.

Dans un mode de réalisation, l'invention concerne un procédé de détection et d'identification d'un microARN tel que défini ci-dessus, dans lequel le dit cadre ouvert de lecture de l'étape a) est contenu dans la partie 5' ou 3' dudit transcrit primaire du microARN, de préférence dans la partie 5'.

Dans un mode de réalisation, l'invention concerne un procédé de détection et d'identification d'un microARN tel que défini ci-dessus, dans lequel ledit microARN est présent dans une cellule végétale sauvage.

Dans un mode de réalisation, l'invention concerne un procédé de détection et d'identification d'un microARN tel que défini ci-dessus, dans lequel ladite cellule eucaryote, et ladite cellule eucaryote de même type que la susdite cellule eucaryote déterminée, utilisées à l'étape b) sont des cellules végétales, de préférence des cellules de *Medicago truncatula.*

Dans un mode de réalisation, l'invention concerne un procédé de détection et d'identification d'un microARN tel que défini ci-dessus, dans lequel ledit microARN est d'origine endogène dans ladite cellule eucaryote et dans ladite cellule eucaryote de même type que la susdite cellule eucaryote déterminée, utilisées à l'étape b).

Dans un mode de réalisation, l'invention concerne un procédé de détection et d'identification d'un microARN tel que défini ci-dessus dans lequel ledit microARN est d'origine exogène dans ladite cellule eucaryote et dans ladite cellule eucaryote de même type que la susdite cellule eucaryote déterminée, utilisées à l'étape b), lesdites cellules eucaryotes contenant un vecteur permettant l'expression dudit microARN.

Dans un mode de réalisation, est décrit un procédé de détection et d'identification d'un microARN tel que défini ci-dessus, dans lequel l'accumulation dudit microARN est déterminée en mettant en oeuvre une RT-PCR quantitative ou un Northern blot.

Dans un mode de réalisation, est décrit un procédé de détection et d'identification d'un microARN tel que défini ci-dessus, dans lequel l'accumulation dudit microARN est déterminée en mettant en oeuvre une puce à ADN ou à ARN.

Dans un autre aspect, l'invention concerne également l'utilisation d'au moins :
- un miPEP tel que défini ci-dessus,
- un acide nucléique codant ledit miPEP, ou
- un vecteur contenant ledit acide nucléique,

en tant qu'agent phytopharmaceutique,
   - pour favoriser la croissance et/ou le développement des plantes, notamment pour la modulation des paramètres physiologiques d'une plante, en particulier la biomasse, la surface foliaire, la floraison, le calibre du fruit ; ou
   - pour prévenir ou traiter les maladies des plantes, en particulier pour favoriser la résistance aux maladies infectieuses, ledit miPEP ayant :
   - une taille de 3 à 100 acides aminés, de préférence 4 à 20 acides aminés ;
   - une séquence peptidique identique à celle codée par une séquence nucléotidique contenue dans le transcrit primaire d'un microARN régulant l'expression dudit gène ; et
   - étant capable de moduler l'accumulation dudit miR,
ladite composition étant appliquée par pulvérisation foliaire.

L'invention repose sur l'observation surprenante faite par les Inventeurs qu'il est possible de moduler l'expression d'un ou plusieurs gènes cibles d'un même microARN en modulant l'accumulation dudit microARN à l'aide d'un miPEP.

Dans un mode de réalisation, l'invention concerne l'utilisation telle que définie ci-dessus dans laquelle ladite cellule eucaryote déterminée est une cellule végétale.

Dans un mode de réalisation, l'invention concerne l'utilisation telle que définie ci-dessus dans laquelle ladite cellule eucaryote déterminée est une cellule végétale d'une plante crucifère telle *qu'Arabidopsis thaliana,* d'une plante légumineuse telle que *Glycine max* (soja), *Medicago truncatula* et *Medicago sativa* (luzerne) ou d'une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne l'utilisation telle que définie ci-dessus dans laquelle ledit microARN et ledit gène sont d'origine endogène dans ladite cellule eucaryote déterminée.

Dans un mode de réalisation, l'invention concerne l'utilisation telle que définie ci-dessus dans laquelle ledit microARN et ledit gène sont d'origine exogène dans ladite cellule eucaryote déterminée, ladite cellule eucaryote déterminée contenant au moins un vecteur permettant l'expression dudit microARN et dudit gène.

Dans l'invention, les expressions « *d'origine endogène »* et *« d'origine exogène »* sont utilisées pour distinguer lesdits microARNs et/ou les gènes d'espèces différentes, étant donné la conservation des séquences entre espèces.

Ainsi, le terme *« d'origine endogène* » indique que le microARN et/ou le gène peuvent être présents de manière naturelle dans la cellule en question. De manière artificielle, d'autres copies du microARN et/ou du gène d'origine endogène peuvent néanmoins être ajoutées dans la cellule en question, comme par exemple par clonage.

A l'inverse, le terme *« d'origine exogène »* indique que le microARN et/ou le gène ne sont jamais présents naturellement dans la cellule en question. Il s'agit d'un microARN et/ou d'un gène identifié dans un autre type cellulaire ou dans un organisme d'une autre espèce, ce microARN et/ou ce gène sont donc nécessairement introduits artificiellement dans la cellule en question.

Dans l'invention, une cellule transformée génétiquement peut donc contenir 2 groupes de microARNs et/ou de gènes potentiellement proches en termes de séquence, l'un d'origine endogène et l'autre d'origine exogène.

Dans un mode de réalisation, l'invention concerne l'utilisation telle que définie ci-dessus dans laquelle le transcrit primaire du miARN et ledit gène sont d'origine exogène dans ladite cellule eucaryote déterminée, ladite cellule eucaryote déterminée contenant au moins un vecteur permettant l'expression du transcrit primaire du microARN.

Dans un mode de réalisation, l'invention concerne l'utilisation telle que définie ci-dessus dans laquelle le transcrit primaire du miARN est codé par un vecteur introduit articiellement dans la cellule.

Dans l'invention, le terme « *cellule eucaryote modifiée »* signifie que ladite cellule eucaryote contient un miPEP introduit artificiellement dans la cellule, que ce soit en tant que peptide, ou par l'intermédiaire d'un vecteur codant ledit miPEP.

En plus d'un miPEP introduit artificiellement dans la cellule, une cellule eucaryote modifiée selon l'invention contient également au moins un acide nucléique correspondant au miORF codant ledit miPEP.

En plus d'un miPEP introduit artificiellement dans la cellule et du miORF, une cellule eucaryote modifiée selon l'invention contient également au moins un miR, dont le transcrit primaire contient ledit miORF.

Les Inventeurs ont constaté de manière surprenante que l'utilisation de peptides dont la séquence comprend ou consiste en une séquence identique à celle de miPEPs codés sur les transcrits primaires de miRs, permet de favoriser la croissance des plantes.

Dans l'invention, le terme « *plante* » fait référence de manière générale à tout ou partie d'une plante quel que soit son stade de développement (y compris la plante sous forme de graine ou de jeune pousse), à un ou plusieurs organes de la plante (comme par exemple les feuilles, les racines, la tige, les fleurs), à une ou plusieurs cellules de la plante, ou encore à un amas de cellules de la plante.

Dans l'invention, le terme *« croissance »* fait référence au développement de tout ou partie d'une plante au cours du temps. La croissance de la plante peut ainsi être déterminée et quantifiée par le suivi de paramètres évolutifs observables pour certaines parties, cellules ou organes de la plante, tels que les feuilles, les racines, les tiges ou encore les fleurs.

De manière non limitative, les paramètres permettant de déterminer et quantifier la croissance d'une plante peuvent être notamment :
- la taille, la surface, le volume, la masse et le nombre de feuilles,
- la taille et le nombre de fleurs,
- la taille de la tige (ou de la hampe florale),
- la longueur et le nombre de racines,
- la précocité de la germination,
- la précocité du bourgeonnement,
- la précocité de l'induction florale (ou transition florale),
- ou encore le nombre de cellules.

Dans le cas des plantes légumineuses, la croissance de la plante peut également être liée à la vitesse de nodulation, ou encore à la taille et au nombre de nodules sur les racines.

Par ailleurs, dans l'invention, l'expression « *favoriser la croissance de la plante* », ou *« améliorer la croissance de la plante* », indique :
- soit une accélération du développement (comme par exemple une taille des feuilles plus importante pour une plante à un instant donné par rapport à une plante de référence),
- soit à un accroissement du développement (comme par exemple une taille de feuilles plus importante pour une plante qui ne peut être atteinte par une plante de référence),
- soit à une accélération et un accroissement du développement de la plante.

Il est important de noter que l'utilisation selon l'invention possède l'avantage d'être écologique, en comparaison des méthodes chimiques classiquement utilisées dans l'industrie botanique ou dans l'agriculture, car le miPEP est un peptide qui est présent naturellement chez la plante.

Dans l'invention, l'expression « *miPEP introduit»* fait référence à un miPEP introduit artificiellement dans la plante par opposition au « *miPEP présent naturellement dans la plante* ». L'introduction d'un miPEP dans la plante implique donc une étape technique, laquelle étape n'est pas un phénomène naturel et ne correspond ni à un croisement, ni à une sélection.

Le miPEP introduit peut être soit un peptide produit hors de la plante (comme par exemple un peptide isolé et/ou purifié, un peptide synthétique ou un peptide recombinant), soit un peptide produit dans la plante suite à l'introduction non naturelle d'un acide nucléique codant ledit miPEP dans ladite plante.

La plante dans laquelle le miPEP n'a pas été introduit possède une quantité basale dudit miPEP, qui correspond à celle dudit miPEP naturellement présent. L'utilisation d'un miPEP comprenant, ou consistant en, une séquence identique à celle dudit miPEP entraîne une augmentation de la quantité totale de miPEP, ce qui module l'accumulation du miR dont le transcrit primaire contient la séquence codant ledit miPEP.

Par ailleurs, le miPEP introduit se retrouve dans la plante et son introduction n'a pas d'impact sur sa stabilité.

Dans un mode de réalisation, l'invention concerne l'utilisation telle que définie ci-dessus, dans laquelle ledit gène, impliqué dans le développement des parties végétatives ou reproductrices de la plante, est choisi parmi le groupe consistant en : *NAC1* (Accession n°AT1G56010.1), *NAC4* (Accession n° AT5G07680.1), *NAC5* (Accession n° AT5G61430.1), *CUC1* (Accession n° AT3G15170.1), *CUC2* (Accession n° AT5G53950.1), *TCP3* (Accession n°AT1G53230.1) et *TCP4* (Accession n°AT3G15030.1) (numéros d'accession selon la banque de données *The Arabidopsis Information Resource « TAIR »).* En particulier, l'invention concerne l'utilisation telle que définie ci-dessus, dans laquelle ledit gène, impliqué dans le développement des parties végétatives ou reproductrices de la plante, est choisi parmi le groupe consistant en : *NAC1, NAC4, NAC5, CUC1* et *CUC2.*

Dans un mode de réalisation, l'invention concerne l'utilisation telle que définie ci-dessus, dans laquelle ledit gène, impliqué dans le développement des parties végétatives ou reproductrices de la plante, est choisi parmi le groupe consistant en : *TCP3* et *TCP4.*

Dans un mode de réalisation, l'invention concerne l'utilisation telle que définie ci-dessus, dans laquelle ledit miARN est choisi parmi le miR164a et le mir319a.

Dans un mode de réalisation, l'invention concerne l'utilisation telle que définie ci-dessus, dans laquelle ledit miR est choisi parmi le miR164a, le miR319a, le miR171b, le miR165a, le miR160b, le miR169d et le miR171e.

Dans un mode de réalisation, l'invention concerne l'utilisation telle que définie ci-dessus, dans laquelle ledit miPEP est choisi parmi le AtmiPEP164a1, le AtmiPEP319a1, le AtmiPEP319a2, le MtmiPEP171b1, le AtmiPEP165a1, le AtmiPEP160b1, le MtmiPEP169d, le MtmiPEP171e.

En particulier, l'invention concerne l'utilisation telle que définie ci-dessus, dans laquelle, ledit miR164a possède une séquence nucléotidique consistant en SEQ ID NO : 297.

En particulier, l'invention concerne l'utilisation telle que définie ci-dessus, dans laquelle ledit ledit miR164a possède une séquence nucléotidique présentant au moins 80% d'identité, de préférence au moins 90 % d'identité, avec la séquence nucléotidique SEQ ID NO : 297. Dans un mode de réalisation, l'invention concerne l'utilisation telle que définie ci-dessus, dans laquelle ledit miPEP est le AtmiPEP164a1, en particulier, dans laquelle ledit AtmiPEP164a1 possède une séquence d'acides aminés consistant en SEQ ID NO : 24.

En particulier, l'invention concerne l'utilisation telle que définie ci-dessus, dans laquelle, ledit miR319a possède une séquence nucléotidique consistant en SEQ ID NO : 331.

En particulier, l'invention concerne l'utilisation telle que définie ci-dessus, dans laquelle ledit ledit miR319a possède une séquence nucléotidique présentant au moins 80% d'identité, de préférence au moins 90 % d'identité, avec la séquence nucléotidique SEQ ID NO : 331. Dans un mode de réalisation, l'invention concerne l'utilisation telle que définie ci-dessus, dans laquelle ledit miPEP est le AtmiPEP319a1, en particulier, dans laquelle ledit AtmiPEP319a1 possède une séquence d'acides aminés consistant en SEQ ID NO : 76.

Dans un mode de réalisation, l'invention concerne l'utilisation telle que définie ci-dessus, dans laquelle ladite plante est une plante crucifère telle qu*'Arabidopsis thaliana,* une plante légumineuse telle que *Glycine max* (soja), *Medicago truncatula* et *Medicago sativa* (luzerne) ou une plante solanacée telle que *Nicotiana benthamiana* (tabac), *Solanum tuberosum* (pomme de terre), *Solanum lycopersicum* (tomate) ou *Solanum melongena* (aubergine).

Dans un mode de réalisation, l'invention concerne l'utilisation telle que définie ci-dessus, dans laquelle ladite plante est une plante crucifère.

Dans un mode de réalisation, l'invention concerne l'utilisation telle que définie ci-dessus, dans laquelle ladite plante est *Arabidopsis thaliana.*

Dans un mode de réalisation, l'invention concerne l'utilisation telle que définie ci-dessus, pour favoriser la croissance d'une plante *Arabidopsis thaliana,* dans laquelle le AtmiPEP164a1 est introduit dans ladite plante *Arabidopsis thaliana,* ledit AtmiPEP164a1 étant également naturellement présent dans ladite plante *Arabidopsis thaliana,*
ledit AtmiPEP164a1 introduit étant un peptide dont la séquence comprend ou consiste en une séquence identique à celle dudit AtmiPEP164a1 naturellement présent, ladite séquence du AtmiPEP164a1 naturellement présent étant codée par un cadre ouvert de lecture situé en 5' sur le transcrit primaire du miR164a, lequel miR164a régule l'expression d'au moins un gène impliqué dans le développement des parties végétatives ou reproductrices *d'Arabidopsis thaliana,*
la somme de la quantité dudit AtmiPEP164a1 introduit et de celle dudit AtmiPEP164a1 naturellement présent étant strictement supérieure à la quantité dudit AtmiPEP164a1 naturellement présent chez ladite plante *Arabidopsis thaliana.*

Dans un mode de réalisation, l'invention concerne l'utilisation telle que définie ci-dessus, pour favoriser la croissance d'une plante *Arabidopsis thaliana,* dans laquelle le AtmiPEP319a1 est introduit dans ladite plante *Arabidopsis thaliana,* ledit AtmiPEP319a1 étant également naturellement présent dans ladite plante *Arabidopsis thaliana,*
ledit AtmiPEP319a1 introduit étant un peptide dont la séquence comprend ou consiste en une séquence identique à celle dudit AtmiPEP319a1 naturellement présent, ladite séquence du AtmiPEP319a1 naturellement présent étant codée par un cadre ouvert de lecture situé en 5' sur le transcrit primaire du miR319a, lequel miR319a régule l'expression d'au moins un gène impliqué dans le développement des parties végétatives ou reproductrices *d'Arabidopsis thaliana,*
la somme de la quantité dudit AtmiPEP319a1 introduit et de celle dudit AtmiPEP319a1 naturellement présent étant strictement supérieure à la quantité dudit AtmiPEP319a1 naturellement présent chez ladite plante *Arabidopsis thaliana.*

Dans un mode de réalisation, l'invention concerne l'utilisation telle que définie ci-dessus, dans laquelle ledit miPEP est introduit par voie externe dans la plante, de préférence par arrosage, par pulvérisation ou par l'ajout d'un engrais, d'un terreau, d'un substrat de culture ou d'un support inerte.

Dans un mode de réalisation, l'invention concerne l'utilisation telle que définie ci-dessus, dans laquelle ledit miPEP est introduit par arrosage et par pulvérisation.

Dans un mode de réalisation, l'invention concerne l'utilisation telle que définie ci-dessus, dans laquelle ledit miPEP est introduit par arrosage et par l'ajout d'un engrais.

Dans un mode de réalisation, l'invention concerne l'utilisation telle que définie ci-dessus, dans laquelle ledit miPEP est introduit par pulvérisation et par l'ajout d'un engrais.

Dans un mode de réalisation, l'invention concerne l'utilisation telle que définie ci-dessus, dans laquelle ledit miPEP est introduit, par arrosage, par pulvérisation et par l'ajout d'un engrais.

Les Inventeurs ont en effet constaté de manière inattendue qu'il est possible d'appliquer directement une composition comprenant un miPEP sur la plante pour moduler l'accumulation du miR correspondant dans la plante, ce qui indique que le miPEP est capté par la plante.

Dans un mode de réalisation, l'invention concerne l'utilisation telle que définie ci-dessus, dans laquelle la plante est traitée avec une composition comprenant 10⁻⁹ M à 10⁻⁴ M dudit miPEP, notamment 10⁻⁹ M, 10⁻⁸ M, 10⁻⁷ M, 10⁻⁶ M, 10⁻⁵ M ou 10⁻⁴ M dudit miPEP.

De préférence, les compositions ont une concentration de 10⁻⁸ M à 10⁻⁵ M pour une application par arrosage ou par pulvérisation sur la plante.

De manière complémentaire, des compositions plus ou moins concentrées peuvent être envisagées pour traiter la plante avec le miPEP. Par exemple, et de manière non limitative, des compositions plus concentrées comprenant 10⁻¹ M à 10⁻³ M, notamment 10⁻² M de miPEP, peuvent être utilisées dans le cas où le miPEP introduit par voie exogène est administré à la plante par épandage.

Dans un mode de réalisation, l'invention concerne l'utilisation telle que définie ci-dessus, dans laquelle ledit miPEP est introduit dans la plante par le biais d'un acide nucléique codant ledit miPEP, ledit acide nucléique étant introduit dans la plante.

Dans un mode de réalisation, l'invention concerne l'utilisation telle que définie ci-dessus, dans laquelle la taille de la tige est augmentée chez la plante dans laquelle a été introduit ledit miPEP par rapport à la taille de la tige d'une plante identique et de même âge dans laquelle aucun miPEP n'a été introduit, ou bien par rapport à la taille de la tige d'une plante identique et de même âge dans laquelle ledit miPEP n'a pas été introduit.

Dans un mode de réalisation, l'invention concerne l'utilisation telle que définie ci-dessus, dans laquelle le nombre de feuilles est augmenté chez la plante dans laquelle a été introduit ledit miPEP par rapport au nombre de feuilles d'une plante identique et de même âge dans laquelle aucun miPEP n'a été introduit, ou bien par rapport au nombre de feuilles d'une plante identique et de même âge dans laquelle ledit miPEP n'a pas été introduit.

Dans un mode de réalisation, l'invention concerne l'utilisation telle que définie ci-dessus, dans laquelle la taille des feuilles est augmentée chez la plante dans laquelle a été introduit ledit miPEP par rapport à la taille des feuilles d'une plante identique et de même âge dans laquelle aucun miPEP n'a été introduit, ou bien par rapport à la taille des feuilles d'une plante identique et de même âge dans laquelle ledit miPEP n'a pas été introduit.

Dans un mode de réalisation, l'invention concerne l'utilisation telle que définie ci-dessus, dans laquelle le nombre de racines est augmenté chez la plante dans laquelle a été introduit ledit miPEP par rapport au nombre de racines d'une plante identique et de même âge dans laquelle aucun miPEP n'a été introduit, ou bien par rapport au nombre de racines d'une plante identique et de même âge dans laquelle ledit miPEP n'a pas été introduit.

Dans un mode de réalisation, l'invention concerne l'utilisation telle que définie ci-dessus, dans laquelle la longueur des racines est augmentée chez la plante dans laquelle a été introduit ledit miPEP par rapport à la longueur des racines d'une plante identique et de même âge dans laquelle aucun miPEP n'a été introduit, ou bien par rapport à la longueur des racines d'une plante identique et de même âge dans laquelle ledit miPEP n'a pas été introduit.

Dans un mode de réalisation, l'invention concerne l'utilisation telle que définie ci-dessus, dans laquelle la vitesse de nodulation est augmentée chez la plante dans laquelle a été introduit ledit miPEP par rapport à la vitesse de nodulation d'une plante identique et de même âge dans laquelle aucun miPEP n'a été introduit, ou bien par rapport à la vitesse de nodulation d'une plante identique et de même âge dans laquelle ledit miPEP n'a pas été introduit.

Dans un mode de réalisation, l'invention concerne l'utilisation telle que définie ci-dessus, dans laquelle le nombre de nodules est augmenté chez la plante dans laquelle a été introduit ledit miPEP par rapport au nombre de nodules d'une plante identique et de même âge dans laquelle aucun miPEP n'a été introduit, ou bien par rapport au nombre de nodules d'une plante identique et de même âge dans laquelle ledit miPEP n'a pas été introduit.

L'augmentation des paramètres permettant de déterminer et quantifier la croissance chez la plante dans laquelle a été introduit le miPEP (tels que la taille de la tige, le nombre et la taille des feuilles, le nombre et la longueur des racines, la vitesse de nodulation ou encore le nombre de nodules sur les racines) est de préférence mis en évidence par comparaison avec une plante identique (c'est-à-dire une plante de la même espèce et/ou variété), de même âge et cultivée dans les mêmes conditions mais dans laquelle aucun miPEP n'a été introduit.

L'ensemble des séquences des miPEPs, des miORFs, des miRs et des transcrits primaires de miRs est indiqué dans les tables 1, 2, 3, 4, 5 et 6.

La table 7 présente une analyse du polymorphisme de la séquence d'ADN des différentes régions du pri-miR171b (un haplotype est défini lorsqu'il diffère d'au moins un acide aminé des autres haplotypes).

**Table 1. Liste des miPEPs (miPEPs) potentiels**

| **miPEP (pI / taille MW)** | **miR** | **Organisme** | **Gènes cibles du miR** | **Séquence du miPEP** | **SEQ ID** |
|---|---|---|---|---|---|
| AtmiPEP156a1 (10,57 / 3824) | miR156a | *Arabidopsis thaliana* | Famille du gène *SPL,* impliqué dans le développement de la tige et la floraison | MFCSIQCVARHLFPLHVREIKKATRAI KKGKTL | SEQ ID NO :1 |
| AtmiPEP156a2 | miR156a | *Arabidopsis thaliana* | | MRRQTSVPFACKRDKESDKSHKER | SEQ ID NO :2 |
| AtmiPEP156a3 | miR156a | *Arabidopsis thaliana* | | MVMFFLDLDKNPRFDLLKGLKWNLF SSHISPSLPPSL | SEQ ID NO :3 |
| AtmiPEP156c1 (8,5 / 1359) | miR156c | *Arabidopsis thaliana* | | MKDNFPLLLRL | SEQ ID NO :4 |
| AtmiPEP156c2 | miR156c | *Arabidopsis thaliana* | | MSDD | SEQ ID NO :5 |
| AtmiPEP156e1 (4.03 / 1818) | miR156e | *Arabidopsis thaliana* | | MIYINKYGSISAVEDD | SEQ ID NO :6 |
| AtmiPEP156f1 (9,5 / 520) | miR156f | *Arabidopsis thaliana* | | MSQR | SEQ ID NO :7 |
| AlmiPEP159a | miR159a | *Arabidopsis lyrata* | Famille du gène *MYB,* impliqué dans la germination et la floraison | MTCPLLSLSFLLSKYI | SEQ ID NO : 8 |
| AtmiPEP159a1 (8,34 / 1898) | miR159a | *Arabidopsis thaliana* | | MTWPLLSLSFLLSKYV | SEQ ID NO :9 |
| CrmiPEP159a | miR159a | *Capsella rubella* | | MTCTLSALSLSLNMFRVN | SEQ ID NO : 10 |
| AtmiPEP159b1 (5,27 / 659) | miR159b | *Arabidopsis thaliana* | | MFYLS | SEQ ID NO : 11 |
| AtmiPEP159b2 | miR159b | *Arabidopsis thaliana* | | MVNTSSFFISSFILPLVLSESNCLLFRTI YKFSMVLY | SEQ ID NO : 12 |
| AtmiPEP160a1 (8,02 / 2936) | miR160a | *Arabidopsis thaliana* | Famille du gène *ARF,* impliqué dans la germination, le développement et la floraison | MFCLLIPIFSFVFSPNRHLRLQEQ | SEQ ID NO : 13 |
| AtmiPEP160b1 (5,28 / 608) | miR160b | *Arabidopsis thaliana* | | MFSPQ | SEQ ID NO : 14 |
| AtmiPEP160b2 | miR160b | *Arabidopsis thaliana* | | MKYIHILILFKSRSTYKLSTNHI | SEQ ID NO : 15 |
| AtmiPEP161 (10/1199) | miR161 | *Arabidopsis thaliana* | Famille du gène *PPR* | MKIPLFLPKL | SEQ ID NO : 16 |
| AtmiPEP162a1 (4,03 / 3045) | miR162a | *Arabidopsis thaliana* | Gène *DCL1,* impliqué dans le développement | MVSGQEDSWLKLSSLCFLFLSLLDSLI | SEQ ID NO : 17 |
| AtmiPEP 162b 1 (5,71 / 4114) | miR162b | *Arabidopsis thaliana* | | MFLLIFLRLIMICVCS STDFLRSVNYFC LFIYDL | SEQ ID NO : 18 |
| AtmiPEP163-1 (6,5 / 1076) | miR163 | *Arabidopsis thaliana* | Famille du gène *SAMT,* impliqué dans la production de métabolites secondaires | MSTTQEHRS | SEQ ID NO : 19 |
| AtmiPEP163-2 | miR163 | *Arabidopsis thaliana* | | MILKCWSSRFLRVSPYQNAHSLSLG | SEQ ID NO : 20 |
| AlmiPEP164a1 | miR164a | *Arabidopsis lyrata* | Famille du gène *NAC,* impliqué dans le développement racinaire, foliaire et floral | MPLAVIRQGIVWP | SEQ ID NO : 21 |
| AlmiPEP164a2 | miR164a | *Arabidopsis lyrata* | | MPSWHDMVLLPYVKHTHANTRHIT | SEQ ID NO : 22 |
| AlmiPEP164a3 | miR164a | *Arabidopsis lyrata* | | MTWFFCLT | SEQ ID NO : 23 |
| AtmiPEP164a1 (7,05 / 4256) | miR164a | *Arabidopsis thaliana* | | MPSWHGMVLLPYVKHTHASTHTHTH NIYGCACELVFH | SEQ ID NO : 24 |
| AtmiPEP164a2 | miR164a | *Arabidopsis thaliana* | | MAWYGSFALRKTHSRQHTHTHT | SEQ ID NO : 25 |
| AtmiPEP164a3 | miR164a | *Arabidopsis thaliana* | | MVWFFCLT | SEQ ID NO : 26 |
| BrmiPEP164a1 | miR164a | *Brassica rapa* | | MMIILWK | SEQ ID NO : 27 |
| BrmiPEP164a2 | miR164a | *Brassica rapa* | | MLWAKLVSFSTLHSLVFLLSPSFA | SEQ ID NO : 28 |
| BrmiPEP164a3 | miR164a | *Brassica rapa* | | MPSWHGIVILPFVKHTHANIHYSYSC VCI | SEQ ID NO : 29 |
| CpmiPEP164a1 | miR164a | *Carica papaya* | | MIACHPYLPFPLFLSLTFYSIFFSPSPPS PSLPL | SEQ ID NO : 30 |
| CpmiPEP164a2 | miR164a | *Carica papaya* | | MPSLLAFSPFPFSNILLNLLLPLPPFPLS AIITIIKPLSLSLPLSLSLSGFSV | SEQ ID NO : 31 |
| CrmiPEP164a1 | miR164a | *Capsella rubella* | | MELKGLRTWQLLDKV | SEQ ID NO : 32 |
| CrmiPEP164a2 | miR164a | *Capsella rubella* | | MPSWHGMACFYCLT | SEQ ID NO : 33 |
| CrmiPEP164a3 | miR164a | *Capsella rubella* | | MAWHGMFLLPYVKHTHANTYSLYM | SEQ ID NO : 34 |
| GrmiPEP164a1 | miR164a | *Gossypium raimondii* | | MMRSRILKFQYRFGMGIGGRKQLKN QLCQIQGRIS | SEQ ID NO :35 |
| GrmiPEP164a2 | miR164a | *Gossypium raimondii* | | MSNSRSYQLK | SEQ ID NO :36 |
| GrmiPEP164a3 | miR164a | *Gossypium raimondii* | | MNEDLEISTRKRTPQLC | SEQ ID NO :37 |
| MtmiPEP164a1 | miR164a | *Medicago truncatula* | | MPKFDIFFYIFV | SEQ ID NO :38 |
| MtmiPEP164a2 | miR164a | *Medicago truncatula* | | MSYISLSPKLLPINTKPFPWLVQFNFY FSSNTKCNKLHFLGEKLLVGEAGHVQ ILFLIHSLIMHINIFCTCSPSPTRLPHPSL | SEQ ID NO :39 |
| OsmiPEP164a1 | miR164a | *Oryza sativa* | | MQTHSNTPQSTYSLSLSLSE | SEQ ID NO :40 |
| OsmiPEP164a2 | miR164a | *Oryza sativa* | | MCVCDINMHSMLMLL | SEQ ID NO :41 |
| AlmiPEP165a | miR165a | *Arabidopsis lyrata* | Famille du gène *HD-ZIPIII,* impliqué dans développement vasculaire, racinaire, foliaire, floral, nodulation | MRIKLFQLRGMLSGSRILYIYTCVC | SEQ ID NO :42 |
| AtmiPEP165a (12,3 / 2105) | miR165a | *Arabidopsis thaliana* | | MRVKLFQLRGMLSGSRIL | SEQ ID NO :43 |
| BcmiPEP165a | miR165a | *Brassica carinata* | | MRMKLFQLRGMLSGSRILYIHKYVY MLIQVFDHICI | SEQ ID NO :44 |
| BjmiPEP165a | miR165a | *Brassica juncea* | | MRMKLFQLRGMLSGSRILYIHKYVYI C | SEQ ID NO :45 |
| BnmiPEP165a | miR165a | *Brassica napus* | | MRMKLFQLRGMLSGSRILYIHKYVY MIIQVFDHICI | SEQ ID NO :46 |
| BomiPEP165a | miR165a | *Brassica oleracea* | | MRMKLFQLRGMLSGSRILYIHKYVY MLIQVFDHICI | SEQ ID NO :47 |
| BrmiPEP165a | miR165a | *Brassica rapa* | | MRMKLFQLRGMLSGSRILYIHKYVYI C | SEQ ID NO :48 |
| AtmiPEP166a (4, 68 / 2372) | miR166a | *Arabidopsis thaliana* | | MLDLFRSNNRIEPSDFRFD | SEQ ID NO :49 |
| AtmiPEP166b (9,35 / 576) | miR166b | *Arabidopsis thaliana* | | MRDR | SEQ ID NO :50 |
| AtmiPEP167a (11 / 1148) | miR167a | *Arabidopsis thaliana* | Famille du gène *ARF,* impliqué dans le développement racinaire et floral | MNRKISLSLS | SEQ ID NO :51 |
| AtmiPEP167b1 (5,27 / 891) | miR167b | *Arabidopsis thaliana* | | MMGCFVGF | SEQ ID NO :52 |
| AtmiPEP167b2 | miR167b | *Arabidopsis thaliana* | | MQEETYEG | SEQ ID NO :53 |
| AtmiPEP169c1 (9,3 / 7110) | miR169c | *Arabidopsis thaliana* | Famille du gène du facteur CCAAT-bing, impliqué dans la nodulation, la résistance à la sécheresse, la résistance à la carence azotée | MPHTNLKDLFIFSPNVFFSFAIYLHNS WNKNYIHKRENFHNTSFALIFFFSSIM SINYG | SEQ ID NO :54 |
| AtmiPEP169c2 | miR169c | *Arabidopsis thaliana* | | MFFFRLLFISTILGTKTTFTNERIFTTPL LLSFFFFRPL | SEQ ID NO :55 |
| AtmiPEP169l (8,52/ 786) | miR169l | *Arabidopsis thaliana* | | MRHKES | SEQ ID NO :56 |
| AtmiPEP171a1 (11,05 / 4057) | miR171a | *Arabidopsis thaliana* | Famille du gène *GRAS,* impliqué dans le développement floral, foliaire, racinaire, la mycorhization, la nodulation | MNLLKKERQRRRQRSIGSHCIASLVL KDGYMKKI | SEQ ID NO :57 |
| AtmiPEP171b (8,5 / 995) | miR171b | *Arabidopsis thaliana* | | MVLSGKLTF | SEQ ID NO :58 |
| MtmiPEP171bl | miR171b | *Medicago truncatula* | | MLLHRLSKFCKIERDIVYIS | SEQ ID NO :59 |
| MtmiPEP171b2 | miR171b | *Medicago truncatula* | | MKIEE | SEQ ID NO :60 |
| ZmmiPEP171b | miR171b | *Zea mays* | | MHLPSTPSRPPPQHTSLSFLGKEMTKG TTTACFG | SEQ ID NO :61 |
| AtmiPEP171c1 (6,68 / 1187) | miR171c | *Arabidopsis thaliana* | | MLSLSHFHIC | SEQ ID NO :62 |
| MtmiPEP171e | miR171e | *Medicago truncatula* | | MMVFGKPKKAMLVRFNPKTDLHV | SEQ ID NO :63 |
| MtmiPEP171h | miR171h | *Medicago truncatula* | | MASAAKVYMA | SEQ ID NO :64 |
| AtmiPEP 172a1 (8,5 / 734) | miR172a | *Arabidopsis thaliana* | Famille du gène *AP2,* impliqué dans le développement floral | MASKIW | SEQ ID NO :65 |
| AtmiPEP 172a3 | miR172a | *Arabidopsis thaliana* | | MVRFQLSIRD | SEQ ID NO :66 |
| AtmiPEP 172b 1 (7,9 / 1621) | miR172b | *Arabidopsis thaliana* | | MCTYYYLINKYF | SEQ ID NO :67 |
| AtmiPEP172c1 (7,98 / 1367) | miR172c | *Arabidopsis thaliana* | | MFPAKWCRLES | SEQ ID NO :68 |
| AtmiPEP172e1 (8,35 / 2452) | miR172e | *Arabidopsis thaliana* | | MGSLSLFKSQLEILMLLLSLSK | SEQ ID NO :69 |
| AtmiPEP172e2 | miR172e | *Arabidopsis thaliana* | | MSVYIHVPISLNCFSPKSSC | SEQ ID NO :70 |
| AtmiPEP172e3 | miR172e | *Arabidopsis thaliana* | | MGVPNFRPRNR | SEQ ID NO :71 |
| AcmiPEP319a1 | miR319a | *Arabidopsis cebennensis* | | MRSRVSFFFFKIMLFRLLGYRSM | SEQ ID NO :72 |
| AcmiPEP319a2 | miR319a | *Arabidopsis cebennensis* | | MHTYIHTISNISSIFFCSKRSFSPFTYIRI IVVIDPFRIALTFR | SEQ ID NO :73 |
| AhmiPEP319a | miR319a | *Arabidopsis halleri* | | MRSRVSLFLSFSSNFAAYSPRS | SEQ ID NO :74 |
| AlmiPEP319a | miR319a | *Arabidospis lyrata* | | MHTYIPSSSFPISNISSVFFCYKRSFSPY TYIRIIVVIDPFRIALTFR | SEQ ID NO :75 |
| AtmiPEP319a1 (6,56 / 5917) | miR319a | *Arabidopsis thaliana* | | MNIHTYHHLLFPSLVFHQSSDVPNALS LHIHTYEYIIVVIDPFRITLAFR | SEQ ID NO :76 |
| AtmiPEP319a2 | miR319a | *Arabidopsis thaliana* | | MFQTLYLFIYIHTNILLLS | SEQ ID NO :77 |
| BrmiPEP319a | miR319a | *Brassica rapa* | | MFKLYFSAILSTQYMHTYHHRIALIFL SILYPSTNYLMSPILNPT | SEQ ID NO :78 |
| CpmiPEP319a | miR319a | *Carica papaya* | Famille du gène *TCP,* impliqué dans le développement floral et foliaire | MKIKLGFSLIKIIILLDKNS | SEQ ID NO :79 |
| CrmiPEP319a | miR319a | *Capsella rubella* | | MHPHTYIHIPSSSFLISSFCL | SEQ ID NO :80 |
| EgmiPEP319a | miR319a | *Eucalyptus grandis* | | MKHIQRWRYGETSGRQGDWKRLEIK VHSNPSLKVKKNTNNFSSSL | SEQ ID NO : 81 |
| GrmiPEP319a | miR319a | *Gossypium raimondii* | | MIHFNLSQWRAICMANFHLTYSFLFG VL | SEQ ID NO : 82 |
| MtmiPEP319a | miR319a | *Medicago truncatula* | | MHVYLELFMVIKGLGFLLLVK | SEQ ID NO :83 |
| OsmiPEP319a | miR319a | *Oryza sativa* | | MEMIQRPCLILKFFFKLSTLYIP | SEQ ID NO : 84 |
| PpmiPEP319a | miR319a | *Physcomitrella patens* | | MFHRRRSSVLLPPFGQTQPNPRCLPDL RFPSCFTPCTA | SEQ ID NO :85 |
| ThmiPEP319a1 | miR319a | *Thellungiella halophila* | | MTICKVSKACFYAGKIENSRLIKKIGIP KREGAPFSPIRENQ | SEQ ID NO :86 |
| ThmiPEP319a2 | miR319a | *Thellungiella halophila* | | MEIQIKKKNLYIMNTQKLPNLYIYIYK YVFIKLMVVE | SEQ ID NO :87 |
| AtmiPEP319b1 (8,04 / 5120) | miR319b | *Arabidopsis thaliana* | | MVPQINLWSSRVILKIRIDSSTHREED HCIQNHKHGLSFIFSF | SEQ ID NO :88 |
| AtmiPEP394a1 (9,7 / 1977) | miR394a | *Arabidopsis thaliana* | Famille du gène *F-box,* impliqué dans le développement foliaire et résistance à la sécheresse | MSLQFYERVSFKNTVK | SEQ ID NO :89 |
| AtmiPEP395c1 (3,58 / 1429) | miR395c | *Arabidopsis thaliana* | Famille des gènes *APS* et *AST,* impliqués dans la germination et le métabolisme du soufre | MTEQEEESQMST | SEQ ID NO :90 |
| AtmiPEP395e1 (9,98 / 4700) | miR395e | *Arabidopsis thaliana* | | MYLQYIDNVISIYSNNRRVGRMFSRV PLSTSLEIQFFIK | SEQ ID NO :91 |
| AtmiPEP397b1 (4,53 / 1418) | miR397b | *Arabidopsis thaliana* | Famille des gènes de laccases, impliqués dans le métabolisme du cuivre, leur surexpression améliore la croissance | MSKEIFFSPGFE | SEQ ID NO :92 |
| AtmiPEP398c1 | miR398c | *Arabidopsis thaliana* | Famille du gène *CSD,* impliqué dans le métabolisme du cuivre, sa surexpression améliore la croissance | MRTHEQSTAITTLRHCYSSRFMCSQV TPAELFLYRPCFINAVAR | SEQ ID NO :93 |
| AtmiPEP399b (11 / 678) | miR399b | *Arabidopsis thaliana* | Famille du gène *PHO2,* impliqué dans le métabolisme du phosphore | MKRNM | SEQ ID NO :94 |
| AtmiPEP399d1 (4 / 622) | miR399d | *Arabidopsis thaliana* | | MQCEI | SEQ ID NO :95 |
| AtmiPEP403 (5,27470) | miR403 | *Arabidopsis thaliana* | Famille du gène *AGO* | MFCA | SEQ ID NO :96 |
| AtmiPEP447a1 (6,69 / 724) | miR447a | *Arabidopsis thaliana* | Famille des gènes de phosphoglycérate kinase | MVMAHH | SEQ ID NO :97 |
| AtmiPEP447a2 | miR447a | *Arabidopsis thaliana* | | MMKPRWNCSLYGITEWTNNQNQKSK RKGRRKTQIWRIGDRLDTVECITLML SAY | SEQ ID NO :98 |
| AtmiPEP447b1 (4/1155) | miR447b | *Arabidopsis thaliana* | | MLLIIVELVL | SEQ ID NO :99 |
| AtmiPEP447b2 | miR447b | *Arabidopsis thaliana* | | MLCFNFRCVRRFAE | SEQ ID NO : 100 |
| AtmiPEP447c | miR447c | *Arabidopsis thaliana* | | MYTYQLDNSFSWFLCTRFCLYRYFLF NFRCFRRFSE | SEQ ID NO :101 |
| DmmiPEPla | miR1 | *Drosophila melanogaster* | *Différenciation musculaire* | MWREVCAQKSQTKRRNFITGNQR RNKTKANRKAETKQQKVYEFFVQ ARERCKTRKKHEKKTLKKTKKIQ NRYRAVSENEWGKGFPSHI | SEQ ID NO :102 |
| DmmiPEPlb | miR1 | *Drosophila melanogaster* | *Différenciation musculaire* | MRTKKSNKKAQFYYGQPTTKQNK SQPKSRNKAAKSL | SEQ ID NO :103 |
| DmmiPEP8 | miR8 | *Drosophila melanogaster* | Croissance | MEPGFVFVLFPTHLSTQHTQREKSI LVMGLNLQSAKQSDKQNSKERKK NTQINSQRIPYRQGGQCSKVLSP | SEQ ID NO : 104 |
| HsmiPEP155 | miR155 | *Homo sapiens* | inflammation | MEMALMVAQTRKGKSVV | SEQ ID NO :355 |
| AtmiPEP157c (5,95 / 1776) | miR157c | *Arabidopsis thaliana* | Famille du gène *SPL,* impliqué dans le développement de la tige et la floraison | MMLHITHRFESDVGC | SEQ ID NO : 375 |
| AtmiPEP157d (5,27 / 524) | miR157d | *Arabidopsis thaliana* | | MLYV | SEQ ID NO : 376 |
| AtmiPEP160c (9,98 / 1790) | miR160c | *Arabidopsis thaliana* | Famille du gène ARF, impliqué dans la germination, le développement et la floraison | MFMRRGLVYNNIYI | SEQ ID NO : 377 |
| AtmiPEP164b (4,72 / 1949) | miR164b | *Arabidopsis thaliana* | Famille du gène NAC, impliqué dans le développement racinaire, foliaire et floral | MMKVCDEQDGEAGHVHY | SEQ ID NO : 378 |
| AtmiPEP166c (10,42 / 3407) | miR166c | *Arabidopsis thaliana* | Famille du gène HD-ZIPIII, impliqué dans développement vasculaire, racinaire, foliaire, floral, nodulation | MKKRITRINLEEQIKKTLDDSRTRL HSP | SEQ ID NO : 379 |
| AtmiPEP166d (8,35 / 1125) | miR166d | *Arabidopsis thaliana* | | MKKIGSIDSF | SEQ ID NO : 380 |
| AtmiPEP169a (9,5 / 784) | miR169a | *Arabidopsis thaliana* | Famille du gène du facteur CCAAT-bing, impliqué dans la nodulation, la résistance à la sécheresse, la résistance à la carence azotée | MTCRFK | SEQ ID NO : 381 |
| AtmiPEP169h1 (5,28 / 349) | miR169h | *Arabidopsis thaliana* | | MVT | |
| AtmiPEP169h2 | miR169h | *Arabidopsis thaliana* | | MKNENLCGSQG | SEQ ID NO : 382 |
| AtmiPEP169n (8,96 / 5315) | miR169n | *Arabidopsis thaliana* | | MKCMMKKRGLTWRKASCLVAKD DLPDLFRLHDSISNSCILDYYTF | SEQ ID NO : 383 |
| AtmiPEP170 (5,75 / 879) | miR170 | *Arabidopsis thaliana* | Famille du gène GRAS, impliqué dans le développement floral, foliaire, racinaire, la mycorhization, la nodulation | MFPRESL | SEQ ID NO : 384 |
| AtmiPEP396a (5,3 / 3636) | miR396a | *Arabidopsis thaliana* | Famille des gènes GRF impliqués dans le développement racinaire et la prolifération cellulaire, la mycorhization | MTLSVFFHSFLELQNFFRFFFFSFDI SYA | SEQ ID NO : 385 |
| AtmiPEP399c (8,66/2703) | miR399c | *Arabidopsis thaliana* | Famille du gène PHO2, impliqué dans le métabolisme du phosphore | MSLAKGELPCHCFRLNTVYNRFC | SEQ ID NO : 386 |
| MtmiPEP169d | miR169d | *Medicago tuncatula* | Famille des gènes NF-YA (ou HAP2) | MVKESFMERLKVR | SEQ ID NO : 424 |

**Table 2. Liste des miORFs**

| **miPEP** | **Organisme** | **Séquence du miORF** | **SEQ ID** |
|---|---|---|---|
| AtmiPEP156a1 | *Arabidopsis thaliana* | ATGTTCTGTTCAATTCAATGCGTCGCCAGACATCTGTTCCCTTTGC ATGTAAGAGAGATAAAGAAAGCGACAAGAGCCATAAAGAAAGG TAA | SEQ ID NO : 105 |
| AtmiPEP156a2 | *Arabidopsis thaliana* | ATGCGTCGCCAGACATCTGTTCCCTTTGCATGTAAGAGAGATAAA GAAAGCGACAAGAGCCATAAAGAAAGGTAA | SEQ ID NO : 106 |
| AtmiPEP156a3 | *Arabidopsis thaliana* | ATGGTTATGTTTTTTCTCGATTTAGACAAAAACCCTAGATTTGATC TTCTAAAGGGTCTCAAATGGAATCTCTTCTCTTCTCATATCTCTCC CTCTCTCCCTCCCTCTCTTTGA | SEQ ID NO : 107 |
| AtmiPEP156c1 | *Arabidopsis thaliana* | ATGAAGGACAACTTTCCTCTTCTCCTTCGGTTATAA | SEQ ID NO :108 |
| AtmiPEP156c2 | *Arabidopsis thaliana* | ATGAGTGATGACTGA | SEQ ID NO : 109 |
| AtmiPEP156e1 | *Arabidopsis thaliana* | ATGATATATATAAATAAATATGGGTCGATATCGGCTGTGGAGGAC GACTAG | SEQ ID NO : 110 |
| AtmiPEP156f1 | *Arabidopsis thaliana* | ATGAGCCAAAGATAA | SEQ ID NO : 111 |
| AlmiPEP159a | *Arabidopsis lyrata* | ATGACGTGTCCTCTTCTCTCTCTCTCTTTCCTTCTCTCTAAGTATAT TTAG | SEQ ID NO : 112 |
| AtmiPEP159a1 | *Arabidopsis thaliana* | ATGACGTGGCCTCTTCTCTCTCTCTCTTTCCTTCTCTCTAAGTATGT TTAG | SEQ ID NO : 113 |
| CrmiPEP159a | *Capsella rubella* | ATGACGTGTACTCTCTCTGCTCTATCTCTCTCTCTAAATATGTTTA GGGTTAA | SEQ ID NO : 114 |
| AtmiPEP159b1 | *Arabidopsis thaliana* | ATGTTTTATCTTTCATAA | SEQ ID NO : 115 |
| AtmiPEP159b2 | *Arabidopsis thaliana* | ATGGTTAATACTAGTAGCTTTTTCATTTCAAGTTTTATCCTTCCAT TGGTTCTTTCTGAGTCAAATTGTCTCCTGTTTCGAACCATATATAA GTTTTCAATGGTTTTGTATTAA | SEQ ID NO : 116 |
| AtmiPEP160a1 | *Arabidopsis thaliana* | ATGTTTTGTTTGTTGATTCCCATCTTCTCTTTTGTCTTTTCACCAAA TCGTCATTTAAGGCTTCAAGAACAGTAA | SEQ ID NO : 117 |
| AtmiPEP160b1 | *Arabidopsis thaliana* | ATGTTTTCCCCTCAATGA | SEQ ID NO : 118 |
| AtmiPEP160b2 | *Arabidopsis thaliana* | ATGAAATACATACACATTTTGATTTTATTTAAATCAAGATCGACG TATAAGCTATCCACCAATCATATTTAA | SEQ ID NO : 119 |
| AtmiPEP161 | *Arabidopsis thaliana* | ATGAAAATTCCATTGTTTCTGCCGAAGCTTTGA | SEQ ID NO : 120 |
| AtmiPEP162a1 | *Arabidopsis thaliana* | ATGGTATCTGGTCAAGAAGATTCCTGGTTAAAACTTTCATCTCTCT GTTTCCTTTTTCTTTCTTTGTTGGATTCATTAATTTGA | SEQ ID NO : 121 |
| AtmiPEP162b1 | *Arabidopsis thaliana* | ATGTTTCTTTTAATCTTTTTGAGATTAATAATGATTTGTGTTTGTTC ATCAACCGATTTTCTCAGATCTGTCAATTATTTTTGTTTATTTATTT ATGATTTATGA | SEQ ID NO : 122 |
| AtmiPEP163-1 | *Arabidopsis thaliana* | ATGTCCACTACTCAAGAGCATAGGTCTTGA | SEQ ID NO : 123 |
| AtmiPEP163-2 | *Arabidopsis thaliana* | ATGATACTAAAGTGCTGGAGTTCCCGGTTCCTGAGAGTGAGTCCA TATCAAAATGCGCATTCGTTATCACTTGGTTGA | SEQ ID NO : 124 |
| AlmiPEP164a1 | *Arabidopsis lyrata* | ATGCCCTTAGCAGTTATTAGACAAGGGATTGTTTGGCCCTAG | SEQ ID NO : 125 |
| AlmiPEP164a2 | *Arabidopsis lyrata* | ATGCCATCATGGCATGACATGGTTCTTTTGCCTTACGTAAAACAC ACTCACGCCAACACACGCCACATAACATAA | SEQ ID NO : 126 |
| AlmiPEP164a3 | *Arabidopsis lyrata* | ATGACATGGTTCTTTTGCCTTACGTAA | SEQ ID NO : 127 |
| AtmiPEP164a1 | *Arabidopsis thaliana* | ATGCCATCATGGCATGGTATGGTTCTTTTGCCTTACGTAAAACAC ACTCACGCCAGCACACACACACACACACATAACATATACGGATG TGCGTGTGAGCTAGTCTTCCATTAA | SEQ ID NO : 128 |
| AtmiPEP164a2 | *Arabidopsis thaliana* | ATGGCATGGTATGGTTCTTTTGCCTTACGTAAAACACACTCACGC CAGCACACACACACACACACATAA | SEQ ID NO : 129 |
| AtmiPEP164a3 | *Arabidopsis thaliana* | ATGGTATGGTTCTTTTGCCTTACGTAA | SEQ ID NO :130 |
| BrmiPEP164a1 | *Brassica rapa* | ATGATGATAATTTTGTGGAAATAA | SEQ ID NO : 131 |
| BrmiPEP164a2 | *Brassica rapa* | ATGCTTTGGGCCAAGCTAGTTTCTTTTAGCACTCTTCACTCACTAG TTTTTCTTCTCAGCCCTTCTTTTGCGTGA | SEQ ID NO : 132 |
| BrmiPEP164a3 | *Brassica rapa* | ATGCCATCATGGCATGGCATTGTCATTTTGCCTTTCGTAAAACAC ACTCACGCCAACATACATTATTCATATTCATGTGTATGTATATGA ATGCCATCATGGCATATGCCATCATGGCAT | SEQ ID NO :133 |
| CpmiPEP164a1 | *Carica papaya* | ATGATTGCATGCCATCCCTACTTGCCTTTTCCCCTTTTCCTTTCTCT AACATTTTACTCAATCTTCTTCTCCCCCTCCCCCCCTTCCCCCTCTC TGCCATTATAA | SEQ ID NO : 134 |
| CpmiPEP164a2 | *Carica papaya* | ATGCCATCCCTACTTGCCTTTTCCCCTTTTCCTTTCTCTAACATTTT ACTCAATCTTCTTCTCCCCCTCCCCCCCTTCCCCCTCTCTGCCATTA TAACCATAATTAAACCTCTCTCCCTCTCTCTCCCTCTCTCTCTCTCT CTCTCTGGGTTCTCAGTATAA | SEQ ID NO :135 |
| CrmiPEP164a1 | *Capsella rubella* | ATGGAATTAAAAGGTTTGAGAACTTGGCAGTTATTAGACAAGGTA TAG | SEQ ID NO :136 |
| CrmiPEP164a2 | *Capsella rubella* | ATGCCATCATGGCATGGCATGGCATGTTTCTATTGCCTTACGTAA | SEQ ID NO :137 |
| CrmiPEP164a3 | *Capsella rubella* | ATGGCATGGCATGTTTCTATTGCCTTACGTAAAACACACTCACGC CAACACATACTCACTATACATGTAAATAAGTATGTGCGCGTGTGA | SEQ ID NO : 138 |
| GrmiPEP164a1 | *Gossypium raimondii* | ATGATGAGATCAAGAATTTTAAAGTTTCAATATAGATTTGGCATG GGTATTGGCGGCAGAAAGCAATTAAAAAACCAGTTATGTCAAAT TCAAGGTCGTATCAGTTAA | SEQ ID NO : 139 |
| GrmiPEP164a2 | *Gossypium raimondii* | ATGTCAAATTCAAGGTCGTATCAGTTAAAATGA | SEQ ID NO :140 |
| GrmiPEP164a3 | *Gossypium raimondii* | ATGAATGAAGATTTAGAAATTTCAACAAGGAAGAGGACCCCACA GCTTTGTTAA | SEQ ID NO : 141 |
| MtmiPEP164a1 | *Medicago truncatula* | ATGCCCAAATTTGATATTTTTTTTTATATATTTGTATAG | SEQ ID NO : 142 |
| MtmiPEP164a2 | *Medicago truncatula* | ATGTCATATATCTCTCTCTCTCCTAAGTTGCTACCTATAAATACTA AGCCTTTCCCTTGGTTGGTTCAATTCAACTTCTACTTCTCATCAAA CACAAAGTGCAATAAGCTTCATTTCCTGGGTGAGAAGCTCCTTGT TGGAGAAGCAGGGCACGTGCAAATCCTCTTTCTGATTCATTCTCT CATAATGCATATCAATATCTTTTGCACGTGCTCCCCTTCTCCAACT AGG | SEQ ID NO :143 |
| OsmiPEP164a1 | *Oryza sativa* | ATGCAAACCCACTCCAACACTCCACAATCCACATACTCTCTCTCT CTCTCTCTCTCTGAGTAG | SEQ ID NO : 144 |
| OsmiPEP164a2 | *Oryza sativa* | ATGTGTGTGTGTGATATCAATATGCATTCGATGTTGATGCTACTGT AG | SEQ ID NO :145 |
| AlmiPEP165a | *Arabidopsis lyrata* | ATGAGAATTAAGCTATTTCAGTTGAGGGGAATGTTGTCTGGATCG AGGATATTATACATATATACATGTGTATGTTGA | SEQ ID NO :146 |
| AtmiPEP165a | *Arabidopsis thaliana* | ATGAGGGTTAAGCTATTTCAGTTGAGGGGAATGTTGTCTGGATCG AGGATATTATAG | SEQ ID NO : 147 |
| BcmiPEP165a | *Brassica carinata* | ATGAGAATGAAGCTATTTCAGTTGAGGGGAATGTTGTCTGGATCG AGGATATTATATATACACAAATACGTATATATGTTAATACAAGTG TTTGATCATATATGTATATAG | SEQ ID NO :148 |
| BjmiPEP165a | *Brassica juncea* | ATGAGAATGAAGCTATTTCAGTTGAGGGGAATGTTGTCTGGATCG AGGATATTATATATACACAAATATGTATATATATGTTAA | SEQ ID NO :149 |
| BnmiPEP165a | *Brassica napus* | ATGAGAATGAAGCTATTTCAGTTGAGGGGAATGTTGTCTGGATCG AGGATATTATATATACACAAATACGTATATATGATAATACAAGTG TTTGATCATATATGTATATAG | SEQ ID NO : 150 |
| BomiPEP165a | *Brassica oleracea* | ATGAGAATGAAGCTATTTCAGTTGAGGGGAATGTTGTCTGGATCG AGGATATTATATATACACAAGTACGTATATATGTTAATACAAGTG TTTGATCATATATGTATATAG | SEQ ID NO : 151 |
| BrmiPEP165a | *Brassica rapa* | ATGAGAATGAAGCTATTTCAGTTGAGGGGAATGTTGTCTGGATCG AGGATATTATATATACACAAATATGTATATATATGTTAA | SEQ ID NO : 152 |
| AtmiPEP166a | *Arabidopsis thaliana* | ATGTTGGATCTCTTTCGATCTAACAATCGAATTGAACCTTCAGATT TCAGATTTGATTAG | SEQ ID NO : 153 |
| AtmiPEP166b | *Arabidopsis thaliana* | ATGAGAGATAGATAA | SEQ ID NO : 154 |
| AtmiPEP167a | *Arabidopsis thaliana* | ATGAACAGAAAAATCTCTCTTTCTCTTTCTTGA | SEQ ID NO : 155 |
| AtmiPEP167b1 | *Arabidopsis thaliana* | ATGATGGGTTGTTTTGTGGGATTTTAA | SEQ ID NO : 156 |
| AtmiPEP167b2 | *Arabidopsis thaliana* | ATGCAGGAGGAAACATATGAGGGGTGA | SEQ ID NO : 157 |
| AtmiPEP169c1 | *Arabidopsis thaliana* | ATGCCACATACAAACTTGAAAGATCTCTTCATCTTTTCTCCAAATG TTTTTTTTTCGTTTGCTATTTATCTCCACAATTCTTGGAACAAAAA CTACATTCACAAACGAGAGAATTTTCACAACACCTCTTTTGCTCTC ATTTTTTTTTTTTCGTCCATTATGAGTATTAATTATGGTTAG | SEQ ID NO : 158 |
| AtmiPEP169c2 | *Arabidopsis thaliana* | ATGTTTTTTTTTCGTTTGCTATTTATCTCCACAATTCTTGGAACAA AAACTACATTCACAAACGAGAGAATTTTCACAACACCTCTTTTGC TCTCATTTTTTTTTTTTCGTCCATTATGA | SEQ ID NO : 159 |
| AtmiPEP169l1 | *Arabidopsis thaliana* | ATGAGACATAAAGAGAGTTAA | SEQ ID NO : 160 |
| AtmiPEP171a1 | *Arabidopsis thaliana* | ATGAACCTCCTCAAGAAGGAAAGACAGAGGAGGAGACAAAGAA GTATAGGTTCACATTGCATAGCCAGTTTAGTTTTGAAGGATGGAT ATATGAAAAAAATATGA | SEQ ID NO : 161 |
| AtmiPEP171b | *Arabidopsis thaliana* | ATGGTTCTCTCCGGTAAATTAACATTTTAG | SEQ ID NO : 162 |
| MtmiPEP171b1 | *Medicago truncatula* | ATGCTTCTTCATAGGCTCTCCAAATTTTGCAAAATTGAAAGAGAC ATAGTATATATATCTTAG | SEQ ID NO : 163 |
| MtmiPEP171b2 | *Medicago truncatula* | ATGAAGATTGAAGAGTAA | SEQ ID NO : 164 |
| ZmmiPEP171b | *Zea mays* | ATGCATCTGCCTTCAACTCCCTCTCGCCCCCCACCCCAACACACAT CTCTCTCTTTTCTAGGGAAGGAAATGACGAAGGGGACGACGACG GCATGCTTCGGCTAG | SEQ ID NO : 165 |
| AtmiPEP171c1 | *Arabidopsis thaliana* | ATGTTGTCTCTTTCTCATTTTCATATCTGCTAA | SEQ ID NO : 166 |
| MtmiPEP171e | *Medicago truncatula* | ATGATGGTGTTTGGGAAGCCGAAAAAAGCGATGTTGGTGAGGTT CAATCCGAAGACGGATTTACATGTATAG | SEQ ID NO : 167 |
| MtmiPEP171h | *Medicago truncatula* | ATGGCTTCAGCTGCAAAAGTATACATGGCGTGA | SEQ ID NO :168 |
| AtmiPEP172a1 | *Arabidopsis thaliana* | ATGGCTTCCAAGATCTGGTAA | SEQ ID NO : 169 |
| AtmiPEP172a3 | *Arabidopsis thaliana* | ATGGTTAGGTTCCAACTAAGTATACGAGATTAA | SEQ ID NO : 170 |
| AtmiPEP172b1 | *Arabidopsis thaliana* | ATGTGTACGTACTATTATCTCATAAATAAATATTTTTAA | SEQ ID NO : 171 |
| AtmiPEP172c1 | *Arabidopsis thaliana* | ATGTTTCCAGCAAAATGGTGCCGTCTTGAGTCTTGA | SEQ ID NO : 172 |
| AtmiPEP172e1 | *Arabidopsis thaliana* | ATGGGATCTCTCTCTTTATTTAAAAGTCAATTAGAGATCTTGATGC TACTTCTGTCCCTTTCCAAGTGA | SEQ ID NO : 173 |
| AtmiPEP172e2 | *Arabidopsis thaliana* | ATGAGTGTATATATTCATGTACCTATCTCTCTCAATTGCTTCTCAC CAAAATCATCTTGCTGA | SEQ ID NO : 174 |
| AtmiPEP172e3 | *Arabidopsis thaliana* | ATGGGAGTTCCCAACTTTAGACCTCGAAACCGATAA | SEQ ID NO : 175 |
| AcmiPEP319a1 | *Arabidopsis cebennensis* | ATGAGATCTAGGGTTTCTTTCTTTTTCTTCAAAATCATGCTTTTTC GCTTGCTAGGTTATAGATCCATGTAA | SEQ ID NO : 176 |
| AcmiPEP319a2 | *Arabidopsis cebennensis* | ATGCATACATACATACATACCATCTCTAATATTTCATCAATCTTCT TTTGTTCCAAACGCCTTTCTCTCCATTTACATACATACGAATCATT GTTGTCATAGATCCGTTTAGAATTGCTTTAACTTTTAGATGA | SEQ ID NO : 177 |
| AhmiPEP319a | *Arabidopsis halleri* | ATGAGATCTAGGGTTTCTTTGTTTCTTTCGTTTTCTTCAAATTTTGC TGCATATTCTCCAAGATCATGA | SEQ ID NO : 178 |
| AlmiPEP319a | *Arabidospis lyrata* | ATGCATACATACATACCATCATCATCTTTTCCCATCTCTAATATTT CATCAGTCTTCTTTTGTTACAAACGCTCTTTCTCGCCATATACATA CATAAGAATCATTGTTGTCATAGATCCGTTTAGAATTGCTTTAACT TTTAGATGA | SEQ ID NO : 179 |
| AtmiPEP319a1 | *Arabidopsis thaliana* | ATGAATATACATACATACCATCATCTTCTTTTCCCATCTCTAGTTT TTCATCAATCTTCTGATGTTCCAAACGCTCTATCTCTTCATATACA TACATACGAATATATTATTGTTGTCATAGATCCATTTAGAATCACT TTAGCTTTTAGATGA | SEQ ID NO : 180 |
| AtmiPEP319a2 | *Arabidopsis thaliana* | ATGTTCCAAACGCTCTATCTCTTCATATACATACATACGAATATAT TATTGTTGTCATAG | SEQ ID NO : 181 |
| BrmiPEP319a | *Brassica rapa* | ATGTTTAAGCTCTACTTCTCAGCAATTCTCTCCACCCAATACATGC ATACATACCATCATCGTATCGCTCTAATTTTTCTATCAATCTTGTA TCCTTCCACAAATTATCTTATGTCTCCCATTTTAAATCCTACATAG | SEQ ID NO : 182 |
| CpmiPEP319a | *Carica papaya* | ATGAAGATTAAATTAGGTTTTAGTCTTATTAAGATTATTATATTAC TAGACAAAAACAGTTAA | SEQ ID NO : 183 |
| CrmiPEP319a | *Capsella rubella* | ATGCATCCACATACATACATACATATACCATCATCTTCTTTTCTCA TCTCTAGTTTTTGTTTATAA | SEQ ID NO : 184 |
| EgmiPEP319a | *Eucalyptus grandis* | ATGAAGCATATTCAAAGGTGGAGATATGGGGAGACTTCCGGAAG GCAAGGGGATTGGAAAAGGCTCGAGATCAAAGTGCATAGCAACC CTTCGCTAAAGGTGAAAAAGAATACGAATAACTTCAGTAGCTCAC TTTAA | SEQ ID NO : 185 |
| GrmiPEP319a | *Gossypium raimondii* | ATGATCCATTTCAACCTGTCACAGTGGAGAGCAATTTGTATGGCT AATTTCCATCTCACCTATTCTTTTCTGTTTGGGGTTCTCTAG | SEQ ID NO : 186 |
| MtmiPEP319a | *Medicago truncatula* | ATGCATGTATATCTTGAATTGTTTATGGTAATAAAGGGGTTAGGA TTTCTCCTTTTGGTGAAGTGA | SEQ ID NO : 187 |
| OsmiPEP319a | *Oryza sativa* | ATGGAAATGATACAAAGGCCGTGTTTAATTTTAAAATTTTTTTTCA AACTTTCAACACTTTACATCCCATAA | SEQ ID NO :188 |
| PpmiPEP319a | *Physcomitrella patens* | ATGTTCCACCGTCGGAGATCCTCGGTGCTGCTACCCCCGTTCGGC CAAACCCAACCCAACCCTAGGTGTCTGCCGGACCTCCGCTTCCCC TCCTGCTTCACCCCCTGCACCGCTTAA | SEQ ID NO : 189 |
| ThmiPEP319a1 | *Thellungiella halophila* | ATGACGATATGTAAAGTAAGCAAGGCATGTTTTTATGCAGGGAA GATTGAAAATTCAAGATTAATCAAGAAAATTGGAATACCAAAAA GAGAGGGAGCTCCCTTCAGTCCAATCAGAGAGAATCAATGA | SEQ ID NO : 190 |
| ThmiPEP319a2 | *Thellungiella halophila* | ATGGAGATTCAAATTAAAAAGAAAAACTTATATATAATGAATAC ACAAAAGCTACCTAATCTGTATATATATATATATAAATATGTCTT CATTAAATTAATGGTCGTGGAATAG | SEQ ID NO : 191 |
| AtmiPEP319b1 | *Arabidopsis thaliana* | ATGGTACCTCAAATTAATCTATGGTCATCTAGGGTTATCTTGAAG ATTAGAATTGATTCTAGCACGCACAGAGAGGAAGATCATTGCATC CAGAATCACAAACATG³GCCTATCTTTTATCTTTTCTTTTTGA | SEQ ID NO : 192 |
| AtmiPEP394a1 | *Arabidopsis thaliana* | ATGTCTCTCCAATTTTATGAGAGGGTTTCCTTCAAGAACACAGTA AAATAG | SEQ ID NO : 193 |
| AtmiPEP395c1 | *Arabidopsis thaliana* | ATGACAGAGCAAGAAGAAGAAAGTCAAATGTCCACATGA | SEQ ID NO : 194 |
| AtmiPEP395e1 | *Arabidopsis thaliana* | ATGTATCTACAATATATTGATAATGTAATATCTATATATTCAAAC AATCGTCGTGTTGGTCGGATGTTTTCTAGAGTTCCTCTGAGCACTT CATTGGAGATACAATTTTTTATAAAATAG | SEQ ID NO : 195 |
| AtmiPEP397b1 | *Arabidopsis thaliana* | ATGAGCAAGGAGATATTTTTTTCCCCTGGGTTTGAATGA | SEQ ID NO : 196 |
| AtmiPEP398c1 | *Arabidopsis thaliana* | ATGAGAACACACGAGCAATCAACGGCTATAACGACGCTACGTCA TTGTTACAGCTCTCGTTTCATGTGTTCTCAGGTCACCCCTGCTGAG CTCTTTCTCTACCGTCCATGTTTTATCAACGCCGTGGCCCGTG | SEQ ID NO : 197 |
| AtmiPEP399b | *Arabidopsis thaliana* | ATGAAGAGAAACATGTAA | SEQ ID NO :198 |
| AtmiPEP399d1 | *Arabidopsis thaliana* | ATGCAATGTGAAATATGA | SEQ ID NO : 199 |
| AtmiPEP403 | *Arabidopsis thaliana* | ATGTTTTGTGCTTGA | SEQ ID NO :200 |
| AtmiPEP447a1 | *Arabidopsis thaliana* | ATGGTCATGGCTCATCATTAG | SEQ ID NO :201 |
| AtmiPEP447a2 | *Arabidopsis thaliana* | ATGATGAAACCTCGATGGAACTGCTCTCTTTATGGAATCACGGAA TGGACAAATAATCAAAATCAGAAATCGAAGCGAAAAGGGAGGA GAAAAACGCAGATTTGGAGGATTGGGGACAGATTAGATACTGTT GAATGCATCACTCTAATGCTATCAGCCTATTAA | SEQ ID NO :202 |
| AtmiPEP447b1 | *Arabidopsis thaliana* | ATGCTGCTTATCATCGTGGAGTTGGTTCTGTAA | SEQ ID NO :203 |
| AtmiPEP447b2 | *Arabidopsis thaliana* | ATGCTTTGTTTCAATTTCAGGTGCGTTAGAAGGTTTGCAGAGTAG | SEQ ID NO :204 |
| AtmiPEP447c | *Arabidopsis thaliana* | ATGTACACCTACCAGCTTGATAACTCTTTTTCGTGGTTTCTGTGTA CTCGTTTCTGTTTGTACAGATACTTCTTGTTCAATTTCAGATGCTTT AGAAGGTTTTCGGAG | SEQ ID NO :205 |
| dmmiPEP1a | *Drosophila melanogaster* | ATGTGGCGCGAAGTATGCGCACAAAAAAGTCAAACAAAAAGGCG CAATTTTATTACGGGCAACCAACGACGAAACAAAACAAAAGCCA ACCGAAAAGCAGAAACAAAGCAGCAAAAAGTTTATGAATTTTTT GTGCAGGCGCGTGAAAGATGCAAAACGAGAAAAAAACATGAAA AAAAAACATTAAAAAAAACAAAAAAAATCCAAAACAGATACCG AGCTGTATCCGAAAACGAGTGGGGAAAGGGGTTTCCCAGTCACA TATAA | SEQ ID NO :206 |
| DmmiPEP1b | *Drosophila melanogaster* | ATGCGCACAAAAAAGTCAAACAAAAAGGCGCAATTTTATTACGG GCAACCAACGACGAAACAAAACAAAAGCCAACCGAAAAGCAGA AACAAAGCAGCAAAAAGTTTATGA | SEQ ID NO :207 |
| DmmiPEP8 | *Drosophila melanogaster* | ATGGAGCCTGGCTTTGTTTTTGTTTTATTTCCAACCCACTTGAGCA CACAGCACACACAGAGAGAAAAATCAATACTCGTTATGGGATTA AATTTACAAAGCGCAAAGCAAAGCGACAAACAAAATTCAAAAGA AAGAAAAAAAAACACTCAAATAAACTCACAAAGAATTCCTTATC GCCAAGGGGGCCAATGTTCTAAGGTTCTTTCGCCTTGA | SEQ ID NO :208 |
| HsmiPEP155 | *Homo sapiens* | TGGAGATGGCTCTAATGGTGGCACAAACCAGGAAGGGGAA ATCTGTGGTTTAA | SEQ ID NO :356 |
| AtmiPEP157c | *Arabidopsis thaliana* | ATGATGTTGCATATCACACATAGGTTTGAGAGTGATGTTGGTTGT TGA | SEQ ID NO : 387 |
| AtmiPEP157d | *Arabidopsis thaliana* | ATGCTGTATGTATAG | SEQ ID NO : 388 |
| AtmiPEP160c | *Arabidopsis thaliana* | ATGTTCATGCGTAGAGGTTTGGTATACAACAATATATACATATAA | SEQ ID NO : 389 |
| AtmiPEP164b | *Arabidopsis thaliana* | ATGATGAAGGTGTGTGATGAGCAAGATGGAGAAGCAGGGCACGT GCATTACTAG | SEQ ID NO : 390 |
| AtmiPEP166c | *Arabidopsis thaliana* | ATGAAGAAGAGAATCACTCGAATTAATTTGGAAGAACAAATTAA GAAAACCCTAGATGATTCTCGGACCAGGCTTCATTCCCCCTAA | SEQ ID NO : 391 |
| AtmiPEP166d | *Arabidopsis thaliana* | ATGAAGAAGATCGGTAGtATTGATTCATTTTAA | SEQ ID NO : 392 |
| AtmiPEP169a | *Arabidopsis thaliana* | ATGACTTGCCGATTTAAATGA | SEQ ID NO : 393 |
| AtmiPEP169h1 | *Arabidopsis thaliana* | ATGGTGACATGA | SEQ ID NO : 394 |
| AtmiPEP169h2 | *Arabidopsis thaliana* | ATGAAGAATGAGAACTTGTGTGGTAGCCAAGGATGA | SEQ ID NO : 395 |
| AtmiPEP169n | *Arabidopsis thaliana* | ATGAAGTGTATGATGAAGAAGAGAGGTCTAACATGGCGGAAAGC GTCATGTTTAGTAGCCAAGGATGACTTGCCTGATCTTTTTCGCCTC CACGATTCAATTTCAAATTCATGCATTTTGGATTATTATACCTTTT AA | SEQ ID NO : 396 |
| AtmiPEP170 | *Arabidopsis thaliana* | ATGTTTCCGAGAGAGTCCCTCTGA | SEQ ID NO : 397 |
| AtmiPEP396a | *Arabidopsis thaliana* | ATGACCCTCTCTGTATTCTTCCACAGCTTTCTTGAACTGCAAAACT TCTTCAGATTTTTTTTTTTTTCTTTTGATATCTCTTACGCATAA | SEQ ID NO : 398 |
| AtmiPEP399c | *Arabidopsis thaliana* | ATGTCACTTGCCAAAGGAGAGTTGCCCTGTCACTGCTTCCGCTTA AACACAGTCTATAACCGGTTCTGCTAA | SEQ ID NO : 399 |
| MtmiPEP169d | *Medicago truncatula* | ATGGTCAAAGAGTCTTTCATGGAGAGGTTGAAAGTGAGATGA | SEQ ID NO : 425 |

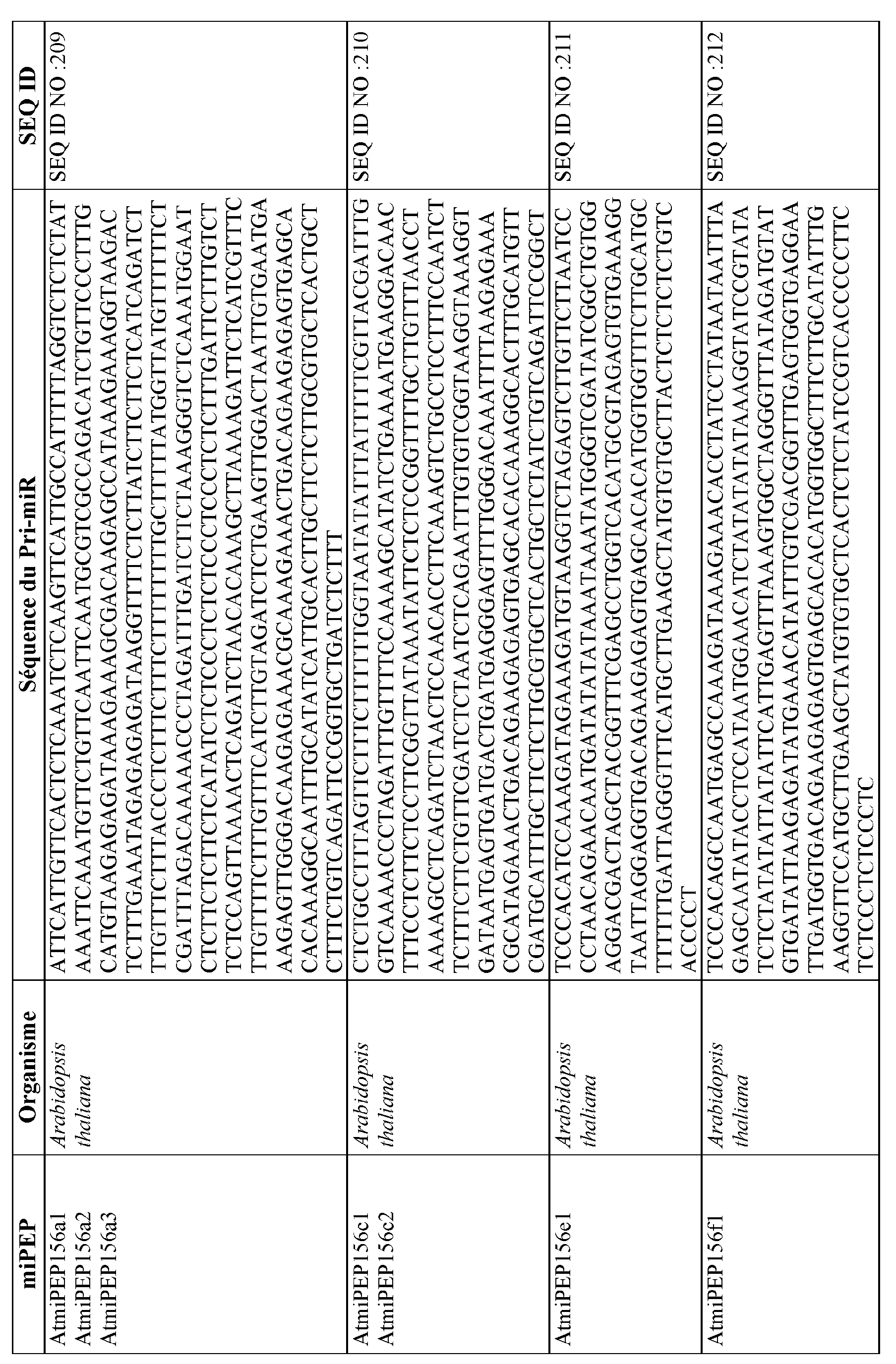

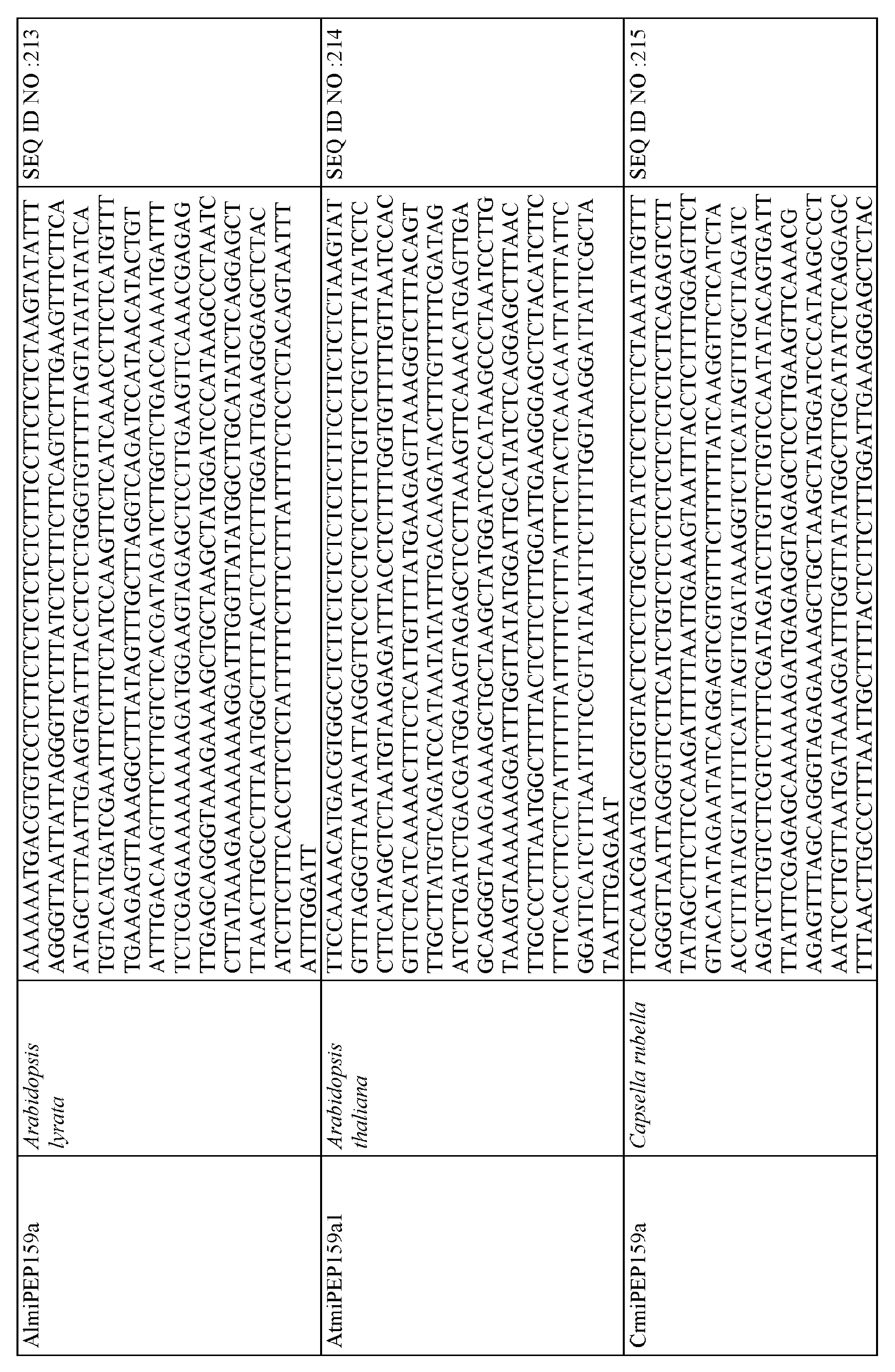

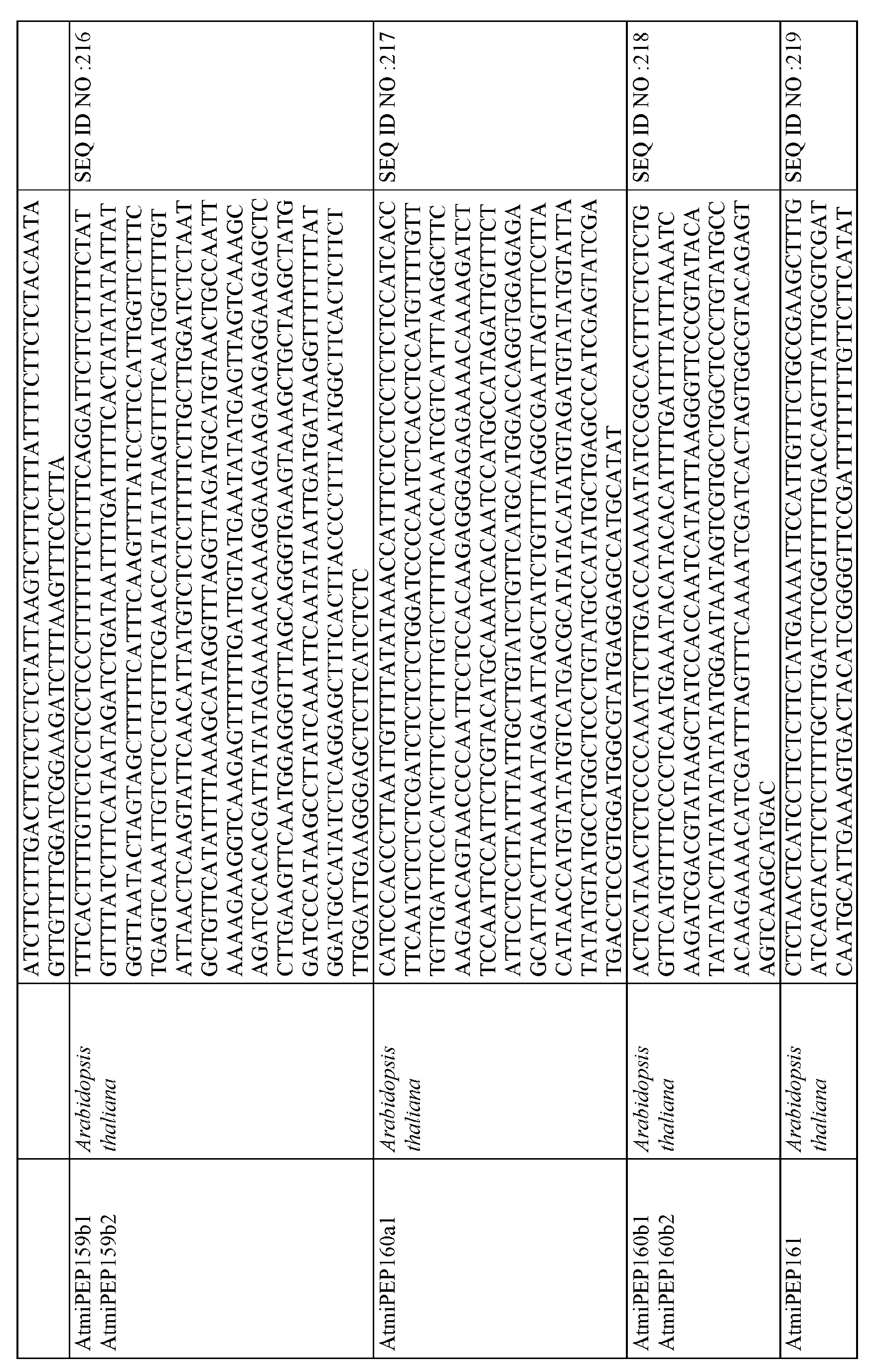

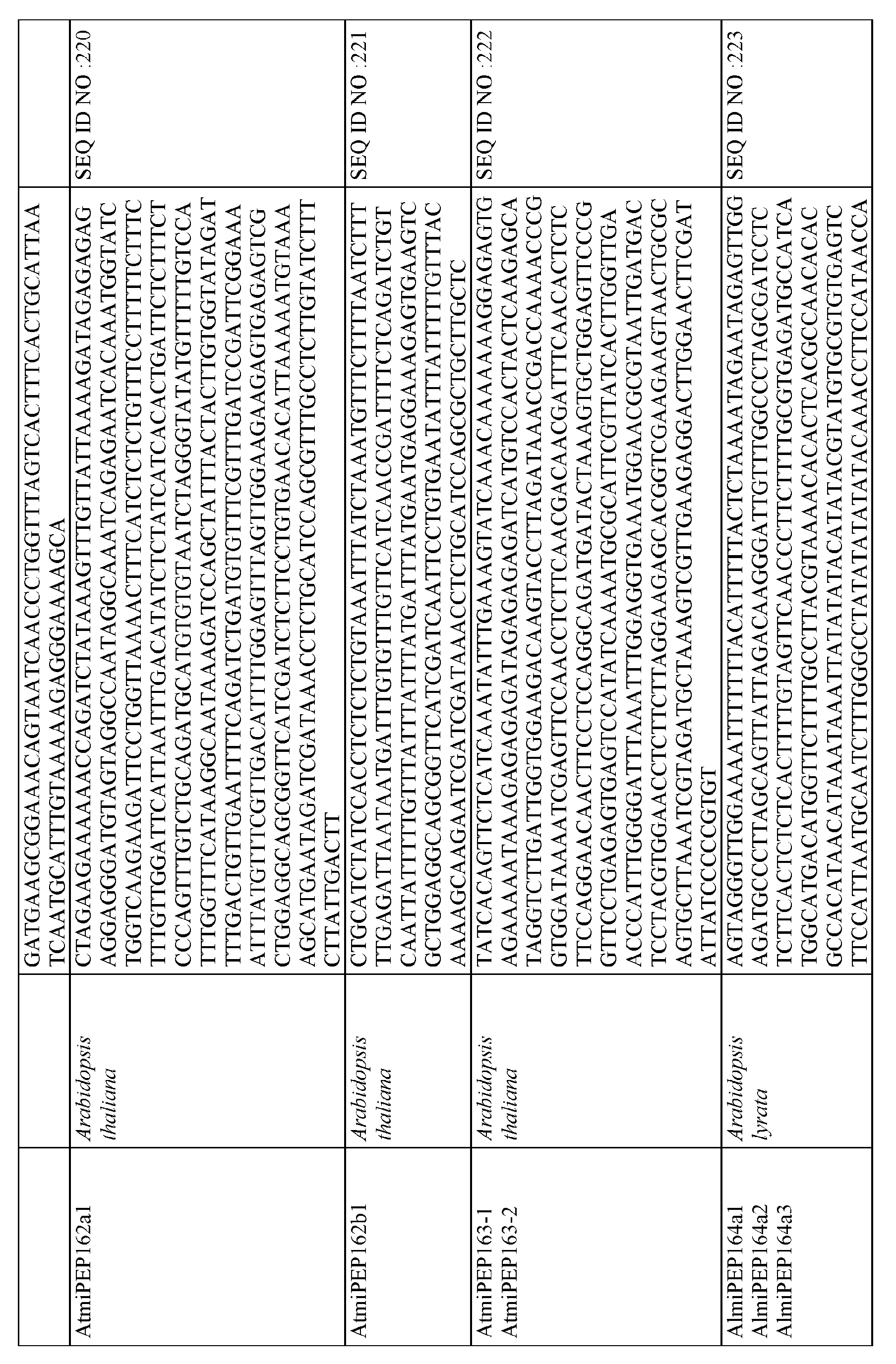

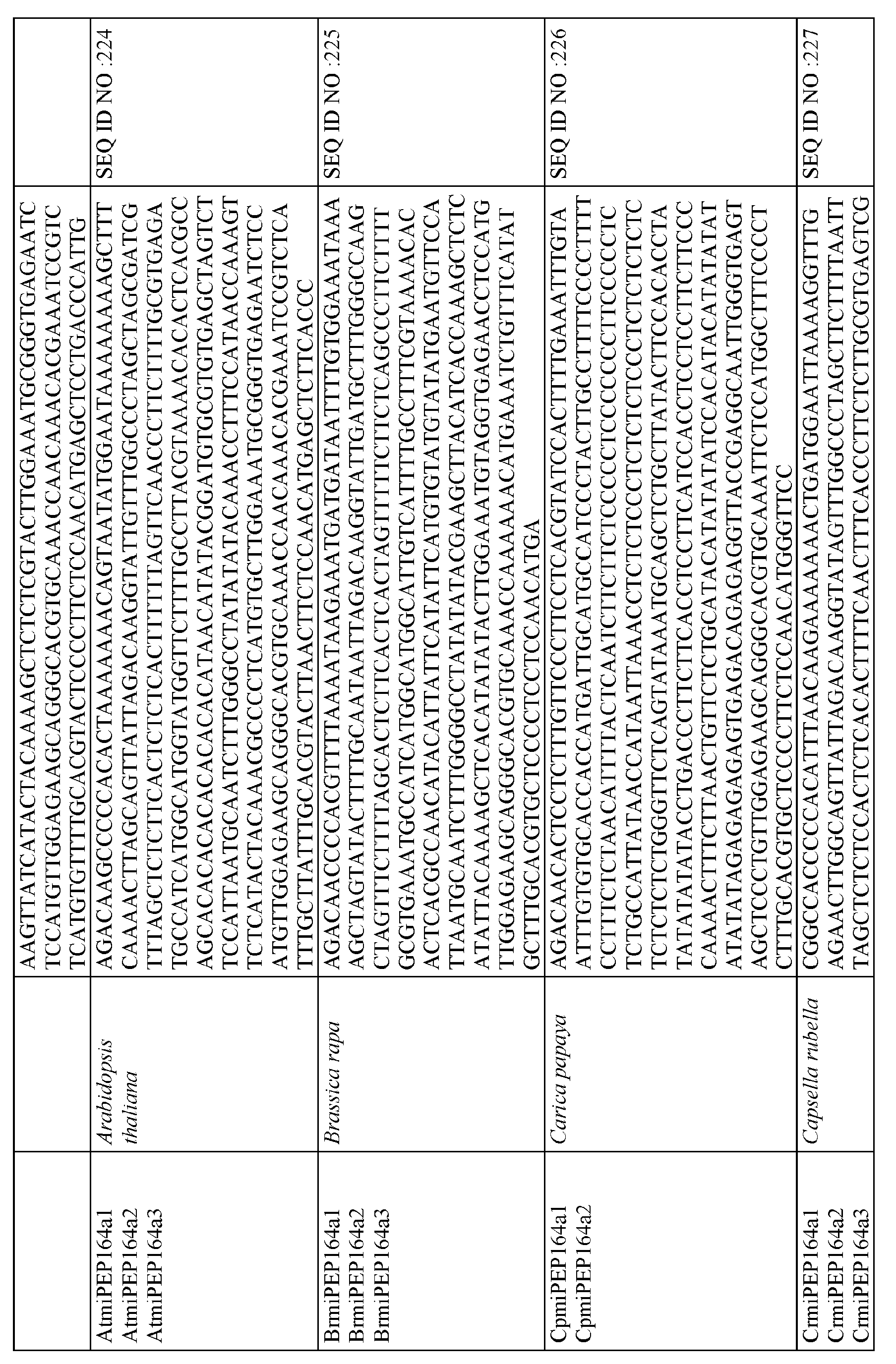

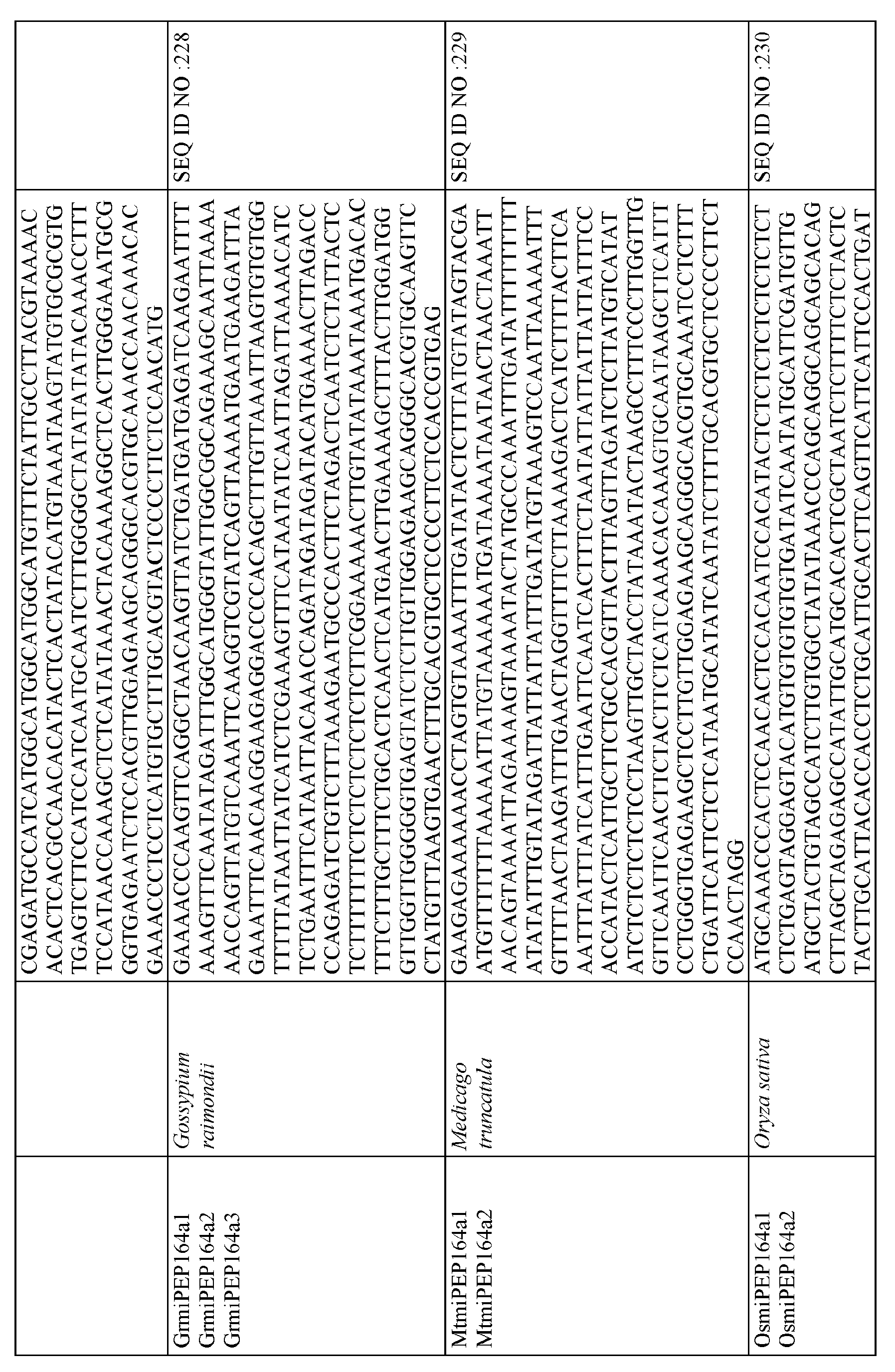

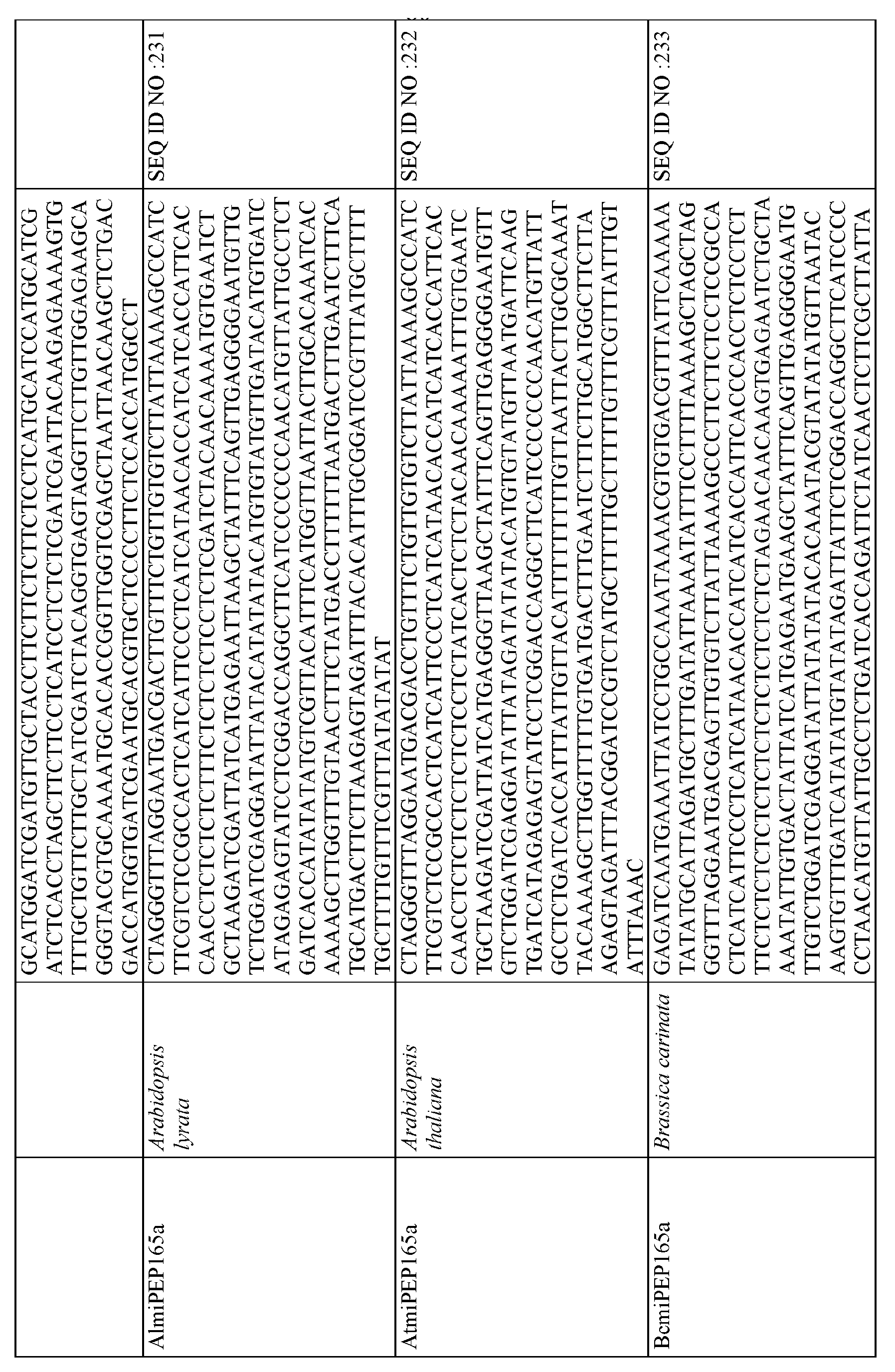

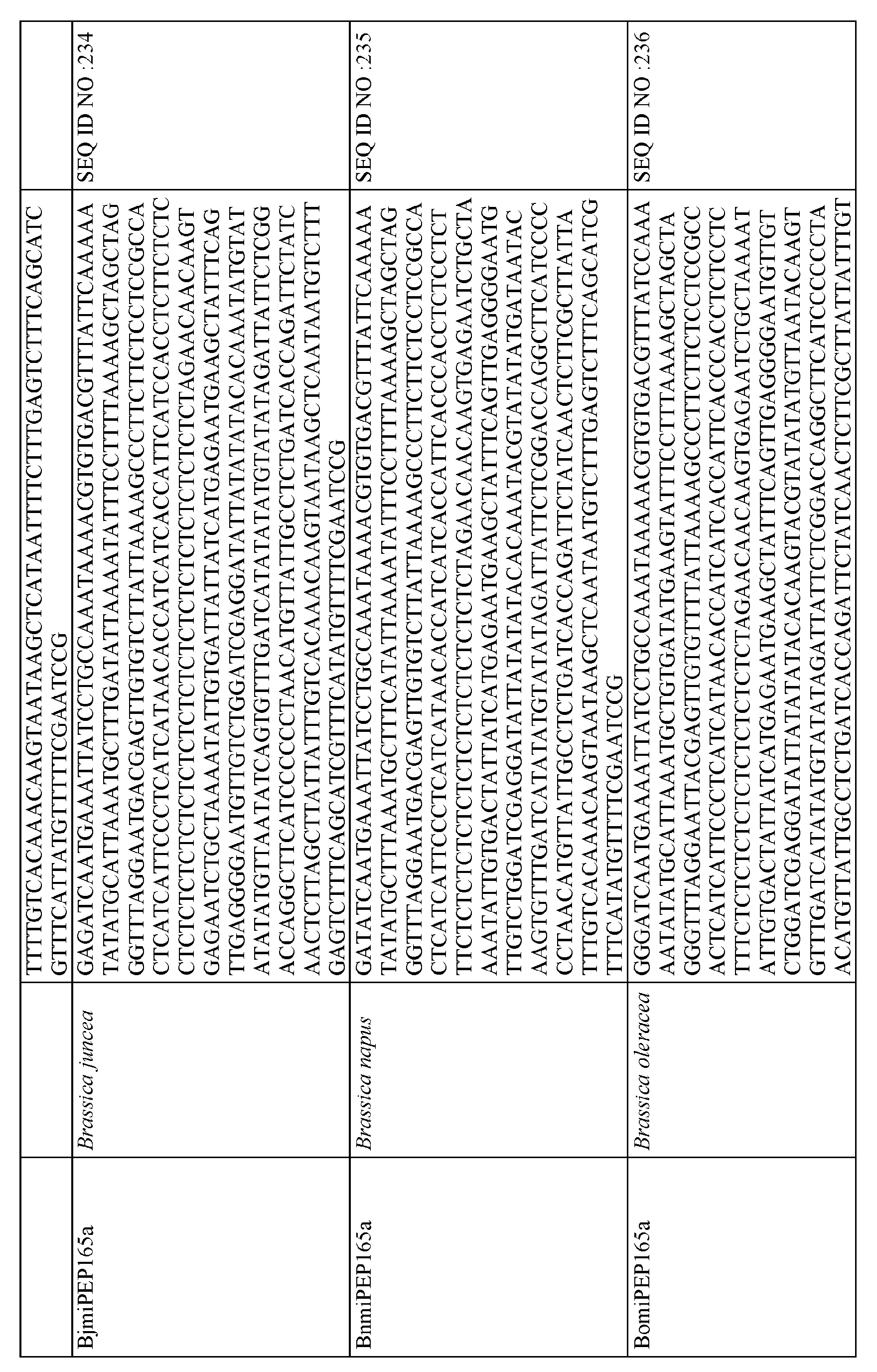

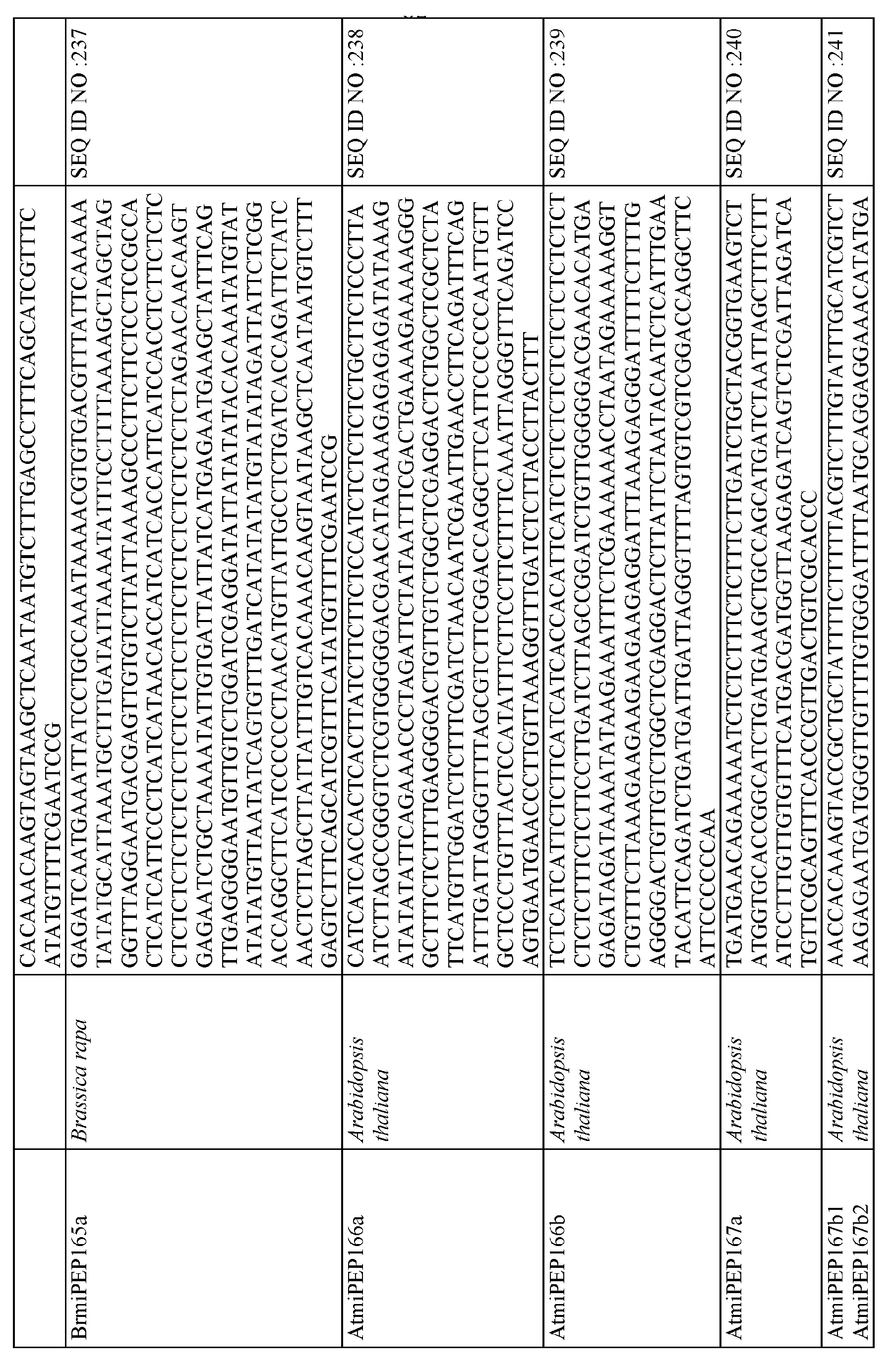

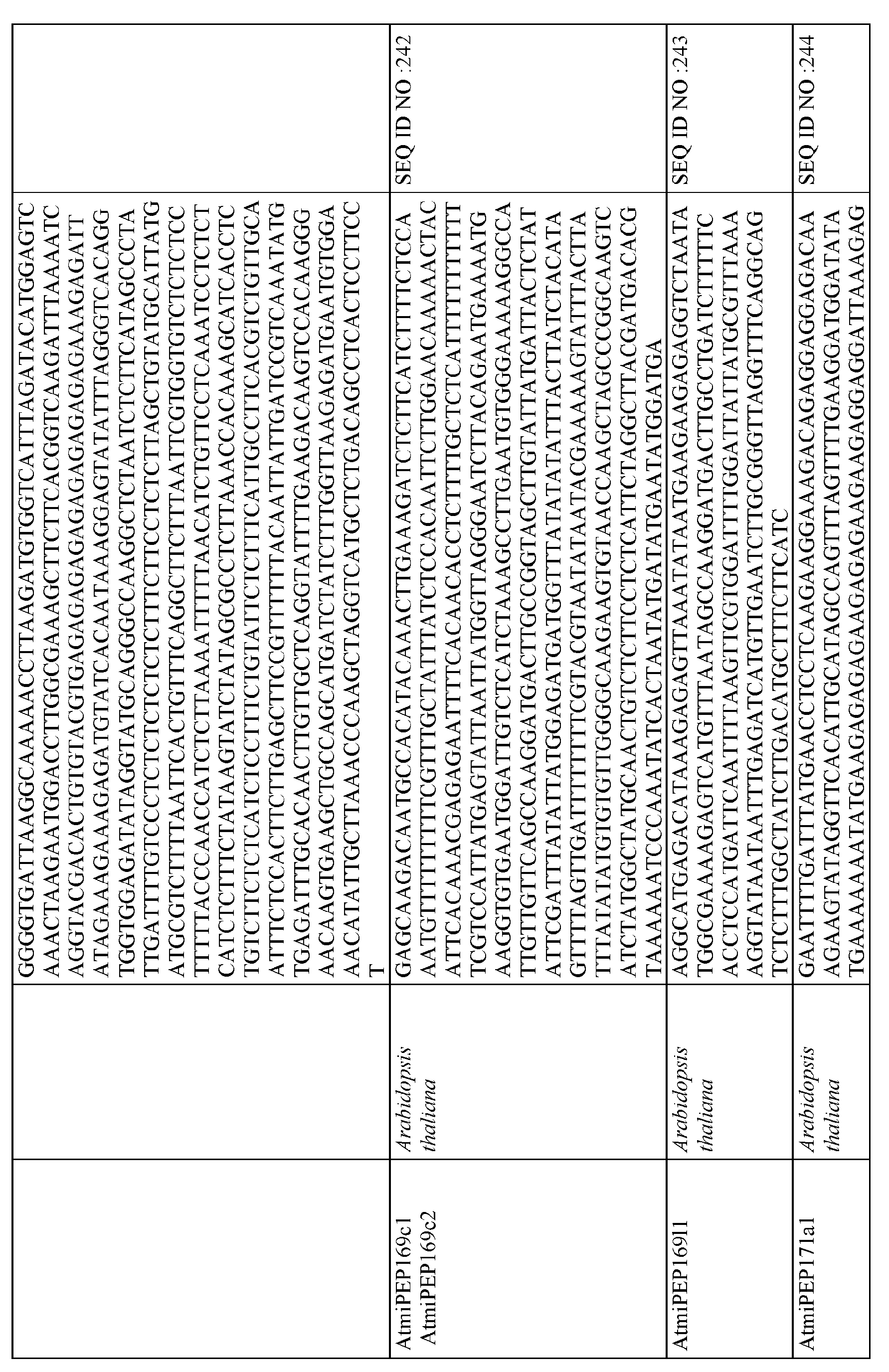

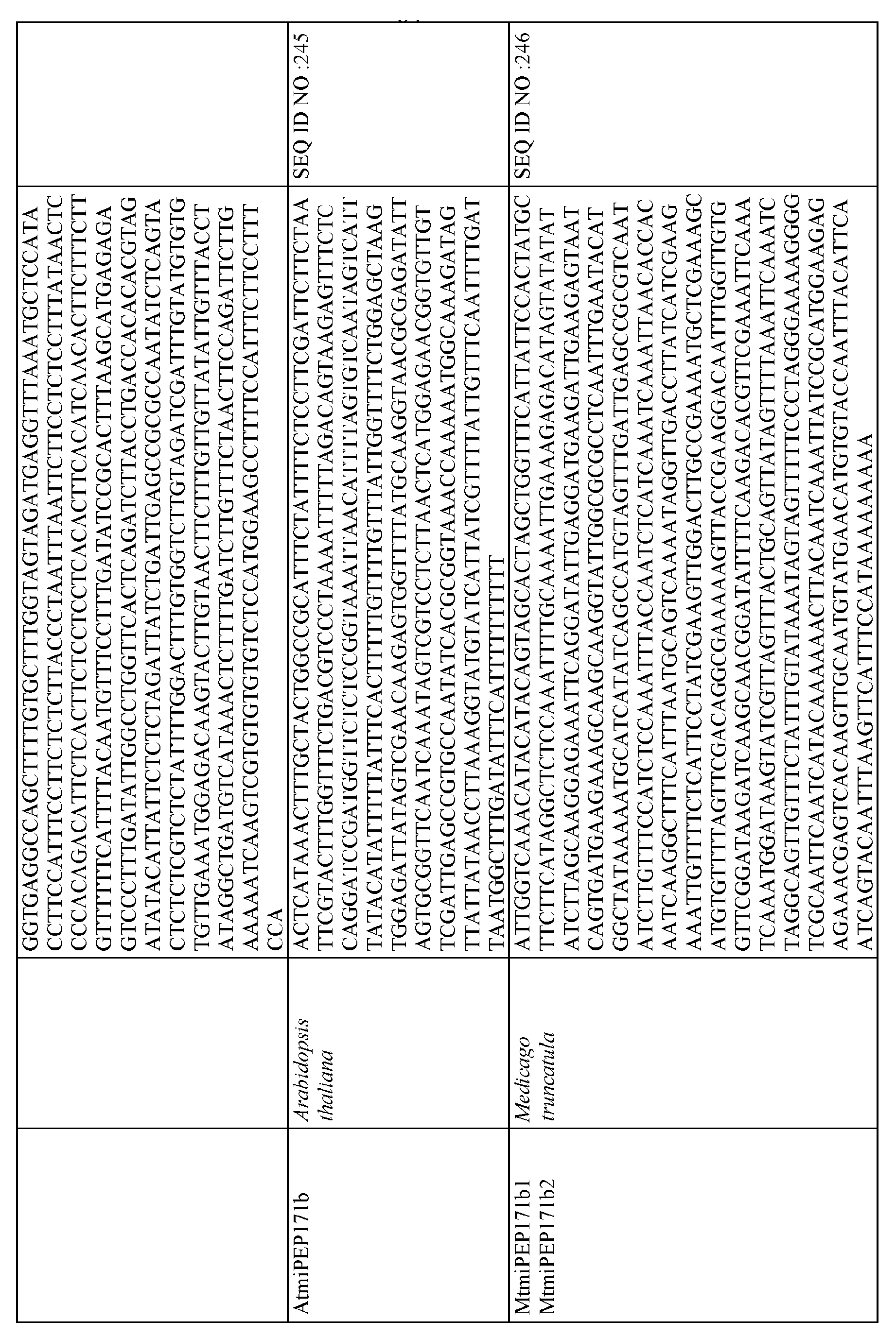

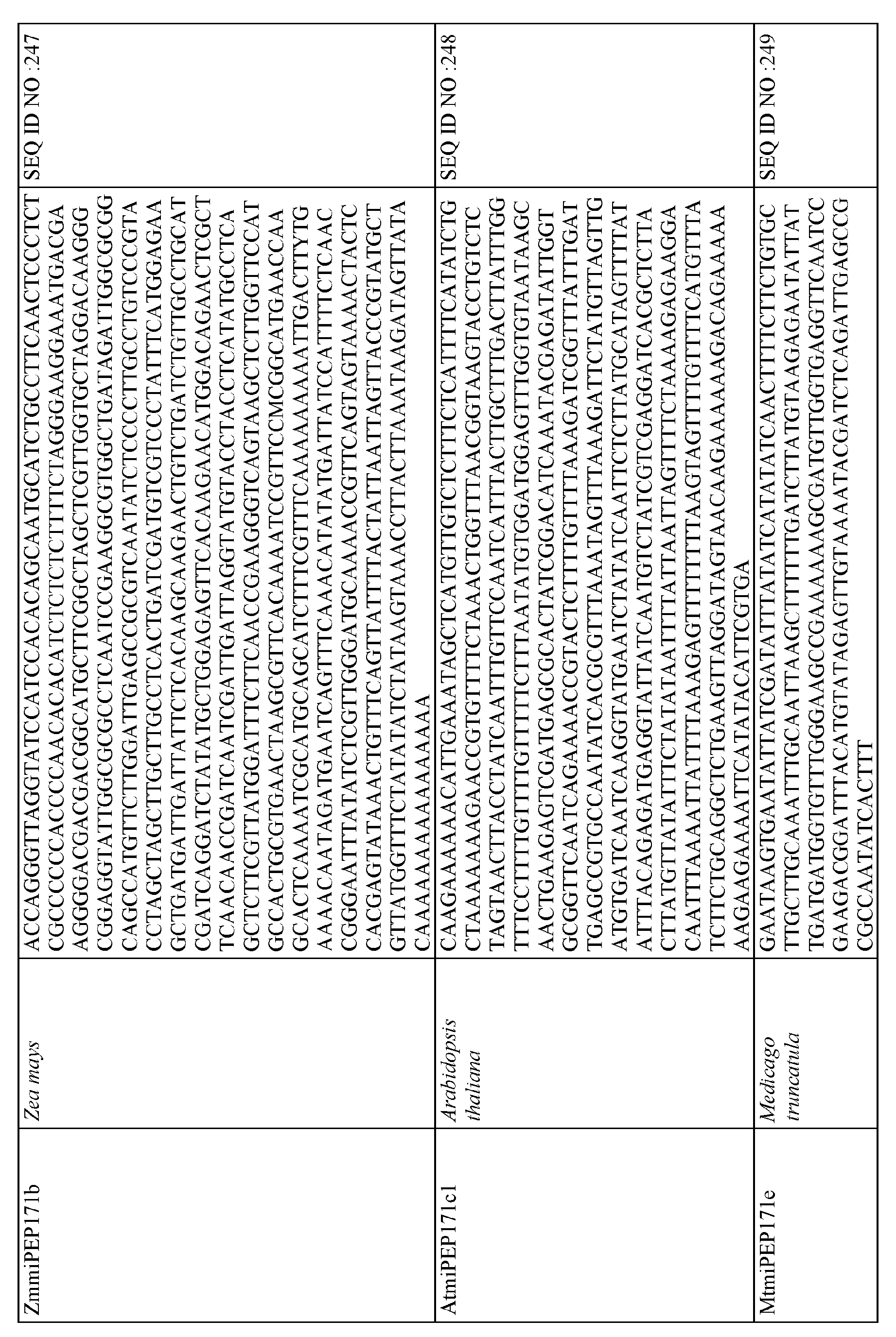

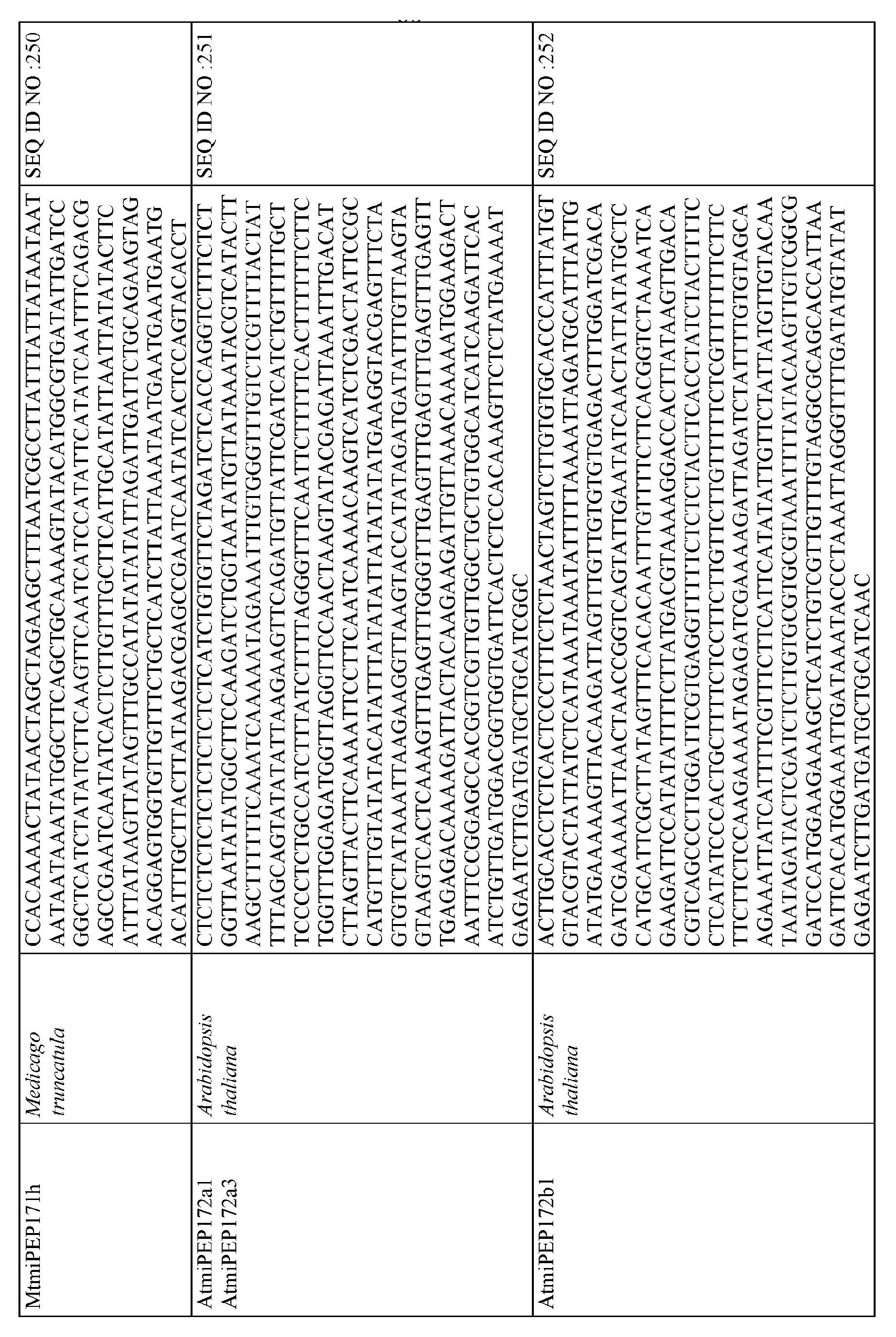

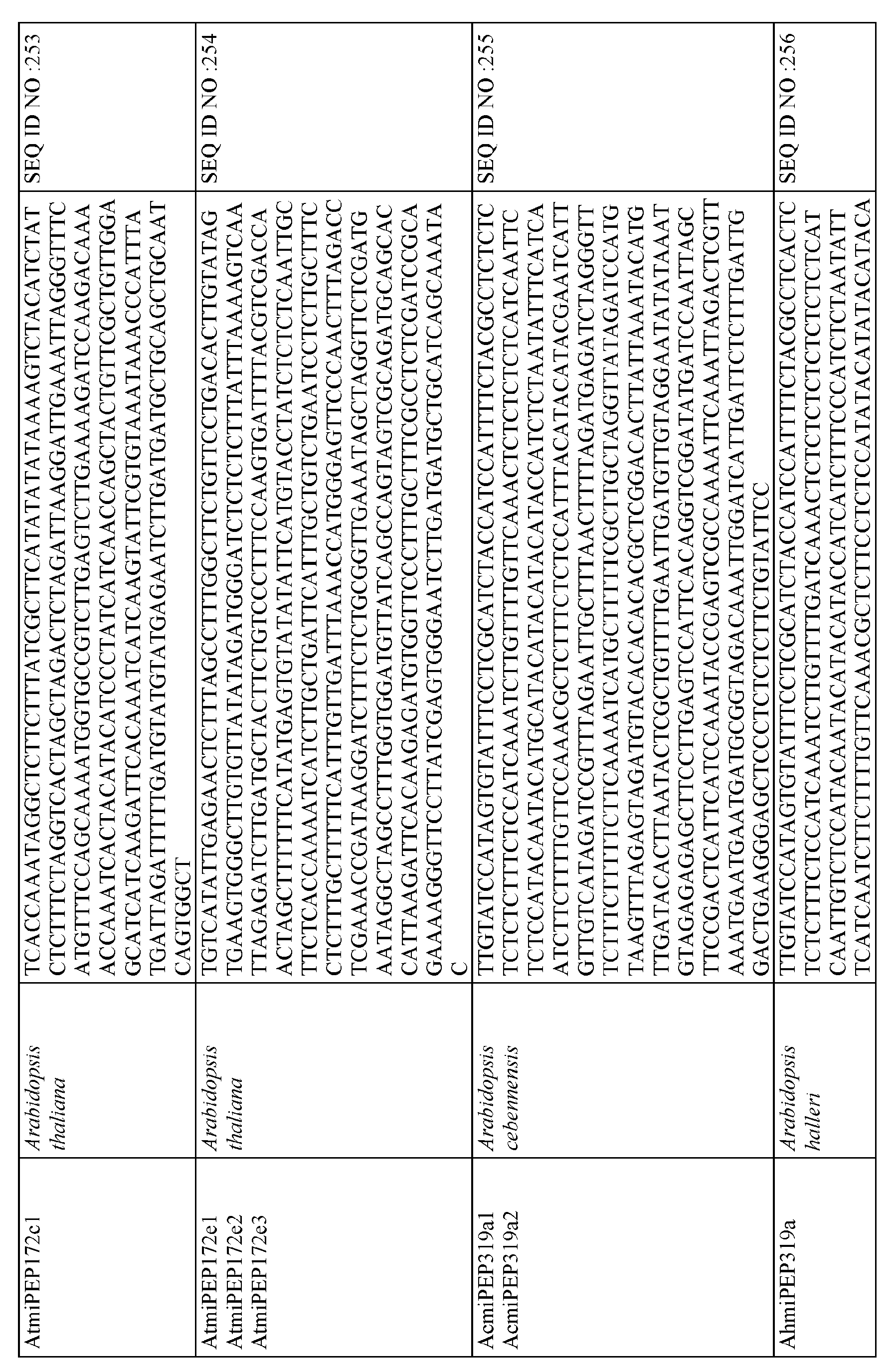

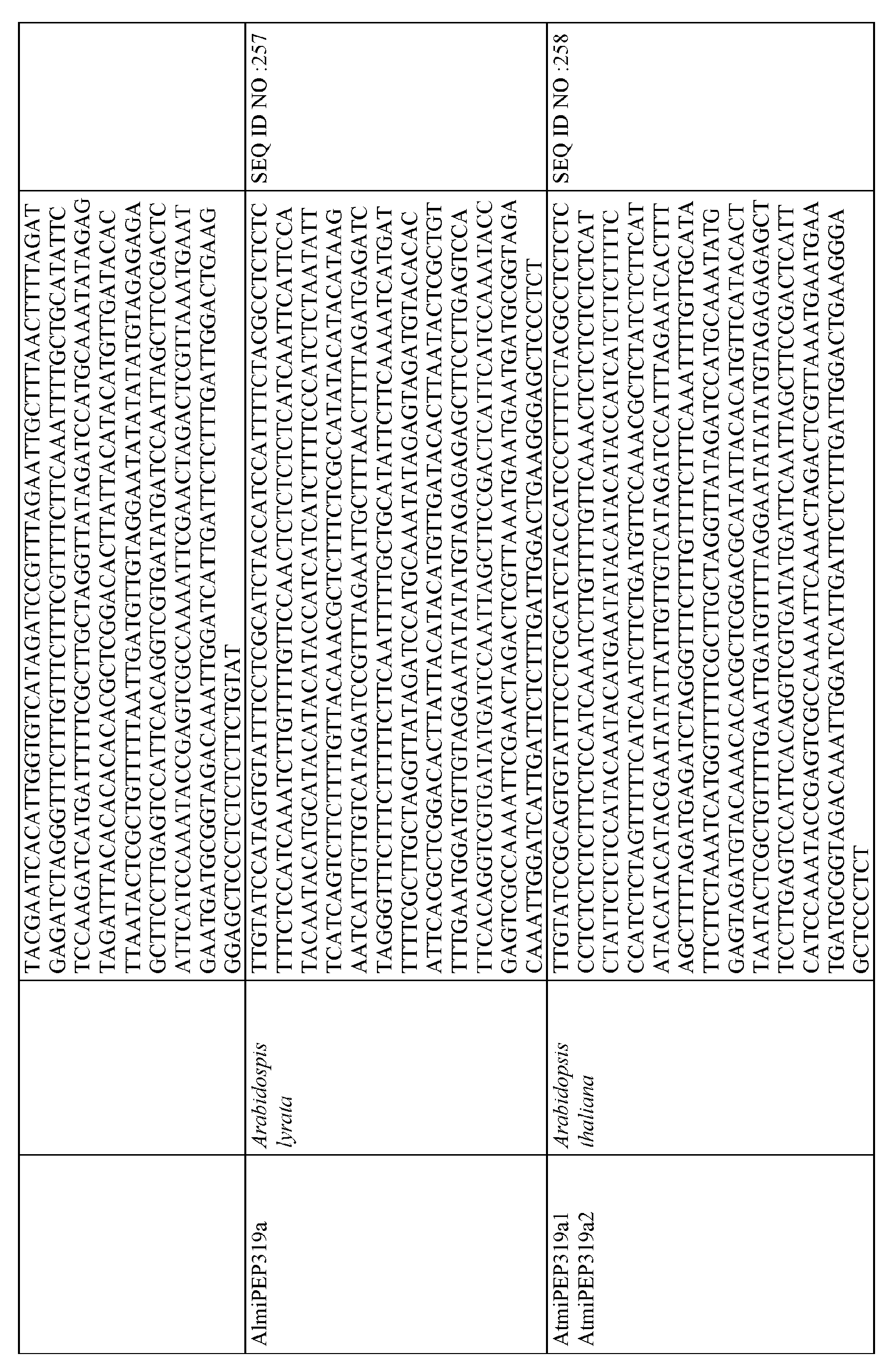

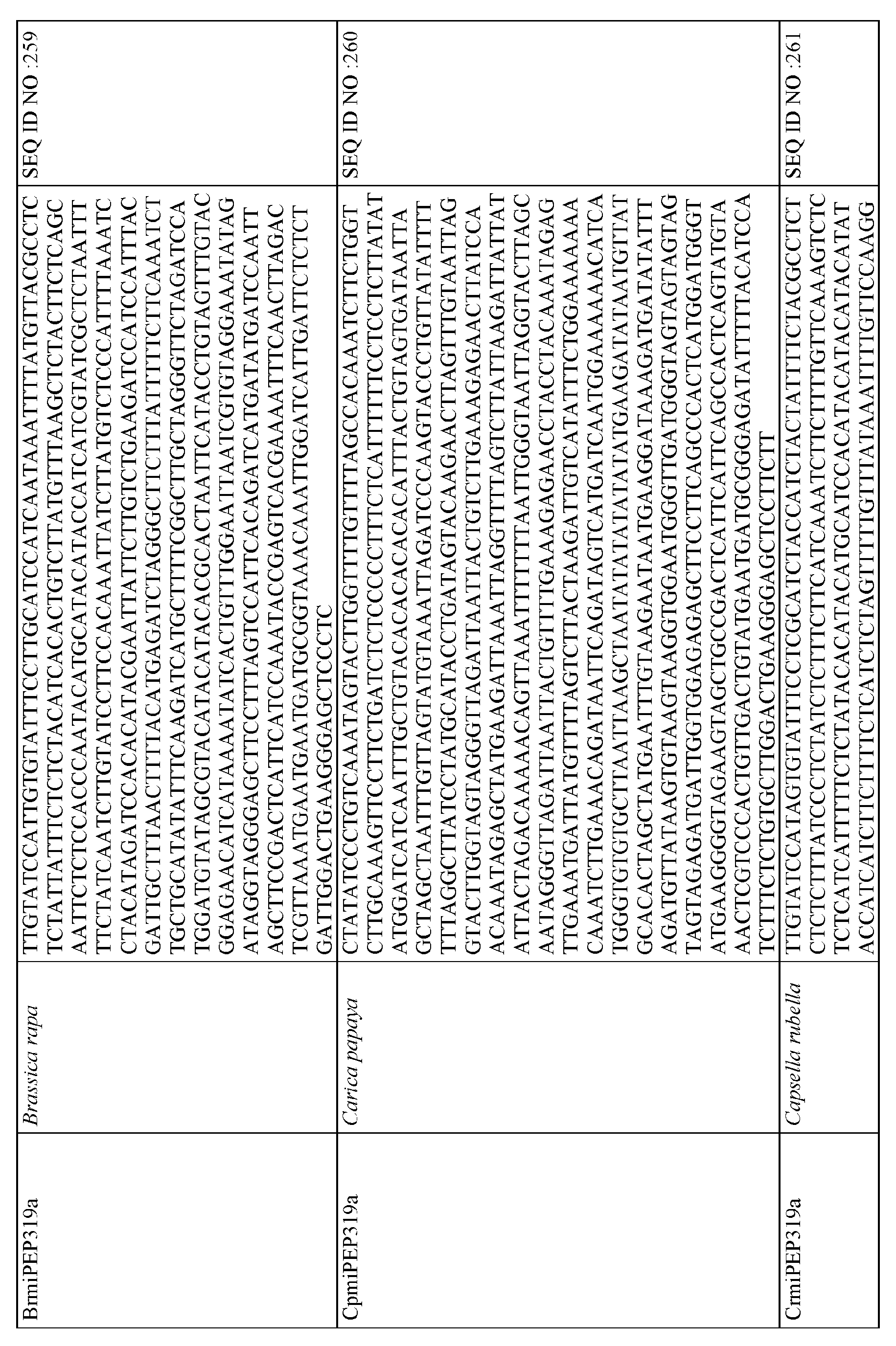

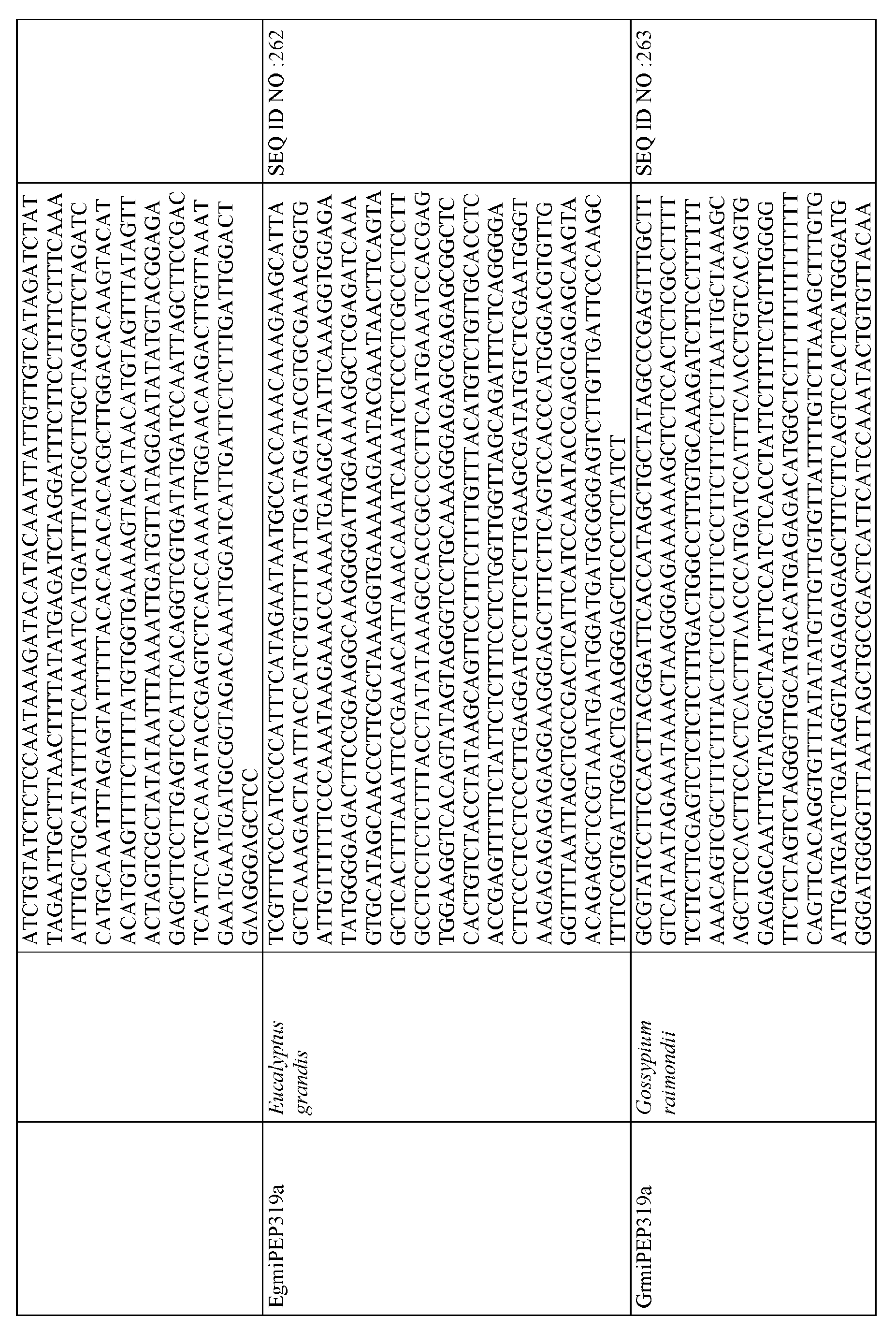

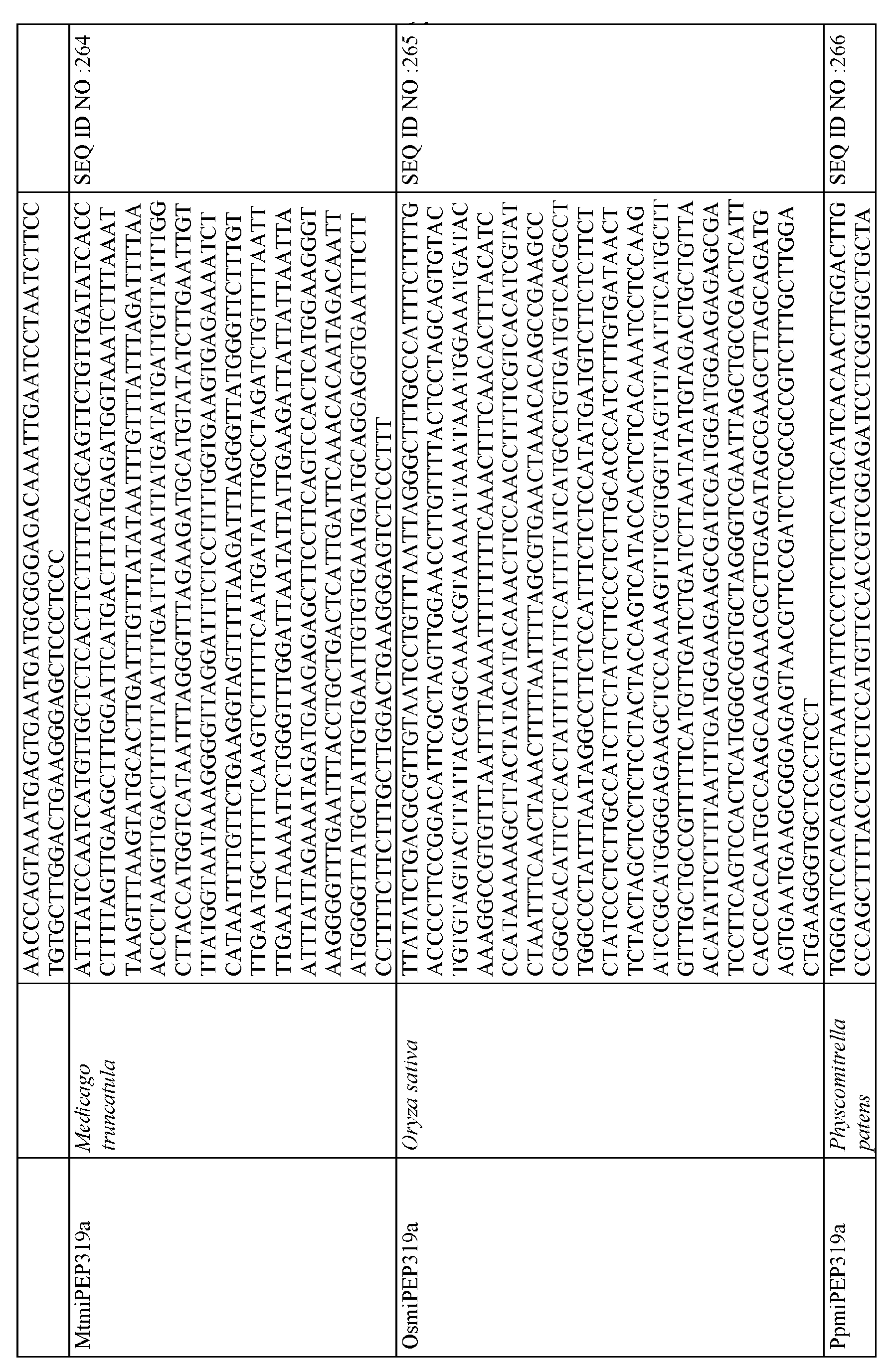

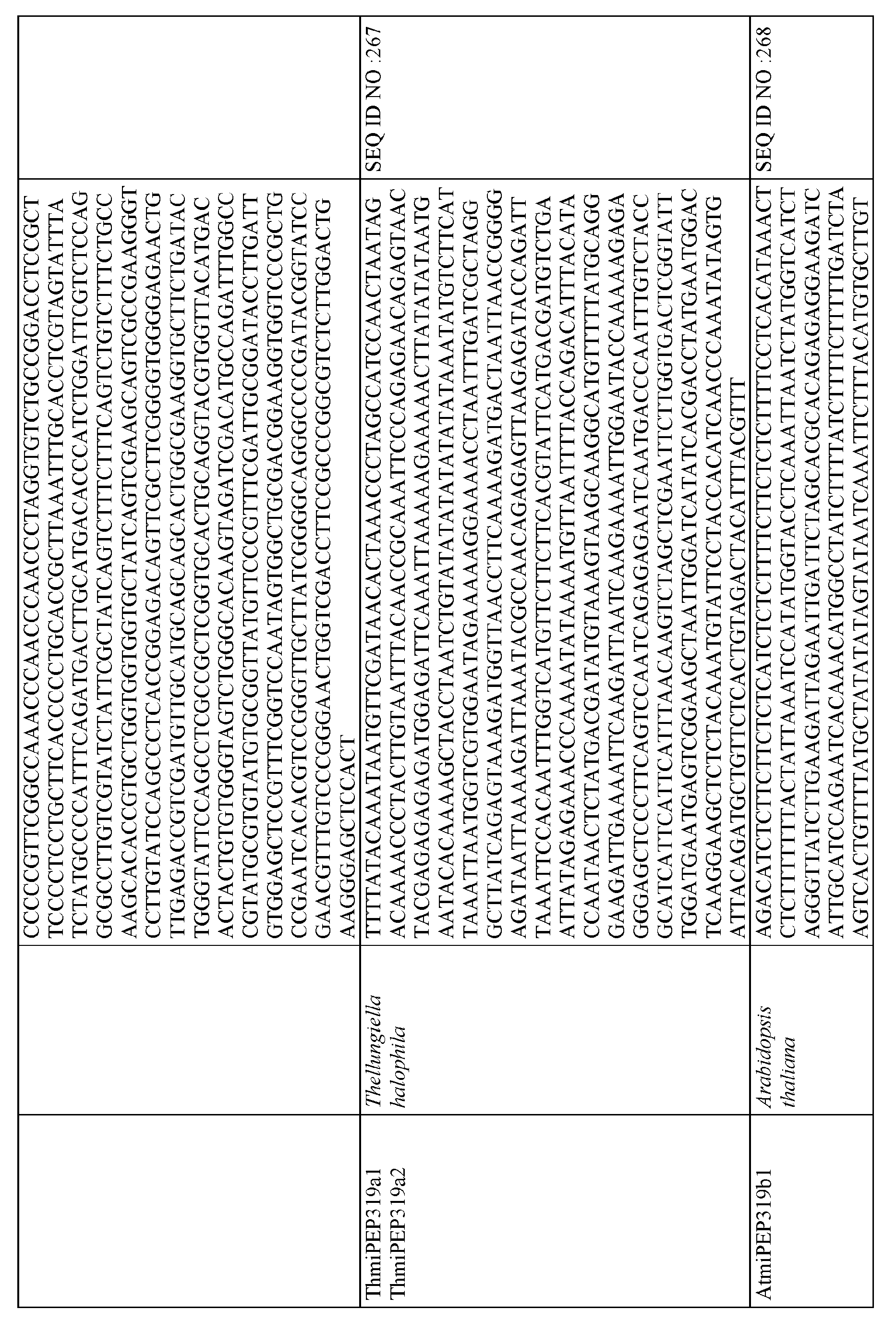

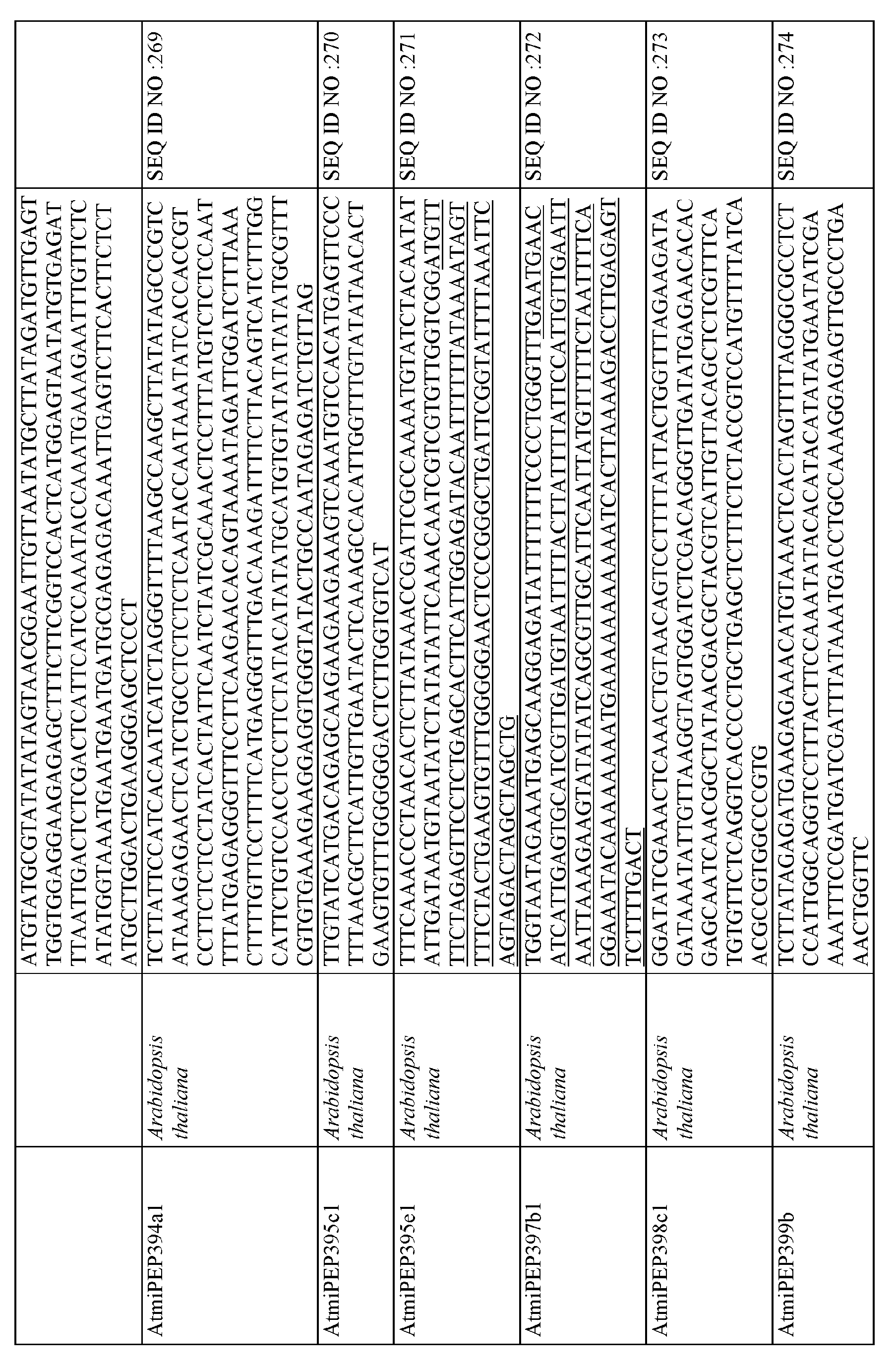

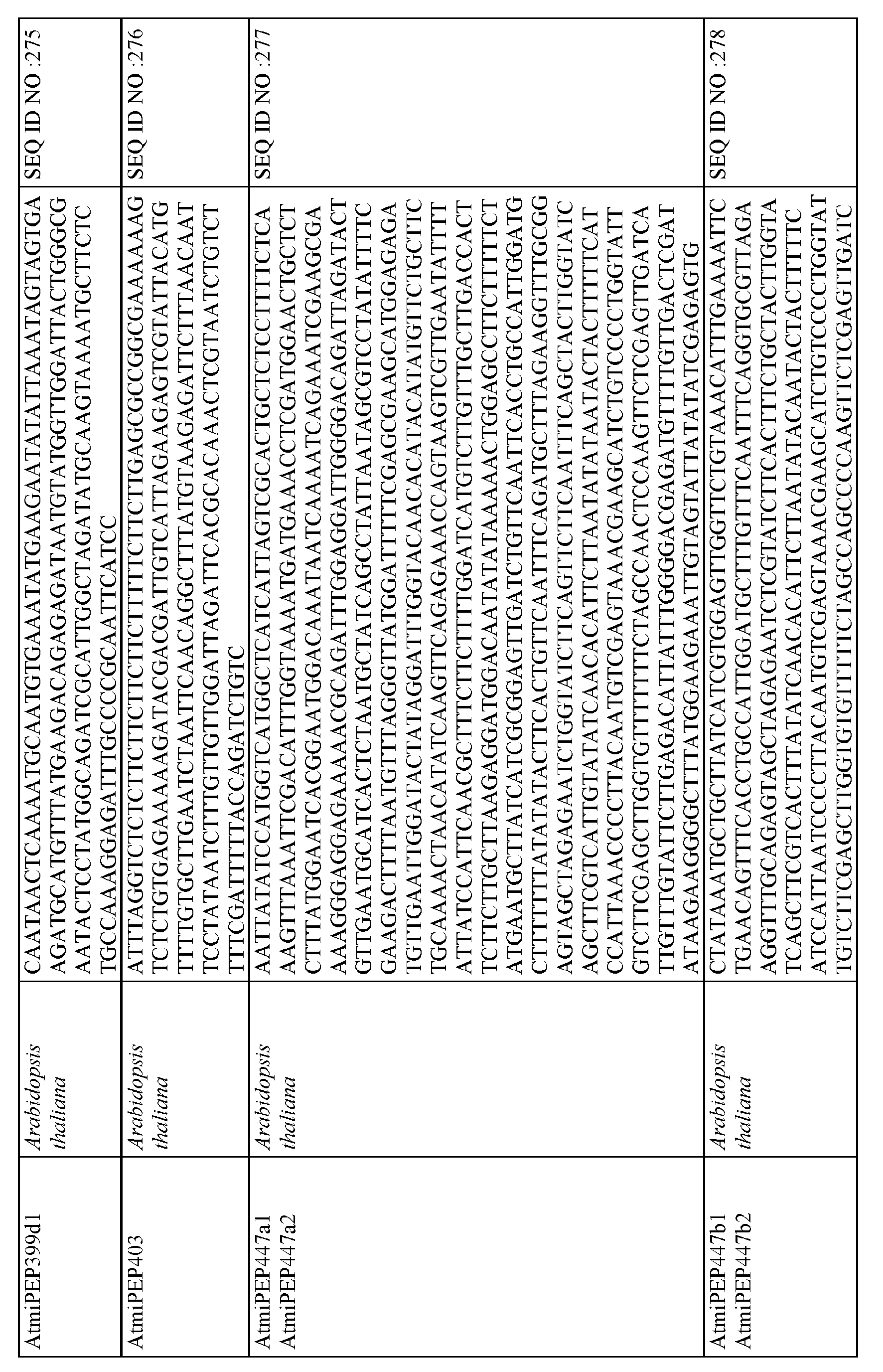

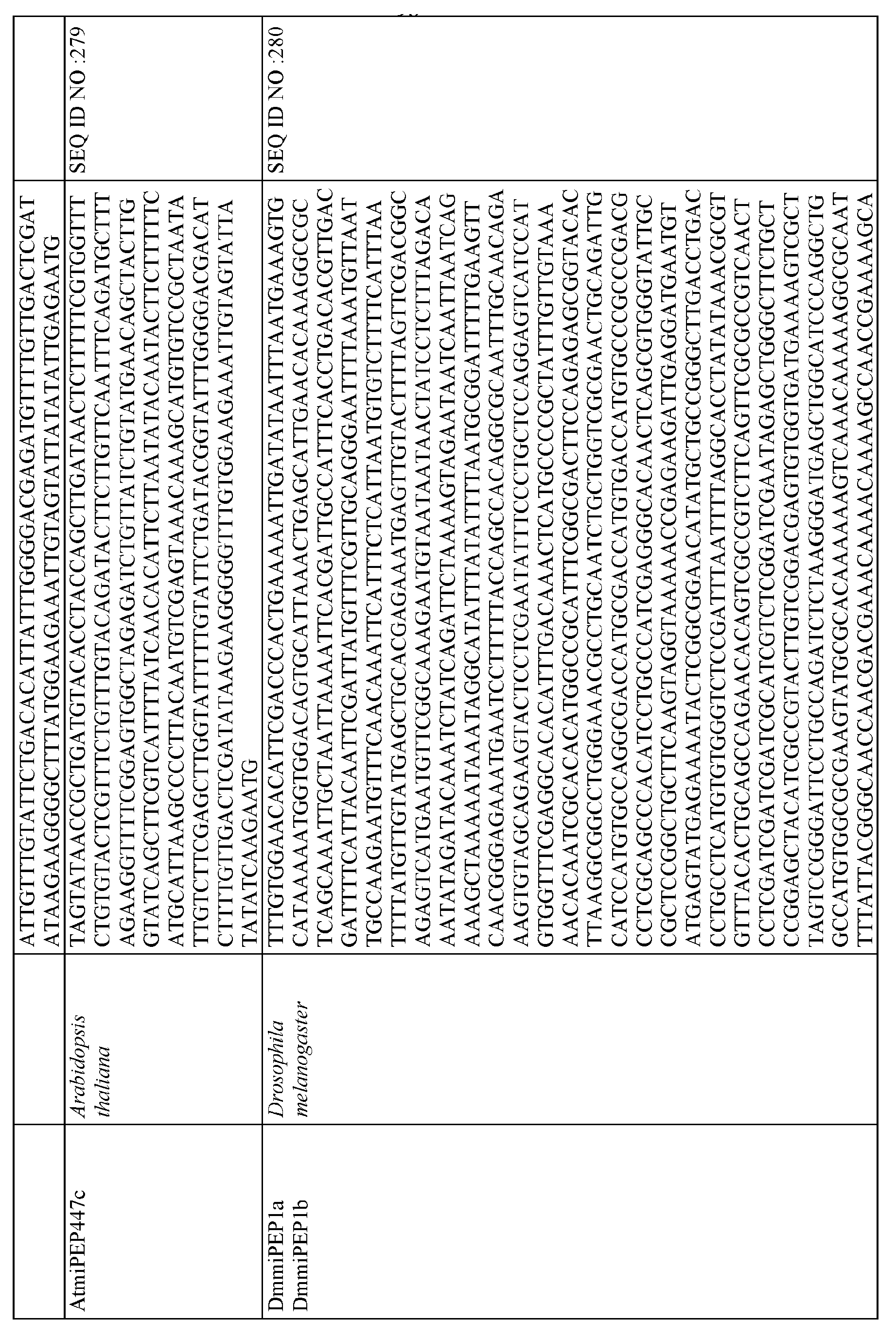

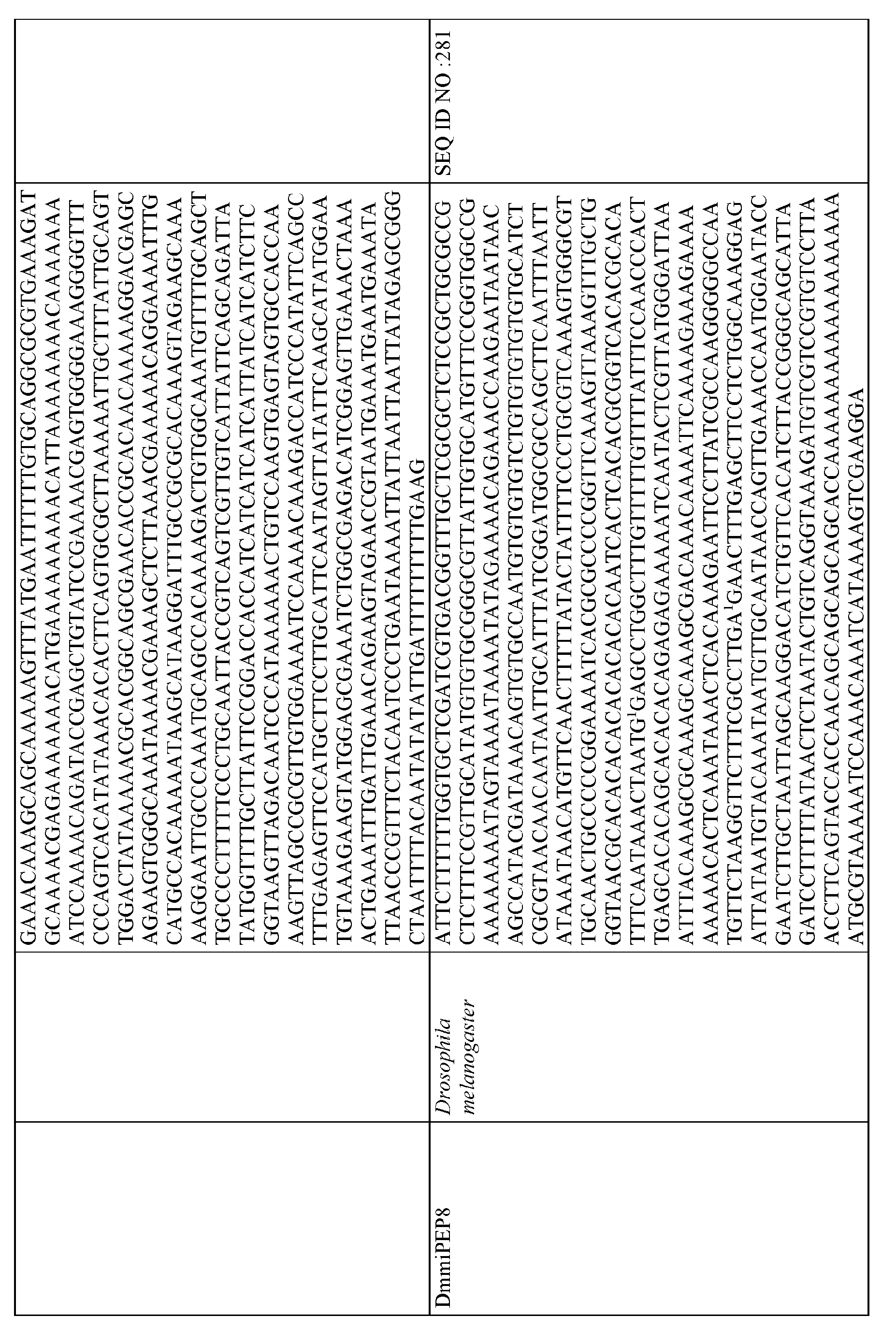

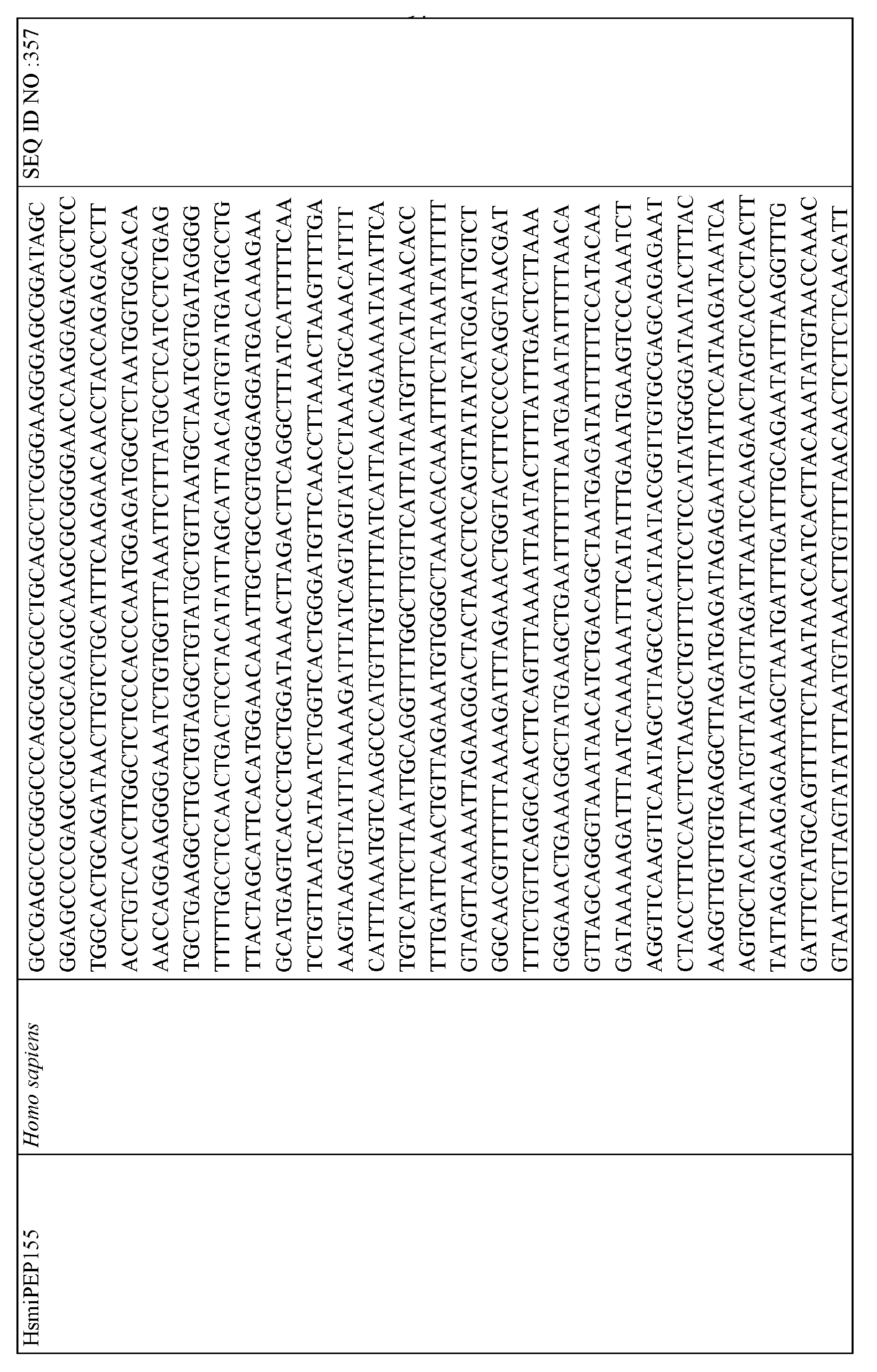

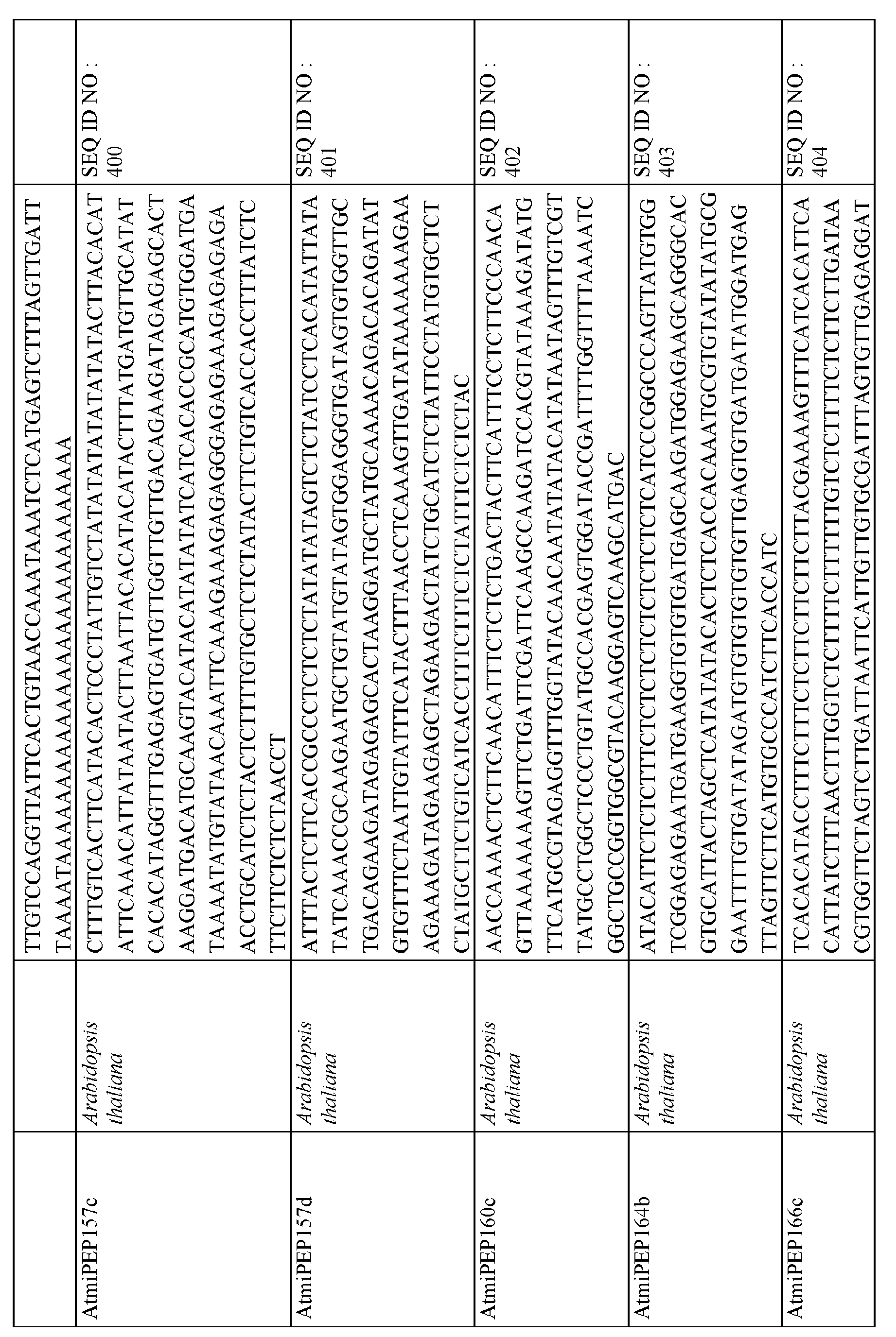

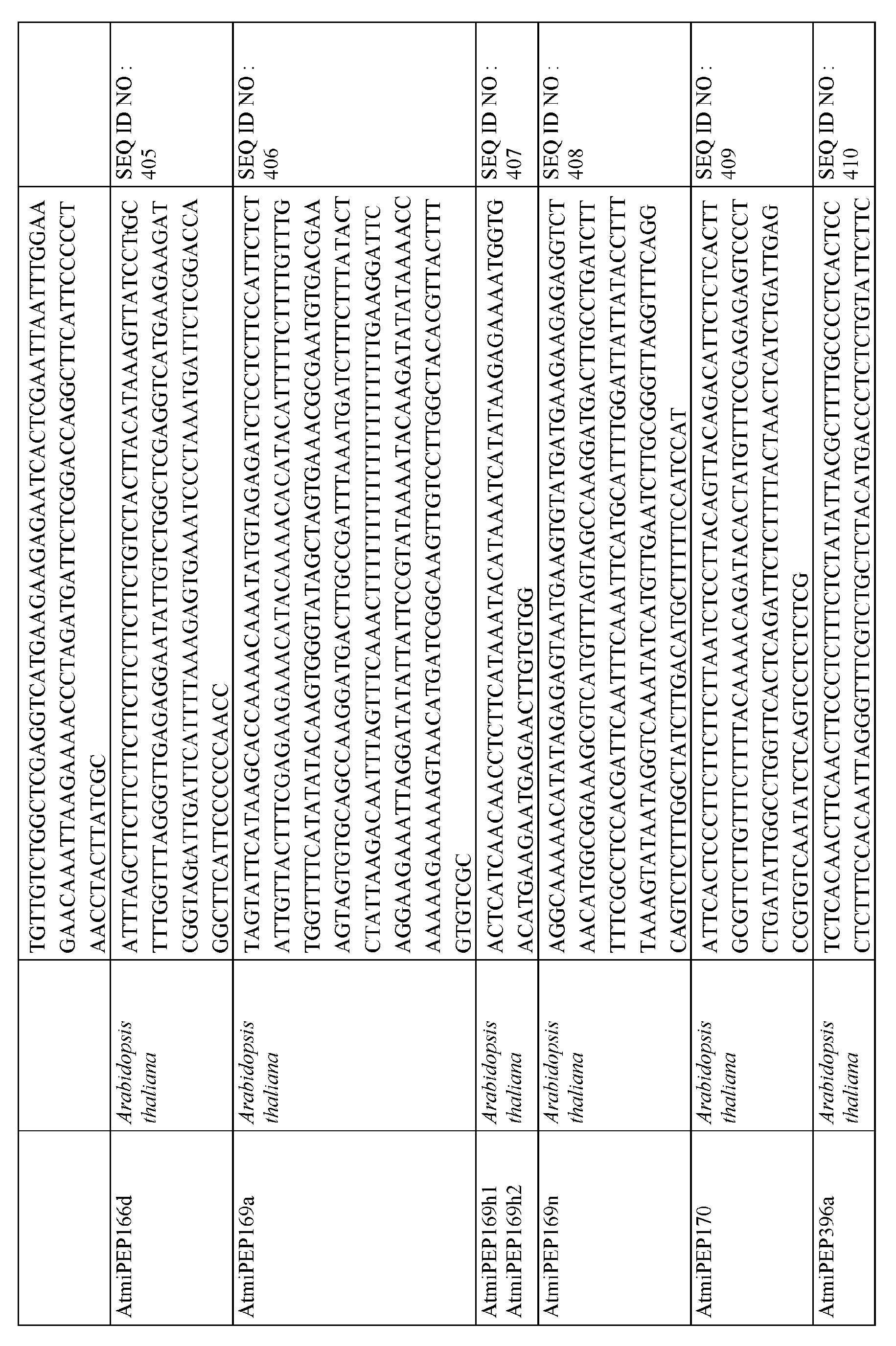

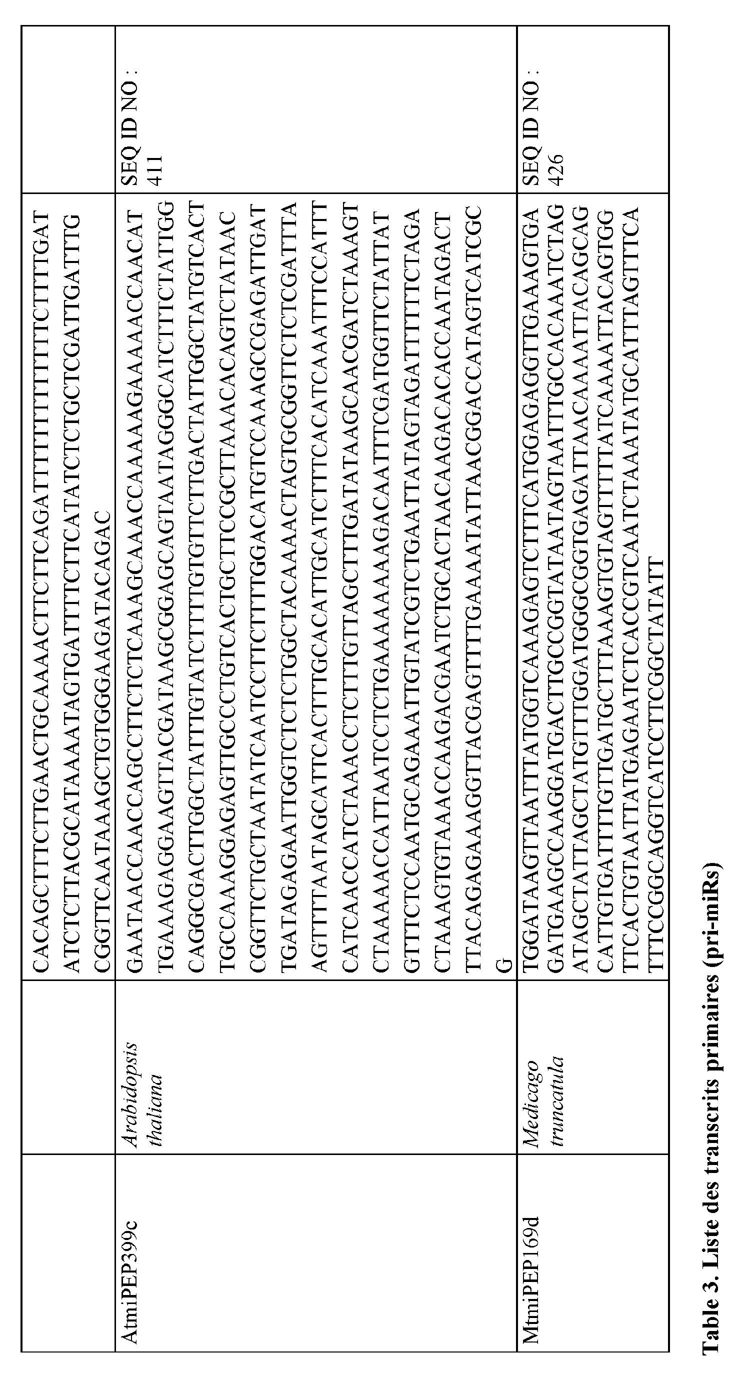

**Table 4. Liste des microARNs (miRs)**

| **miPEP** | **Organisme** | **Séquence du miR** | **SEQ ID** |
|---|---|---|---|
| AtmiPEP156a1 | *Arabidopsis thaliana* | ugacagaagagagugagcac | SEQ ID NO :282 |
| AtmiPEP156a2 | | | |
| AtmiPEP156a3 | | | |
| AtmiPEP156c1 | *Arabidopsis thaliana* | ugacagaagagagugagcac | SEQ ID NO :283 |
| AtmiPEP156c2 | | | |
| AtmiPEP156e1 | *Arabidopsis thaliana* | ugacagaagagagugagcac | SEQ ID NO :284 |
| AtmiPEP156f1 | *Arabidopsis thaliana* | ugacagaagagagugagcac | SEQ ID NO :285 |
| AlmiPEP159a | *Arabidopsis lyrata* | uuuggauugaagggagcucua | SEQ ID NO :286 |
| AtmiPEP159a1 | *Arabidopsis thaliana* | uuuggauugaagggagcucua | SEQ ID NO :287 |
| CrmiPEP159a | *Capsella rubella* | uuuggauugaagggagcucua | SEQ ID NO :288 |
| AtmiPEP159b1 | *Arabidopsis thaliana* | uuuggauugaagggagcucuu | SEQ ID NO :289 |
| AtmiPEP159b2 | | | |
| AtmiPEP160a1 | *Arabidopsis thaliana* | ugccuggcucccuguaugcca | SEQ ID NO :290 |
| AtmiPEP160b1 | *Arabidopsis thaliana* | ugccuggcucccuguaugcca | SEQ ID NO :291 |
| AtmiPEP160b2 | | | |
| AtmiPEP161 | *Arabidopsis thaliana* | ucaaugcauugaaagugacua | SEQ ID NO :292 |
| AtmiPEP162a1 | *Arabidopsis thaliana* | ucgauaaaccucugcauccag | SEQ ID NO :293 |
| AtmiPEP162b1 | *Arabidopsis thaliana* | ucgauaaaccucugcauccag | SEQ ID NO :294 |
| AtmiPEP163-1 | *Arabidopsis thaliana* | uugaagaggacuuggaacuucgau | SEQ ID NO :295 |
| AtmiPEP163-2 | | | |
| AlmiPEP164a1 | *Arabidopsis lyrata* | uggagaagcagggcacgugca | SEQ ID NO :296 |
| AlmiPEP164a2 | | | |
| AlmiPEP164a3 | | | |
| AtmiPEP164a1 | *Arabidopsis thaliana* | uggagaagcagggcacgugca | SEQ ID NO :297 |
| AtmiPEP164a2 | | | |
| AtmiPEP164a3 | | | |
| BrmiPEP164a1 | *Brassica rapa* | uggagaagcagggcacgugca | SEQ ID NO :298 |
| BrmiPEP164a2 | | | |
| BrmiPEP164a3 | | | |
| CpmiPEP164a1 | *Carica papaya* | uggagaagcagggcacgugca | SEQ ID NO :299 |
| CpmiPEP164a2 | | | |
| CrmiPEP164a1 | *Capsella rubella* | uggagaagcagggcacgugca | SEQ ID NO :300 |
| CrmiPEP164a2 | | | |
| CrmiPEP164a3 | | | |
| GrmiPEP164a1 | *Gossypium raimondii* | uggagaagcagggcacgugca | SEQ ID NO :301 |
| GrmiPEP164a2 | | | |
| GrmiPEP164a3 | | | |
| MtmiPEP164a1 | *Medicago truncatula* | uggagaagcagggcacgugca | SEQ ID NO :302 |
| MtmiPEP164a2 | | | |
| OsmiPEP164a1 | *Oryza sativa* | uggagaagcaggguacgugca | SEQ ID NO :303 |
| OsmiPEP164a2 | | | |
| AlmiPEP165a | *Arabidopsis lyrata* | ucggaccaggcuucauccccc | SEQ ID NO :304 |
| AtmiPEP165a | *Arabidopsis thaliana* | ucggaccaggcuucauccccc | SEQ ID NO :305 |
| BcmiPEP165a | *Brassica carinata* | ucggaccaggcuucauccccc | SEQ ID NO :306 |
| BjmiPEP165a | *Brassica juncea* | ucggaccaggcuucauccccc | SEQ ID NO :307 |
| BnmiPEP165a | *Brassica napus* | ucggaccaggcuucauccccc | SEQ ID NO :308 |
| BomiPEP165a | *Brassica oleracea* | ucggaccaggcuucauccccc | SEQ ID NO :309 |
| BrmiPEP165a | *Brassica rapa* | ucggaccaggcuucauccccc | SEQ ID NO :310 |
| AtmiPEP166a | *Arabidopsis thaliana* | ucggaccaggcuucauucccc | SEQ ID NO :311 |
| AtmiPEP166b | *Arabidopsis thaliana* | ucggaccaggcuucauucccc | SEQ ID NO :312 |
| AtmiPEP167a | *Arabidopsis thaliana* | ugaagcugccagcaugaucua | SEQ ID NO :313 |
| AtmiPEP167b1 | *Arabidopsis thaliana* | ugaagcugccagcaugaucua | SEQ ID NO :314 |
| AtmiPEP167b2 | | | |
| AtmiPEP169c1 | *Arabidopsis thaliana* | cagccaaggaugacuugccgg | SEQ ID NO :315 |
| AtmiPEP169c2 | | | |
| AtmiPEP16911 | *Arabidopsis thaliana* | uagccaaggaugacuugccug | SEQ ID NO :316 |
| AtmiPEP171a1 | *Arabidopsis thaliana* | ugauugagccgcgccaauauc | SEQ ID NO :317 |
| AtmiPEP171b | *Arabidopsis thaliana* | uugagccgugccaauaucacg | SEQ ID NO :318 |
| MtmiPEP171b1 | *Medicago truncatula* | ugauugagccgcgucaauauc | SEQ ID NO :319 |
| MtmiPEP171b2 | | | |
| ZmmiPEP171b | *Zea mays* | ggauugagccgcgucaauauc | SEQ ID NO :320 |
| AtmiPEP171c1 | *Arabidopsis thaliana* | uugagccgugccaauaucacg | SEQ ID NO :321 |
| MtmiPEP171e | *Medicago truncatula* | agauugagccgcgccaauauc | SEQ ID NO :322 |
| MtmiPEP171h | *Medicago truncatula* | cgagccgaaucaauaucacuc | SEQ ID NO :323 |
| AtmiPEP172a1 | *Arabidopsis thaliana* | agaaucuugaugaugcugcau | SEQ ID NO :324 |
| AtmiPEP172a3 | | | |
| AtmiPEP172b 1 | *Arabidopsis thaliana* | gcagcaccauuaagauucac | SEQ ID NO :325 |
| AtmiPEP172c1 | *Arabidopsis thaliana* | agaaucuugaugaugcugcag | SEQ ID NO :326 |
| AtmiPEP172e1 | *Arabidopsis thaliana* | ggaaucuugaugaugcugcau | SEQ ID NO :327 |
| AtmiPEP172e2 | | | |
| AtmiPEP172e3 | | | |
| AcmiPEP319a1 | *Arabidopsis cebennensis* | uuggacugaagggagcucccu | SEQ ID NO :328 |
| AcmiPEP319a2 | | | |
| AhmiPEP319a | *Arabidopsis halleri* | uuggacugaagggagcucccu | SEQ ID NO :329 |
| AlmiPEP319a | *Arabidospis lyrata* | uuggacugaagggagcucccu | SEQ ID NO :330 |
| AtmiPEP319a1 | *Arabidopsis thaliana* | uuggacugaagggagcucccu | SEQ ID NO :331 |
| AtmiPEP319a2 | | | |
| BrmiPEP319a | *Brassica rapa* | uuggacugaagggagcucccu | SEQ ID NO :332 |
| CpmiPEP319a | *Carica papaya* | uuggacugaagggagcuccuu | SEQ ID NO :333 |
| CrmiPEP319a | *Capsella rubella* | uuggacugaagggagcucc | SEQ ID NO :334 |
| EgmiPEP319a | *Eucalyptus grandis* | uuggacugaagggagcucccu | SEQ ID NO :335 |
| GrmiPEP319a | *Gossypium raimondii* | uuggacugaagggagcucccu | SEQ ID NO :336 |
| MtmiPEP319a | *Medicago truncatula* | uuggacugaagggagucucccu | SEQ ID NO :337 |
| OsmiPEP319a | *Oryza sativa* | uuggacugaagggugcucccu | SEQ ID NO :338 |
| PpmiPEP319a | *Physcomitrella patens* | cuuggacugaagggagcucc | SEQ ID NO :339 |
| ThmiPEP319a1 | *Thellungiella halophila* | uggacucaaggaagcucucu | SEQ ID NO :340 |
| ThmiPEP319a2 | | | |
| AtmiPEP319b1 | *Arabidopsis thaliana* | uuggacugaagggagcucccu | SEQ ID NO :341 |
| AtmiPEP394a1 | *Arabidopsis thaliana* | uuggcauucuguccaccucc | SEQ ID NO :342 |
| AtmiPEP395c1 | *Arabidopsis thaliana* | cugaaguguuuggggggacuc | SEQ ID NO :343 |
| AtmiPEP395e1 | *Arabidopsis thaliana* | cugaaguguuugggggaacuc | SEQ ID NO :344 |
| AtmiPEP397b1 | *Arabidopsis thaliana* | ucauugagugcaucguugaug | SEQ ID NO :345 |
| AtmiPEP398c1 | *Arabidopsis thaliana* | uguguucucaggucaccccug | SEQ ID NO :346 |
| AtmiPEP399b | *Arabidopsis thaliana* | ugccaaaggagaguugcccug | SEQ ID NO :347 |
| AtmiPEP399d1 | *Arabidopsis thaliana* | ugccaaaggagauuugccccg | SEQ ID NO :348 |
| AtmiPEP403 | *Arabidopsis thaliana* | uuagauucacgcacaaacucg | SEQ ID NO :349 |
| AtmiPEP447a1 | *Arabidopsis thaliana* | uuggggacgagauguuuuguug | SEQ ID NO :350 |
| AtmiPEP447a2 | *Arabidopsis thaliana* | uuggggacgagauguuuuguug | |
| AtmiPEP447b1 | *Arabidopsis thaliana* | uuggggacgagauguuuuguug | SEQ ID NO :351 |
| AtmiPEP447b2 | *Arabidopsis thaliana* | uuggggacgagauguuuuguug | |
| AtmiPEP447c | *Arabidopsis thaliana* | ccccuuacaaugucgaguaaa | SEQ ID NO :352 |
| DmmiPEP1a | *Drosophila melanogaster* | uggaauguaaagaaguauggag | SEQ ID NO :353 |
| DmmiPEP1b | | | |
| DmmiPEP8 | *Drosophila melanogaster* | uaauacugucagguaaagauguc | SEQ ID NO :354 |
| HsmiPEP155 | *Homo sapiens* | uuaaugcuaaucgugauaggggu | SEQ ID NO :358 |
| AtmiPEP157c | *Arabidopsis thaliana* | uugacagaagauagagagcac | SEQ ID NO : 412 |
| AtmiPEP157d | *Arabidopsis thaliana* | ugacagaagauagagagcac | SEQ ID NO : 413 |
| AtmiPEP160c | *Arabidopsis thaliana* | ugccuggcucccuguaugcca | SEQ ID NO : 414 |
| AtmiPEP164b | *Arabidopsis thaliana* | uggagaagcagggcacgugca | SEQ ID NO : 415 |
| AtmiPEP166c | *Arabidopsis thaliana* | ucggaccaggcuucauucccc | SEQ ID NO : 416 |
| AtmiPEP166d | *Arabidopsis thaliana* | ucggaccaggcuucauucccc | SEQ ID NO : 417 |
| AtmiPEP169a | *Arabidopsis thaliana* | cagccaaggaugacuugccga | SEQ ID NO : 418 |
| AtmiPEP169h | *Arabidopsis thaliana* | uagccaaggaugacuugccug | SEQ ID NO : 419 |
| AtmiPEP169n | *Arabidopsis thaliana* | uagccaaggaugacuugccug | SEQ ID NO : 420 |
| AtmiPEP170 | *Arabidopsis thaliana* | ugauugagccgugucaauauc | SEQ ID NO : 421 |
| AtmiPEP396a | *Arabidopsis thaliana* | uuccacagcuuucuugaacug | SEQ ID NO : 422 |
| AtmiPEP399c | *Arabidopsis thaliana* | ugccaaaggagaguugcccug | SEQ ID NO : 423 |
| AtmiPEP169d | *Medicago truncatula* | aagccaaggaugacuugccgg | SEQ ID NO : 427 |

**Table 5. Liste des Pre-miRs témoins**

| **Pre-miR** | **Organisme** | **Séquence du Pre-miR** | **SEQ ID** |
|---|---|---|---|
| Pre-miR169 | *Medicago truncatula* | TTAGGGTTTTCAGCTCATGGTAATAAAAATGTCATCTAATGTCTTGCATGT GGGAATGAGGTCATATATGCAGCCAAGGATGACTTGCCGGCGAGCCTCTT TCGATACTTTTATGACATAATTAATCATGTGGATAGCCAAGGTACTAAACT CACTTTGCACTAAAACAAATATTTTTGCTTTAGTGCAAACTTAGTTTAGGC GCTTCGCAACGGCTAGTCAAATGTCCTAGTTCCAATGTGATTGGTTGTCCG GCAAGTCGTCTCTGGCTACGTAAAGGCCTCCTTTTTTCATGCTAGATTTTTG ATGATTTGATATAGCCACACATATTTTGGAA | SEQ ID NO :359 |
| Pre-miR169a | *Medicago truncatula* | AAGAGGCAGAGAGAGTAATGCAGCCAAGGATGACTTGCCGACAACATTG GCGAATGTTCATGTGATTTCTGCCTCATTGTGCCGGCAAGTTGTCCTTGGCT ATGTTAGTCTCTCATCTTCT | SEQ ID NO :360 |
| Pre-miR171aMI 0001753 | *Medicago truncatula* | TGAATTCCCCTCCGCTTTTTGATGTTGGCTTGTCTCAATCAAATCAAAGTTC TTGAAATTTGAGTTCTTTAGTCTGATTGAGTCGTGCCAATATCATATTAAG CGATAAAAGTC | SEQ ID NO :361 |
| Pre-miR171h | *Medicago truncatula* | CCACAAAACTATAACTAGCTAGAAGCTTTAATCGCCTTATTTATTATAATA ATAATAATAAATATGGCTTCAGCTGCAAAAGTATACATGGCGTGATATTG ATCCGGCTCATCTATATCTTCAAGTTCAATCATCCATATTCATATCAATTTC AGACGAGCCGAATCAATATCACTCTTGTTTGCTTCATTGCATATTAATTAT ATACTTCATTTATAAGTTATAGTTTGCCATATATATATTAGATTGATTCTGC AGAAGTAGACAGGAGTGGTGTTGTTTCTGCTCATCTTATTAAATAATGAAT GAATGAATGACATTTGCTTACTTATAAGACGAGCCGAATCAATATCACTCC AGTACACCT | SEQ ID NO :362 |
| Pre-miR393a | *Medicago truncatula* | AACTGCAACTTGAGGAGGCATCCAAAGGGATCGCATTGATCCTATAATAT TTCAACTTTAGTCACTTTAATTTTCTCTCATATAATACTTAATTGGGATCAT GCCATCCCTTTGGATTTCTCCTTTAGTAGCTAC | SEQ ID NO :363 |
| Pre-miR393b | *Medicago truncatula* | AGGCATCCAAAGGGATCGCATTGATCCCAAATCTAATTAAGTCCCTAGCTA CTTAATTAACAACTTAATTTCCTTAATATCTCATAATATTTGGGATCATGCT ATCCCTTTGGATTCAT | SEQ ID NO :364 |
| Pre-miR396a | *Medicago truncatula* | TGCTTTTCCACAGCTTTCTTGAACTTCTTTCGTATCTTAAATCTGTTTTCAA GATTAAAAGTCCTAGAAGCTCAAGAAAGCTGTGGGAGAATA | SEQ ID NO :365 |
| Pre-miR396b | *Medicago truncatula* | TATTCTTCCACAGCTTTCTTGAACTGCATCCAAATTGAGTTCCTTTGCATTG CCATGGCCATTGTTTTGCGGTTCAATAAAGCTGTGGGAAGATA | SEQ ID NO :366 |

**Table 6. Liste des miRs témoins**

| **miR** | **Organisme** | **Séquence du miR** | **SEQ ID** |
|---|---|---|---|
| miR169 | *Medicago truncatula* | CAGCCAAGGAUGACUUGCCGG | SEQ ID NO :367 |
| miR169a | *Medicago truncatula* | CAGCCAAGGAUGACUUGCCGA | SEQ ID NO :368 |
| miR171a | *Medicago truncatula* | UGAUUGAGUCGUGCCAAUAUC | SEQ ID NO :369 |
| miR171h | *Medicago truncatula* | GAGCCGAAUCAAUAUCACUC | SEQ ID NO :370 |
| miR393a | *Medicago truncatula* | UCCAAAGGGAUCGCAUUGAUC | SEQ ID NO :371 |
| miR393b | *Medicago truncatula* | UCCAAAGGGAUCGCAUUGAUC | SEQ ID NO :372 |
| miR396a | *Medicago truncatula* | UUCCACAGCUUUCUUGAACUU | SEQ ID NO :373 |
| miR396b | *Medicago truncatula* | UUCCACAGCUUUCUUGAACUG | SEQ ID NO :374 |

**Table 7. Polymorphisme de la séquence d'ADN des différentes régions du pri-miR171b**

| | Taille | # SNPs | # mutations | %SNP | # haplotypes |
|---|---|---|---|---|---|
| pri-mir171b | 1127 | 91 | 100 | 8,07 | 161 |
| 5' pri-miR171b | 129 | 4 | 4 | 3,1 | 5 |
| miPEP171b | 62 | 2 | 2 | 3,22 | 3 |
| Pre-miR171b | 118 | 1 | 1 | 0,85 | 2 |
| miR171b+miR171b* | 42 | 0 | 0 | 0 | 1 |
| 3' pri-miR171b | 259 | 39 | 42 | 15,06 | 89 |

Les figures et les exemples suivants illustreront mieux l'invention, sans pour autant en limiter sa portée.

### LEGENDES DES FIGURES

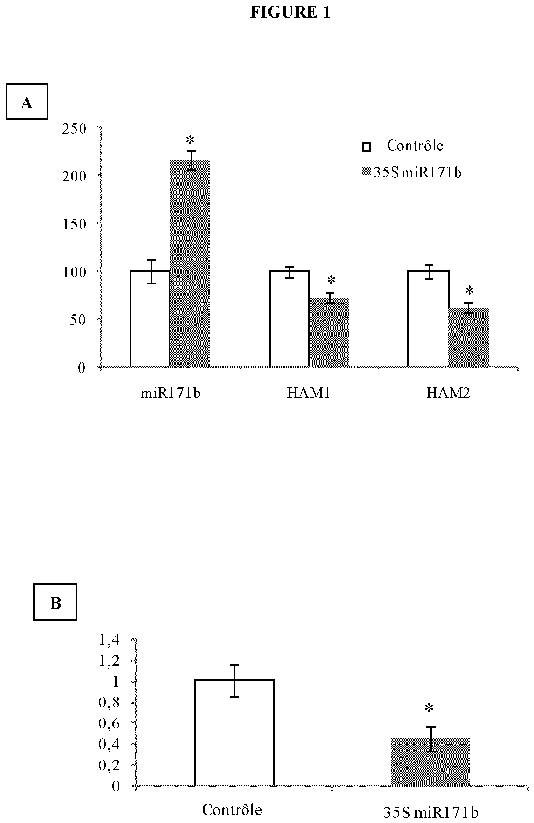

**FIGURE 1****. Effets de la surexpression du MtmiR171b (miR171b identifié chez** ***Medicago truncatula**)* **sur l'expression des gènes *HAM1* et *HAM2* (A) ou sur le nombre de racines latérales (B) chez *M. truncatula.***
   (A) L'axe des ordonnées indique l'expression relative du MtmiR171b (colonnes de gauche), de *HAM1* (colonnes du milieu) ou de *HAM2* (colonnes de droite) chez une plante témoin (colonnes blanches) ou chez une plante dans laquelle est surexprimé MtmiR171b (colonnes noires). La barre d'erreur correspond à l'erreur standard à la moyenne (nombre d'individus = 10). La surexpression de MtmiR171b induit une diminution de l'expression des gènes *HAM1 et HAM2.*
   (B) L'axe des ordonnées indique le nombre moyen de racines latérales observées chez une plante témoin (colonne blanche) ou chez une plante dans laquelle est surexprimé MtmiR171b (colonne noire). La barre d'erreur correspond à l'erreur standard à la moyenne (nombre d'individus = 100). La surexpression de MtmiR171b entraîne une réduction du nombre de racines latérales.

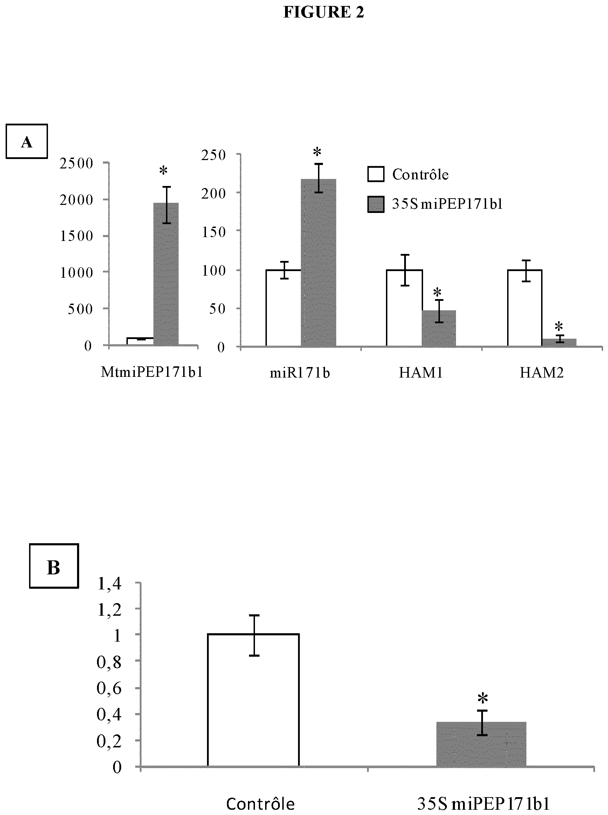

**FIGURE 2****. Effets de la surexpression du MtmiPEP171bl sur l'expression du MtmiR171b et des gènes *HAM1* et *HAM2* (A) ou sur le nombre racines latérales (B) chez *M. truncatula.***
   (A) L'axe des ordonnées indique l'expression relative du MtmiPEP171b1 (graphique de gauche), du miR171b (graphique de droite, colonnes de gauche), de *HAM1* (accession n°MtGI9- TC114268) (graphique de droite, colonnes du milieu) ou de *HAM2* (accession n° MtGI9- TC120850) (graphique de droite, colonnes de droite) chez une plante témoin (colonnes blanches) ou chez une plante dans laquelle est surexprimé MtmiPEP171b1 (colonnes noires). La barre d'erreur correspond à l'erreur standard à la moyenne (nombre d'individus = 10). La surexpression de MtmiPEP171b1 induit une augmentation de l'accumulation de MtmiR171b, ainsi qu'une diminution de l'expression des gènes *HAM1* et *HAM2.*
   (B) L'axe des ordonnées indique le nombre moyen de racines latérales observées chez une plante témoin (colonne blanche) ou chez une plante dans laquelle est surexprimé MtmiPEP171b1 (colonne noire). La barre d'erreur correspond à l'erreur standard à la moyenne (nombre d'individus = 100). La surexpression de MtmiPEP171b1 entraîne une réduction du nombre de racines latérales.

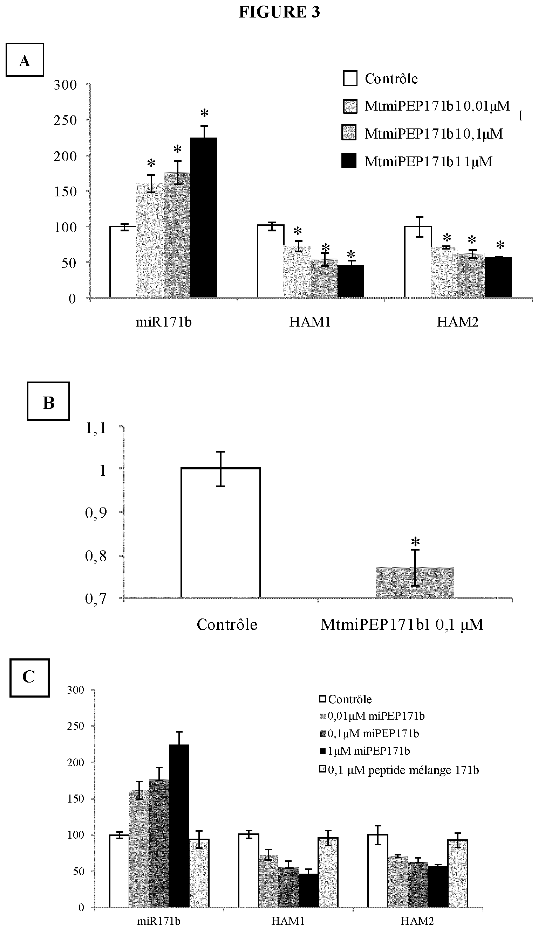

**FIGURE 3****. Effets du MtmiPEP171bl sur l'expression du MtmiR171b et des gènes *HAM1* et *HAM2* (A) et sur le nombre racines latérales (B) chez *M. truncatula.***
   (A) L'axe des ordonnées indique l'expression relative du MtmiR171b (colonnes de gauche), de *HAM1* (colonnes du milieu) ou de *HAM2* (colonnes de droite) chez une plante témoin (colonnes blanches) ou chez une plante traitée par arrosage pendant 5 jours et 1 fois par jour avec le MtmiPEP171b1 à 0,01 µM (colonnes gris clair), 0,1 µM (colonnes gris foncé) ou 1µM (colonnes noires). La barre d'erreur correspond à l'erreur standard à la moyenne (nombre d'individus = 10). L'application du MtmiPEP171b1 à différentes concentrations induit une augmentation de l'accumulation de MtmiR171b, ainsi qu'une diminution de l'expression des gènes *HAM1* et *HAM2.*
   (B) L'axe des ordonnées indique le nombre moyen de racines latérales observées chez une plante témoin (colonne blanche) ou chez une plante traitée par arrosage avec le MtmiPEP171b1 à 0,1 µM pendant 5 jours et 1 fois par jour (colonne noire). La barre d'erreur correspond à l'erreur standard à la moyenne (nombre d'individus = 100). L'application du MtmiPEP171b1 à 0,1 µM entraîne une réduction du nombre de racines latérales.
   (C) L'axe des ordonnées indique l'expression relative du MtmiR171b (colonnes de gauche), de *HAM1* (colonnes du milieu) ou de *HAM2* (colonnes de droite) chez une plante témoin (colonnes blanches) ou chez une plante traitée par arrosage pendant 5 jours et 1 fois par jour avec le MtmiPEP171b1 à 0,01 µM (colonnes gris), 0,1 µM (colonnes gris foncé) ou 1µM (colonnes noires) ou avec 0,01 µM d'un peptide mélange (LIVSHLYSEKFDCMRKILRI, SEQ ID NO : 428)(colonnes gris clair) dont la composition en acides aminés est identique au miPEP171b mais dont la séquence est différente. La barre d'erreur correspond à l'erreur standard à la moyenne (nombre d'individus = 10).

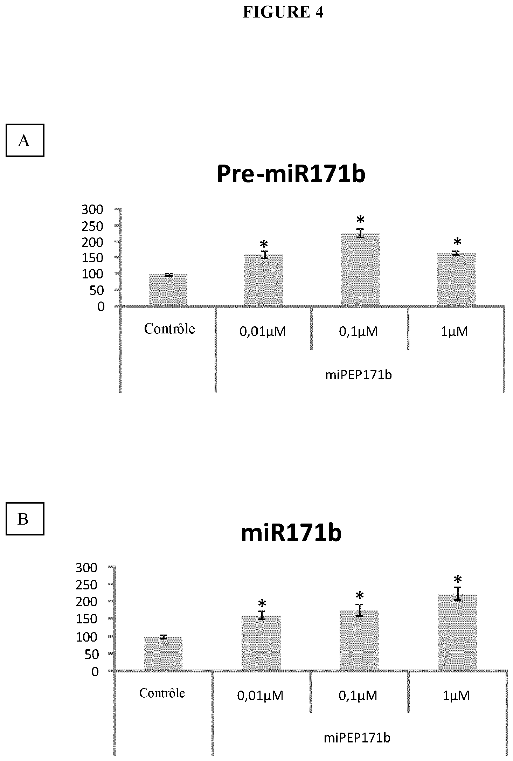

**FIGURE 4****. Effets du MtmiPEP171bl sur l'expression du pre-MtmiR171b (A) et du MtmiR171b (B) chez *M. truncatula.***
   L'axe des ordonnées indique l'expression relative des précurseurs des différentes formes du microARN chez des plantes contrôles (colonne de gauche) ou chez des plantes traitées par arrosage pendant 5 jours et 1 fois par jour avec le MtmiPEP 171b1 à 0,01 µM, 0,1 µM ou 1 µM (colonnes de droite). La barre d'erreur correspond à l'erreur standard à la moyenne (nombre d'individus = 200). L'application du MtmiPEP171b1 à différentes concentrations entraîne une augmentation de l'accumulation du pre-MtmiR171b (A) et du MtmiR171b (B).

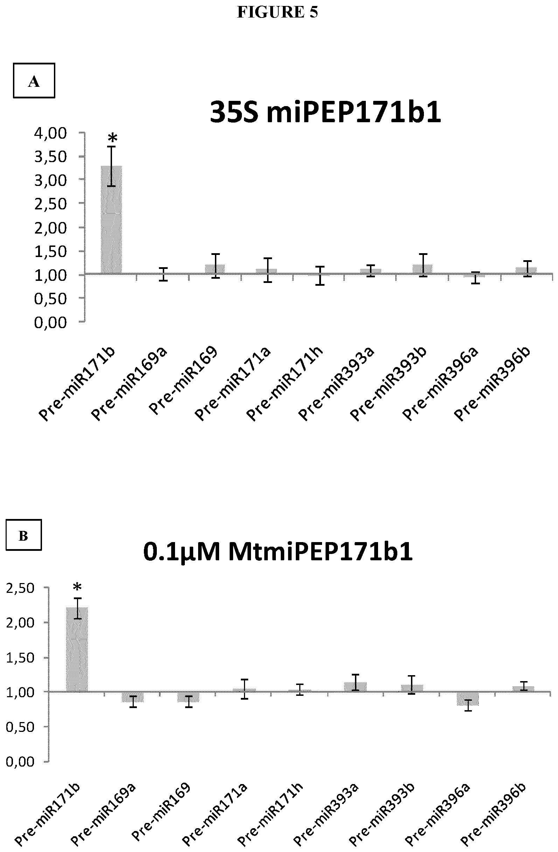

**FIGURE 5****. Effets de la surexpression du MtmiPEP171bl (A) et effets du MtmiPEP171bl (B) sur l'expression de différents précurseurs de microARNs chez *M*. *truncatula.***
   L'axe des ordonnées indique le ratio de l'expression des précurseurs de microARNs dans des plantes surexprimant le MtmiPEP171b1 sur l'expression de ces mêmes précurseurs dans des racines contrôle (A), ou le ratio de l'expression des précurseurs de microARNs dans des plantes traitées avec le MtmiPEP171b1 (0,1 µM) sur l'expression de ces mêmes précurseurs dans des racines contrôle (B). Les différents précurseurs de microARNs testés sont indiqués de gauche à droite sur l'axe des abscisses, à savoir pre-MtmiR171b (SEQ ID NO : 246), pre-MtmiR169 (SEQ ID NO : 359), pre-MtmiR169a (SEQ ID NO : 360), pre-MtmiR171a (SEQ ID NO : 361), pre-MtmiR171h (SEQ ID NO : 362), pre-MtmiR393a (SEQ ID NO : 363), pre-MtmiR393b (SEQ ID NO: 364), pre-MtmiR396a (SEQ ID NO: 365) et pre-MtmiR396b (SEQ ID NO : 366). La barre d'erreur correspond à l'erreur standard à la moyenne (nombre d'individus = 10). On constate que le MtmiPEP 171b1 n'entraîne un effet que sur l'accumulation du MtmiR171b et pas sur les autres miRs.

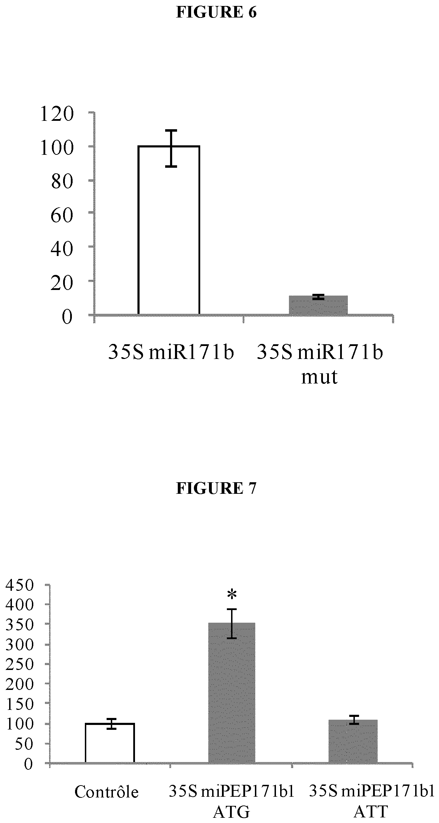

**FIGURE 6****. Effets de la traduction du MtmiPEP171bl sur l'expression du MtmiR171b mis en évidence chez la plante modèle** ***Nicotiana benthamiana.*** L'axe des ordonnées indique l'expression relative du MtmiR171b chez des plantes de tabac transformées pour exprimer le pri-MtmiR171b (colonne blanche) ou un pri-MtmiR171b muté dans lequel le codon ATG a été remplacé par ATT (colonne noire). Le pri-MtmiR171b muté est donc incapable de produire le MtmiPEP171b1. La barre d'erreur correspond à l'erreur standard à la moyenne (nombre d'individus = 30). On constate que l'absence de la traduction du MtmiPEP171b1 entraîne une forte diminution de l'accumulation du miR171b.
**FIGURE 7****. Effets de la surexpression du MtmiPEP171bl sur l'expression du pre-MtmiR171b mis en évidence chez la plante modèle *Nicotiana benthamiana.***
   L'axe des ordonnées indique l'expression relative du pre-MtmiR171b chez des plantes de tabac qui ont été transformées pour exprimer le MtmiR171b (colonne de gauche), le MtmiR171b et le MtmiPEP171b1 (colonne du centre), ou le MtmiR171b et une version mutée du MtmiORF171b dans laquelle le codon d'initiation ATG a été remplacé ATT (colonne de droite). La barre d'erreur correspond à l'erreur standard à la moyenne (nombre d'individus = 30). On constate que l'expression du MtmiPEP171b1 augmente l'expression du MtmiR171b, et cet effet est dépendant de la traduction du MtmiORF171b en MtmiPEP171b1.

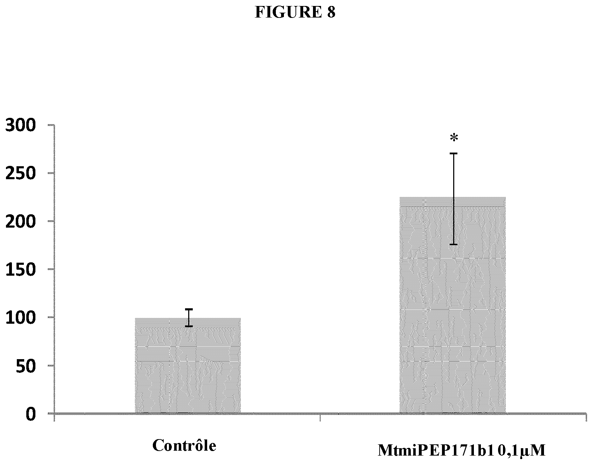

**FIGURE 8****. Effets du MtmiPEP171bl sur l'expression du pre-MtmiR171b mis en évidence chez la plante modèle *Nicotiana benthamiana.***
   L'axe des ordonnées indique l'expression relative du MtmiR171b chez des plantes de tabac transformées pour exprimer le MtmiR171b sur lesquelles le MtmiPEP171b1 a été pulvérisé (0,1 µM) 2 fois, 12h puis 30min avant prélèvement (colonne de droite) ou non (colonne de gauche). La barre d'erreur correspond à l'erreur standard à la moyenne (nombre d'individus = 6). Le peptide MtmiPEP171b1 utilisé par pulvérisation induit une augmentation de l'accumulation du MtmiR171b.

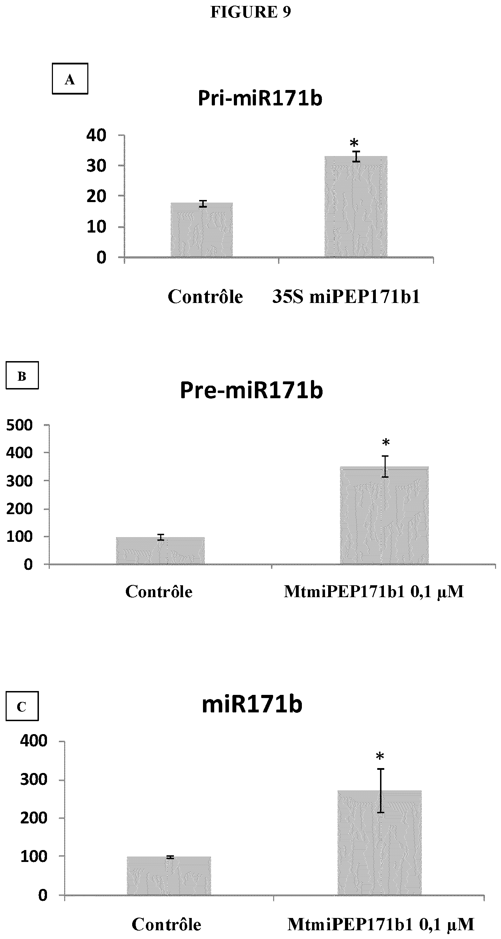

**FIGURE 9****. Effets du MtmiPEP171bl sur l'expression du pri-miR171b (A), du pre-MtmiR171b (B) et du MtmiR171b (C) mis en évidence chez la plante modèle *Nicotiana benthamiana.***
   L'axe des ordonnées indique l'expression relative des précurseurs des différentes formes du microARN chez des plantes de tabac modifiées pour exprimer le MtmiR171b (colonne de gauche) ou modifiées pour exprimer le MtmiR171b et surexprimer le MtmiPEP171b1 (colonnes de droite, fig. 9A) ou traitées par 0.1µM de miPEP171b1 (Fig. 9B et C). La barre d'erreur correspond à l'erreur standard à la moyenne (nombre d'individus = 30). La surexpression du MtmiPEP171b1 ou l'application du miPEP171b1 augmente l'accumulation du pri-MtmiR171b (A), du pre-MtmiR171b (B) et du MtmiR171b (C).
**FIGURE 10****. Localisation du MtmiPEP171bl dans les cellules de feuilles de tabac qui ont été modifiées pour exprimer le MtmiPEP171b1.**
   Les photographies représentent des cellules de feuilles de tabac modifiées pour exprimer la protéine GFP seule (cadre de gauche) ou la protéine GFP fusionnée au MtmiPEP171b1 (cadre de droite). Ces observations indiquent que le MtmiPEP171b1 est localisé dans des petits corps nucléaires.
**FIGURE 11****. Effets de l'expression du AtmiPEP165a (identifié chez *Arabidopsis thaliana*) sur l'expression du AtmiR165a (A) et de l'expression du AtmiPEP319a2 (identifié chez *Arabidopsis thaliana*) sur le AtmiR319a (B), mis en évidence chez la plante modèle du tabac.**
   (A) L'axe des ordonnées indique l'expression relative du AtmiR165a chez des plantes de tabac modifiées pour exprimer le AtmiR165a (colonne de gauche) ou pour exprimer le AtmiR165a et le AtmiPEP165a (colonne de droite).
   (B) L'axe des ordonnées indique l'expression relative du AtmiR319a chez des plantes de tabac modifiées pour exprimer le AtmiR319a (colonne de gauche) ou pour exprimer le AtmiR319a et le AtmiPEP319a (colonne de droite).
   La barre d'erreur correspond à l'erreur standard à la moyenne (nombre d'individus = 30). Dans les deux cas, on constate que l'expression du miORF, et donc la production du miPEP, entraîne une augmentation de l'accumulation du pre-miR.

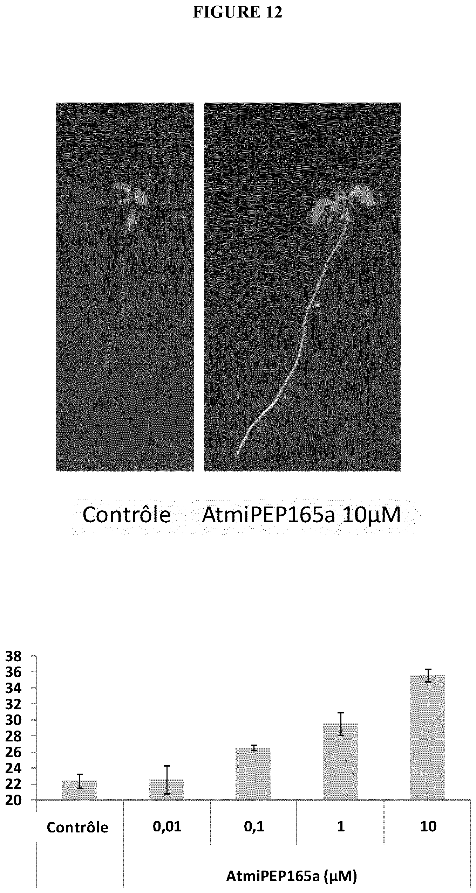

**FIGURE 12****. Effets du traitement par le AtmiPEP165a sur la croissance des racines *d'Arabidopsis thaliana.***
   La photographie présente deux plantes de même âge : une plante contrôle (plante de gauche) et une plante traitée par le AtmiPEP165a (plante de droite). Le traitement par le AtmiPEP165a entraine un phénotype de croissance racinaire très accélérée chez *Arabidopsis thaliana.* Le graphique montre l'expression du pre-miR165 en réponse au traitement par des doses croissantes de AtmiPEP165a.

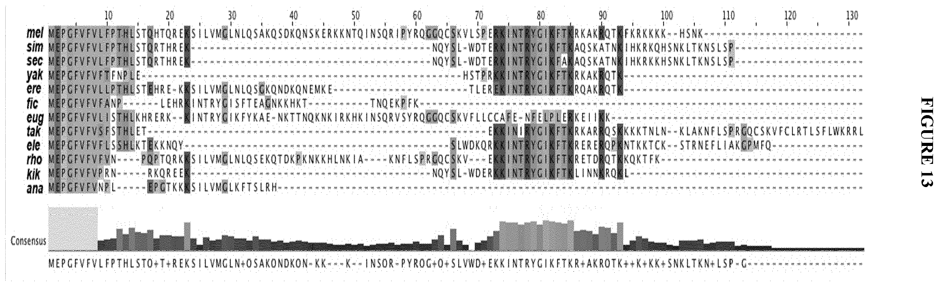

**FIGURE 13****. Conservation de la séquence du miPEP8 identifié chez la drosophile.**
   Les séquences du miPEP8 (SEQ ID NO : 104) ont été déduites à partir des séquences du miORF8 (SEQ ID NO : 208) de 12 espèces de drosophile différentes et ont été alignées. Un histogramme représente la conservation de chaque acide aminé entre les séquences du miORF8 chez les 12 espèces analysées.

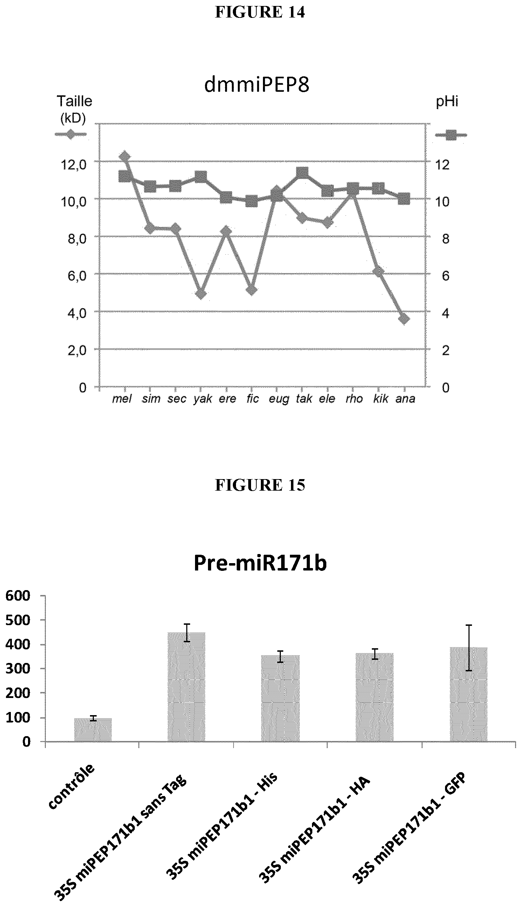

**FIGURE 14****. Evolution de la masse (kDa) et du point isoélectrique (pl) du miPEP8 chez les espèces de drosophile.**
   L'axe des ordonnées de gauche indique la taille du miPEP8 (en kD). L'axe des ordonnées de droit indique le point isoélectrique du miPEP. L'axe des abscisses indique l'origine du miPEP, c'est-à-dire l'espèce de drosophile. On constate que malgré une modification importante de leur taille (plus d'un facteur 3), la charge des miPEPs reste très basique (>9,8) chez les 12 espèces étudiées.
**FIGURE 15****. Effet de l'ajout de séquences sur la fonction du miPEP.**
   Les feuilles de tabac ont été transformées pour surexprimer le miPEP171b. Ces graphiques montrent que l'ajout de séquences (tag His, HA ou GFP) n'altère pas la fonction du miPEP. L'axe des ordonnées indique l'expression relative du pre-MtmiR171b chez des plantes de tabac qui ont été transformées pour exprimer le MtmiR171b (colonne de gauche), le MtmiR171b et le MtmiPEP171b1 avec ou sans ajout de tags proteiques (colonnes de droite) La barre d'erreur correspond à l'erreur standard à la moyenne (nombre d'individus = 6). On constate que l'expression du MtmiPEP171b1 augmente l'expression du MtmiR171b, et cet effet est indépendant de la présence de tags.

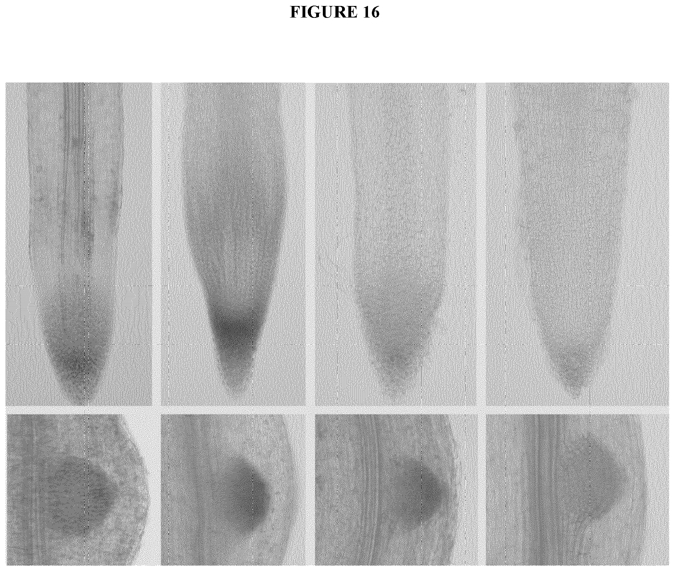

**FIGURE 16****. Expression du MtmiPEP171bl dans le système racinaire de *Medicago truncatula.***
   Les racines de *Medicago truncatula* ont été transformées pour exprimer des fusions entre la protéine GUS (en bleu) et le promoteur du miR17b (A, E), l'ATG du miPEP171b1 (B, F), le miPEP171b1 entier (C, G) ou bien l'ATG2 (deuxième ATG se trouvant sur le précurseur, après le miPEP) (D, H). On voit bien une expression du miR dans les pointes racinaires (A) ainsi que les racines latérales (E). Les fusions transcriptionnelles (B, F) et traductionnelles (C, G) montrent une expression du miPEP171b dans les mêmes tissus, alors que l'ATG suivant n'est pas actif (D, H).

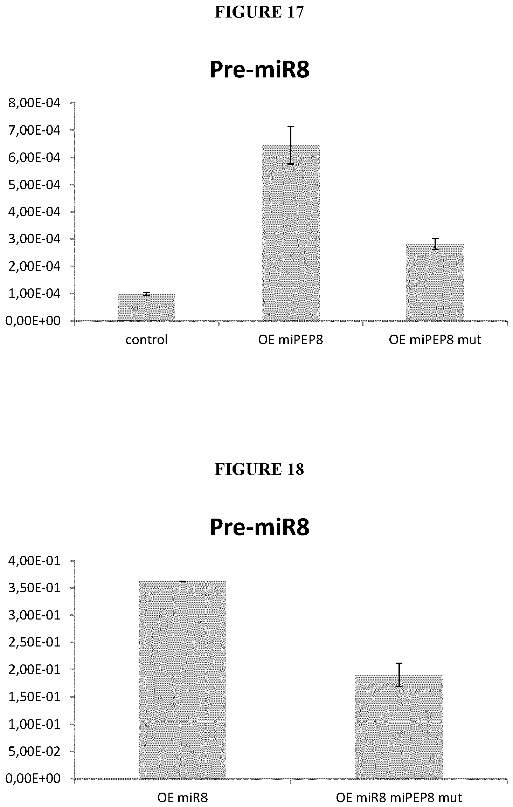

**FIGURE 17****. Expression du DmmiPEP8 dans des cellules de *Drosophila melanogaster*** Les cellules de *Drosphila melanogaster* ont été transfectées pour surexprimer le DmmiPEP8 (OE miPEP8) ou bien le miPEP8 dont les codons d'initiation de traduction ont été mutés (OE miPEP8 mut). L'axe des ordonnées indique l'expression relative du Pre-miR8. La barre d'erreur correspond à l'erreur standard à la moyenne (nombre d'expériences indépendantes = 6). On constate que l'expression du DmmiPEP8 augmente l'expression du DmmiR8, et cet effet est lié à la traduction de l'ARNm.
**FIGURE 18****. Impact du DmmiPEP8 sur l'accumulation du DmmiR8 dans des cellules de *D. melanogaster***
   Les cellules de *Drosphila melanogaster* ont été transfectées pour surexprimer le DmmiR8 sauvage (OE miR8) ou bien le DmmiR8 dans lequel les codons d'intitiation de la traduction ont été mutés (OE miR8 miPEP8 mut) L'axe des ordonnées indique l'expression relative du Pre-miR8. La barre d'erreur correspond à l'erreur standard à la moyenne (nombre d'expériences indépendantes = 2). On constate que la présence du DmmiPEP8 augmente l'expression du DmmiR8.

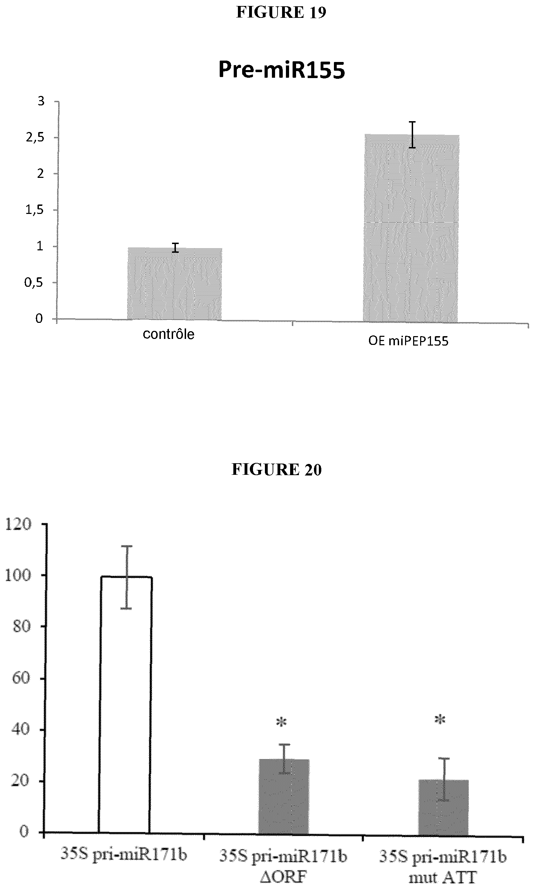

**FIGURE 19****. Impact du HsmiPEP155 sur l'accumulation du HsmiR155 dans des cellules d*'Homo sapiens***
   Les cellules HeLA d'Homo sapiens ont été transfectées pour surexprimer le HsmiPEP155 (OE miPEP155). L'axe des ordonnées indique l'expression relative du Pre-miR155. La barre d'erreur correspond à l'erreur standard à la moyenne (nombre d'expériences indépendantes = 2). On constate que l'expression du HsmiPEP155 augmente l'expression du HsmiR155.
**FIGURE 20****. Effets de la traduction du MtmiPEP171bl sur l'expression du MtmiR171b mis en évidence chez la plante modèle *Nicotiana benthamiana.***
   L'axe des ordonnées indique l'expression relative du MtmiR171b chez des plantes de tabac transformées pour exprimer le pri-miR171b (colonne de gauche), un pri-miR171b dans lequel le miORF171b a été délété (colonne du milieu) ou un pri-miR171b muté dans lequel le codon ATG a été remplacé par ATT (colonne de droite). Le pri-miR171b muté est donc incapable de produire le miPEP171b1. La barre d'erreur correspond à l'erreur standard à la moyenne (nombre d'individus = 30). On constate que l'absence de la traduction du miPEP171b1 entraîne une forte diminution de l'accumulation du miR171b.

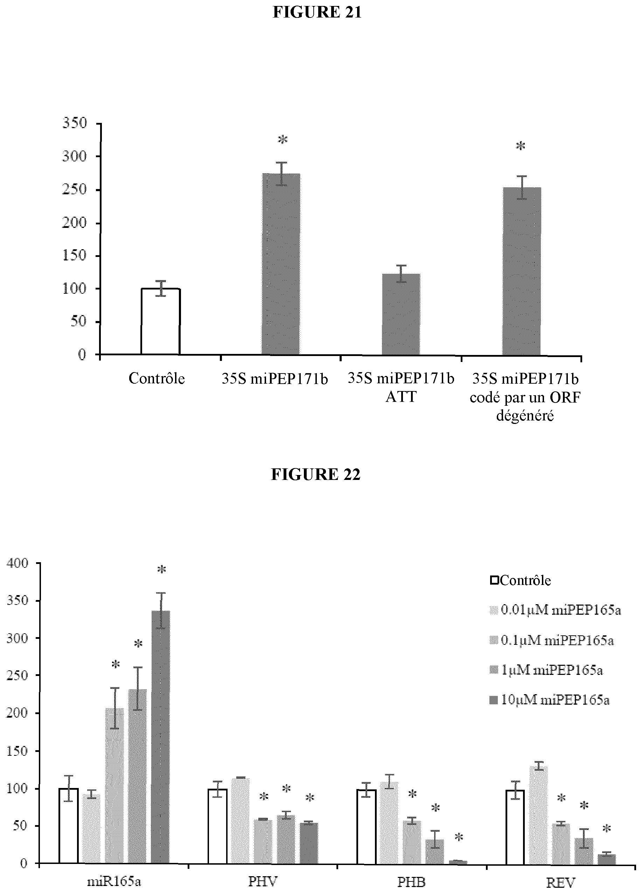

**FIGURE 21****. Effets de la surexpression du MtmiPEP171bl sur l'expression du MtmiR171b mis en évidence chez la plante modèle *Nicotiana benthamiana.***
   L'axe des ordonnées indique l'expression relative du MtmiR171b chez des plantes de tabac qui ont été transformées avec un vecteur permettant l'expression du miPEP171b et soit un second vecteur vide (colonne blanche), soit un vecteur permettant l'expression du mtmiPEP171b (colonne noire de gauche), soit un vecteur dans lequel le codon ATG de l'ORF codant le mtmiPEP171b a été remplacé par ATT (colonne noire du milieu), soit un vecteur dans lequel la séquence nucléotidique de l'ORF a été mutée sans modifier la séquence d'acides aminés du peptide traduit (miPEP codé par un ORF dégénéré) (colonne noire de droite). La barre d'erreur correspond à l'erreur standard à la moyenne (nombre d'individus = 30). On constate que l'expression du MtmiPEP171b1 augmente l'expression du MtmiR171b, et cet effet est dépendant de la traduction du MtmiORF171b en MtmiPEP171b1.
**FIGURE 22****. Effets du AtmiPEP165a sur l'accumulation du AtmiR165a et de ses gènes cibles (*PHAVOLUTA : PHV, PHABOL USA : PHB* et *REVOLUTA : REV*)*.***
   L'axe des ordonnées indique l'expression relative de AtmiR165a, PHV, PHB et REV dans des racines *d'Arabidopsis thaliana* traitées avec de l'eau (contrôle) ou différentes concentrations de AtmiPEP165a (0,01 µM, 0,1 µM, 1 µM ou 10 µM). La barre d'erreur correspond à l'erreur standard à la moyenne (nombre d'individus = 10).
   Le traitement des plantes avec des concentrations de plus en plus importantes de AtmiPEP165a met en évidence un effet dose-dépendant de l'accumulation du AtmiR165a et de la régulation négative de ses gènes cibles en fonction de la quantité de AtmiPEP165a.

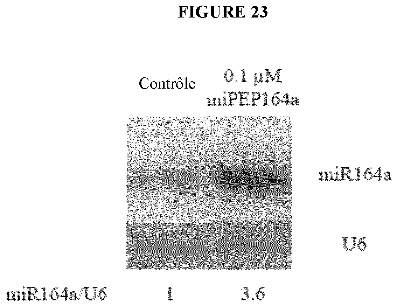

**FIGURE 23****. Effets d'un traitement avec le AtmiPEP164a sur l'expression du AtmiR164a chez *A. thaliana.***
   Les photographies représentent les résultats d'une analyse par Northern blot de l'accumulation du AtmiR164a dans des racines traitées avec de l'eau (contrôle, photographie de gauche) ou avec 0,1 µM d'un peptide synthétique, ayant une séquence identique à celle du AtmiPEP164a, dissout dans de l'eau (0,1 µM miPEP164a). L'ARN U6 est utilisé comme témoin de charge permettant de quantifier la quantité de AtmiR164a.
   Cette expérience a été répétée 4 fois de manière indépendante et a abouti à des résultats similaires.
   Le traitement des pousses *d'A. thaliana* avec 0,1 µM de miPEP164a entraîne une augmentation de l'accumulation du miR164a.

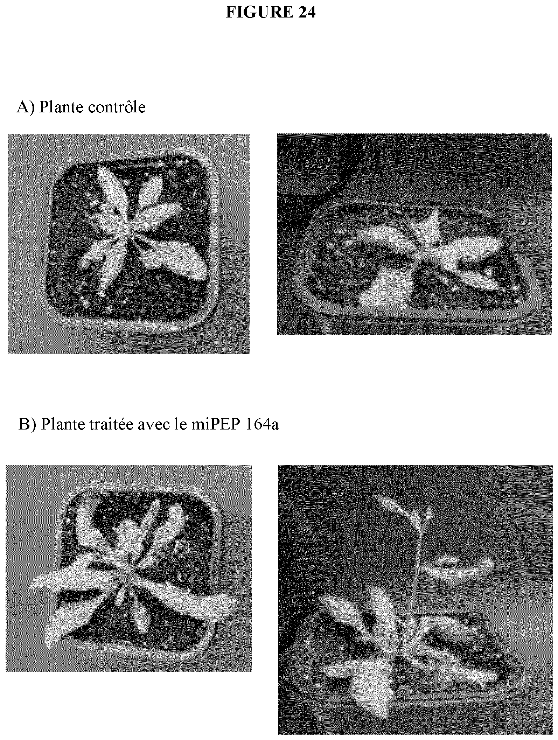

**FIGURE 24****. Effets du traitement par le AtmiPEP164a sur la croissance d'*Arabidopsis thaliana.***
   Les photographies présentent deux plantes (vues de dessus et vues de côté) après 3 semaines de croissance : une plante contrôle arrosée avec de l'eau (A), et une plante arrosée avec une composition de 0,1 µM de peptide synthétique correspondant à AtmiPEP164a (B). L'arrosage des plantes *d'Arabidopsis thaliana avec* AtmiPEP164a augmente significativement la croissance de la plante.

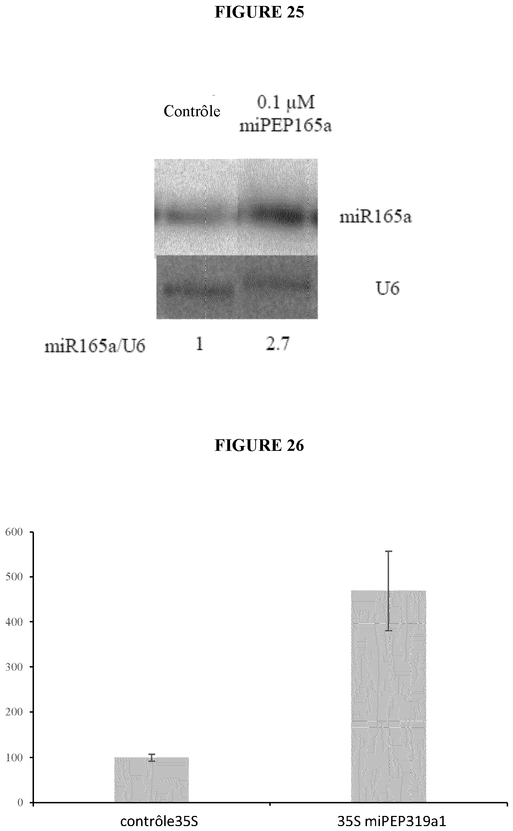

**FIGURE 25****. Effets d'un traitement avec le AtmiPEP165a sur l'expression du AtmiR165a chez *A. thaliana.***
   Les photographies représentent les résultats d'une analyse par Northern blot de l'accumulation du AtmiR165a dans des racines traitées avec de l'eau (contrôle, photographie de gauche) ou avec 0,1 µM d'un peptide synthétique, ayant une séquence identique à celle du AtmiPEP165a, dissout dans de l'eau (0,1 µM miPEP165a). L'ARN U6 est utilisé comme témoin de charge permettant de quantifier la quantité de AtmiR165a.
   Cette expérience a été répétée 4 fois de manière indépendante et a abouti à des résultats similaires.
   Le traitement des pousses d'A. *thaliana* avec 0,1 µM de miPEP165a entraîne une augmentation de l'accumulation du miR165a.
**FIGURE 26****. Effets de la surexpression du AtmiPEP319al sur l'expression du AtmiR319a chez *A. thaliana.***
   L'axe des ordonnées indique l'expression relative du AtmiR319a chez une plante témoin (colonne de gauche) ou chez une plante dans laquelle est surexprimé AtmiPEP319a1 (colonne de droite). La barre d'erreur correspond à l'erreur standard à la moyenne (nombre d'individus = 10). La surexpression de AtmiPEP319a1 induit une augmentation de l'accumulation de Atmir319a.

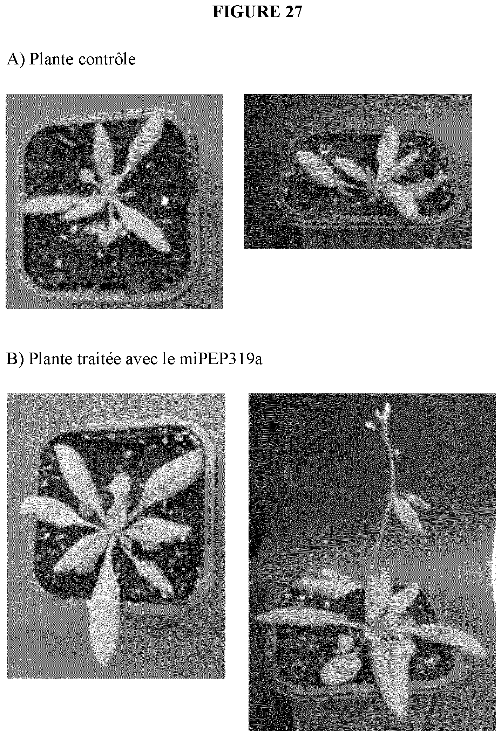

**FIGURE 27****. Effets du traitement par le AtmiPEP319a sur la croissance d'*Arabidopsis thaliana.***
   Les photographies présentent deux plantes (vues de dessus et vues de côté) après 3 semaines de croissance : une plante contrôle arrosée avec de l'eau (A), et une plante arrosée avec une composition de 0,1 µM de peptide synthétique correspondant à AtmiPEP319a1 (B). L'arrosage des plantes *d'Arabidopsis thaliana avec* AtmiPEP319a1 augmente significativement la croissance de la plante.

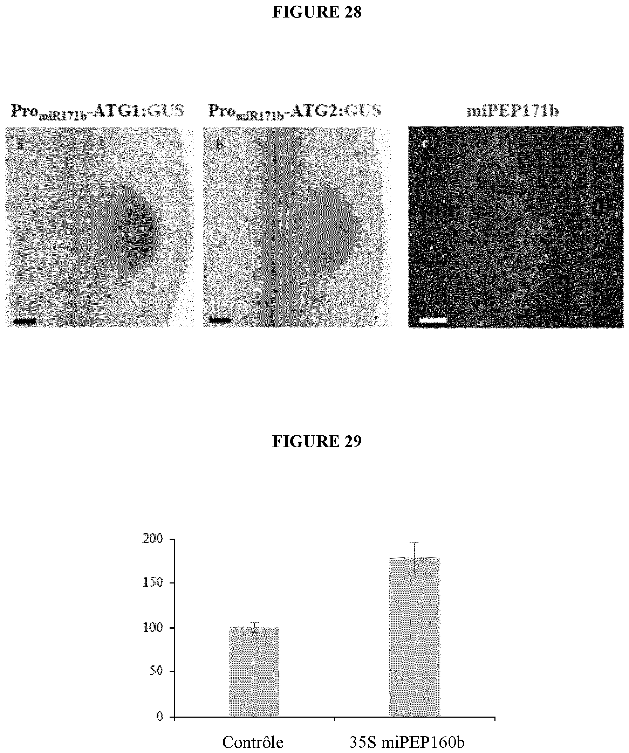

**FIGURE 28****. Immunolocalisation.**
   Les racines de *Medicago truncatula* ont été transformées pour exprimer des fusions entre la protéine GUS (en bleu) et l'ATG du miPEP171b (Pro_{miR171b}-ATG1 :GUS) ou bien l'ATG2 (deuxième ATG se trouvant sur le précurseur, après le miPEP) (Pro_{miR171b}-ATG2 :GUS). Un marquage a été également réalisé avec un anticorps anti-miPEP171b (miPEP171b). L'immunolocalisation du miPEP171b dans les racines de *M. truncatula* révèle la présence du miPEP171b dans les sites d'initiation des racines latérales, montrant une co-localisation entre le microARN et le miPEP correspondant.
**FIGURE 29****. Effets de la surexpression du AtmiPEP160bl sur l'expression du AtmiR160b chez *A. thaliana.***
   L'axe des ordonnées indique l'expression relative du AtmiR160b chez une plante témoin (barre de gauche) ou chez une plante dans laquelle est surexprimé AtmiPEP 160b 1 (barre de droite). La barre d'erreur correspond à l'erreur standard à la moyenne (nombre d'individus = 10).
   La surexpression de AtmiPEP 160b 1 entraîne une augmentation de l'expression relative du AtmiR160b.

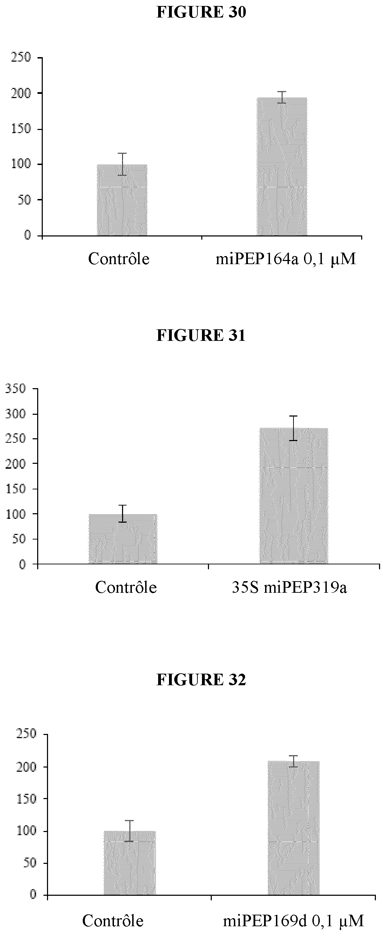

**FIGURE 30****. Effets du AtmiPEP164al sur l'expression du AtmiR164a .**
   L'axe des ordonnées indique l'expression relative du AtmiR164a chez une plante témoin (barre de gauche) ou chez une plante traitée par arrosage 1 fois par jour avec le AtmiPEP164a1 à 0,1 µM (barre de droite). La barre d'erreur correspond à l'erreur standard à la moyenne (nombre d'individus = 10).
   L'ajout de AtmiPEP164a1 entraîne une augmentation de l'expression relative du AtmiR164a.
**FIGURE 31****. Effets de la surexpression du AtmiPEP319al sur l'expression de AtmiR319a chez *A. thaliana.***
   L'axe des ordonnées indique l'expression relative de AtmiR319a chez une plante témoin (barre de gauche) ou chez une plante dans laquelle est surexprimé AtmiPEP319a1 (barre de droite). La barre d'erreur correspond à l'erreur standard à la moyenne (nombre d'individus = 10).
   La surexpression de AtmiPEP319a1 entraîne une augmentation de l'expression relative de AtmiR319a.
**FIGURE 32****. Effets du MtmiPEP169d sur l'expression du MtmiR169d chez *M***. ***truncatula.***
   L'axe des ordonnées indique l'expression relative du MtmiR169d chez une plante témoin (barre de gauche) ou chez une plante traitée par arrosage 1 fois par jour avec le MtmiPEP169d à 0,1 µM (barre de droite). La barre d'erreur correspond à l'erreur standard à la moyenne (nombre d'individus = 10).
   L'ajout de MtmiPEP169d entraîne une augmentation de l'expression relative du MtmiR169d.

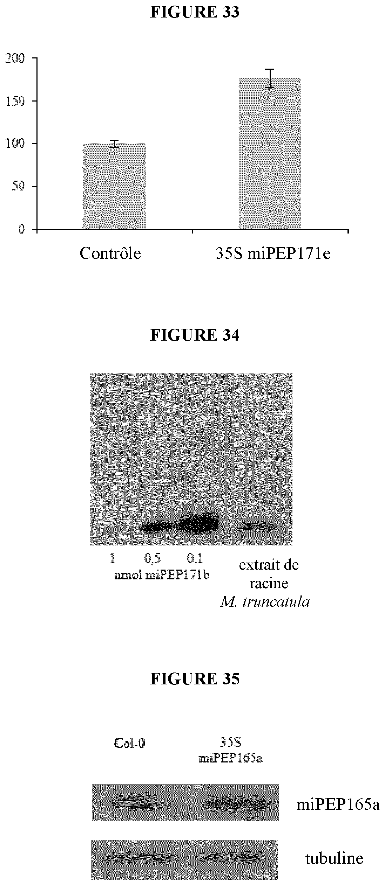

**FIGURE 33****. Effets de la surexpression du MtmiPEP171e sur l'expression de MtmiR171e chez** ***M**. **truncatula***
   L'axe des ordonnées indique l'expression relative de MtmiR171e chez une plante témoin (barre de gauche) ou chez une plante dans laquelle est surexprimé MtmiPEP171e (barre de droite). La barre d'erreur correspond à l'erreur standard à la moyenne (nombre d'individus = 10).
   La surexpression de MtmiPEP171e entraîne une augmentation de l'expression relative de MtmiR171e.
**FIGURE 34****. Localisation du MtmiPEP171bl dans une plante *M. truncatula* sauvage.** Différentes quantités de peptides synthétiques MtmiPEP171b1 (1, 0,5, ou 0,1 nmol) et un extrait total de racines de *M. truncatula* ont été analysés par immunoblot avec un anticorps spécifique du MtmiPEP171b1.
   Le MtmiPEP171b1 est naturellement produit dans les racines de *M*. *truncatula.*
**FIGURE 35****. Présence du AtmiPEP165a dans une plante *A. thaliana* sauvage.**
   La photographie du haut correpond à une analyse par western blot de la quantité de AtmiPEP165a chez un jeune plant d'A. *thaliana* sauvage Col-0 (gauche) et un jeune plant d'*A. thaliana* surexprimant AtmiPEP165a (droite).
   La photographie du bas correspond à l'analyse par western blot de la quantité d'une protéine contrôle, la tubuline, chez un jeune plant d'A. *thaliana* sauvage Col-0 (gauche) et un jeune plant d'*A. thaliana* surexprimant AtmiPEP165a (droite).
   Le AtmiPEP165a est naturellement produit chez *A. thaliana* et est présent en plus grande quantité dans les plantes surexprimant AtmiPEP165a.

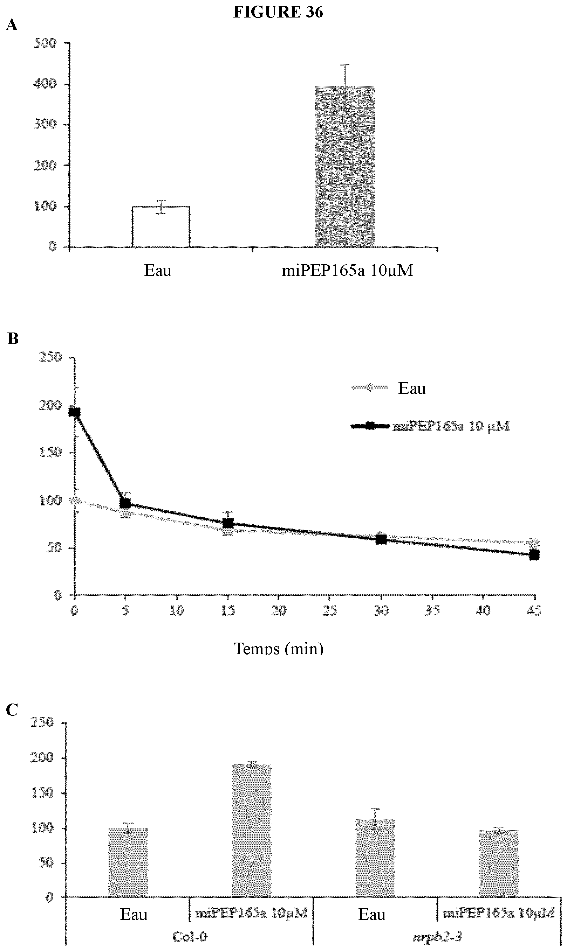

**FIGURE 36****. Mode d'action du AtmiPEP165a chez *A. thaliana.***
   (A) L'axe des ordonnées indique l'expression relative du pri-miR165a dans des racines témoins (barre de gauche) ou traitées par arrosage 1 fois par jour avec le AtmiPEP165a à 10 µM (barre de droite). La barre d'erreur correspond à l'erreur standard à la moyenne (nombre d'individus = 10).
      L'ajout de AtmiPEP165a entraîne une augmentation de l'expression relative du AtmiR165a.
   (B) L'axe des ordonnées indique l'expression relative du pri-miR165a, en présence de cordycepine (inhibiteur de synthèse de l'ARN), dans des racines témoins (courbe grise) ou traitées par arrosage 1 fois par jour avec le AtmiPEP165a à 10 µM (courbe noire). L'axe des abscisses indique la durée du traitement à la cordycepine.
      L'ajout de AtmiPEP165a n'entraîne pas une meilleure stabilité du pri-miR165a.
   (C) L'axe des ordonnées indique l'expression relative du pri-miR165a, chez des plantes sauvages (Col-0) ou mutées avec un allèle faible de la seconde grande sous-unité de l'ARN polymerase II (*nrpb2-3*)*.* L'axe des abscisses indique si les plantes ont été arrosées avec de l'eau ou avec le AtmiPEP165a à 10 µM.
      Les plantes mutées, contrairement aux plantes sauvages, ne sont pas capables d'accumuler le AtmiR165a en réponse au AtmiPEP165a.

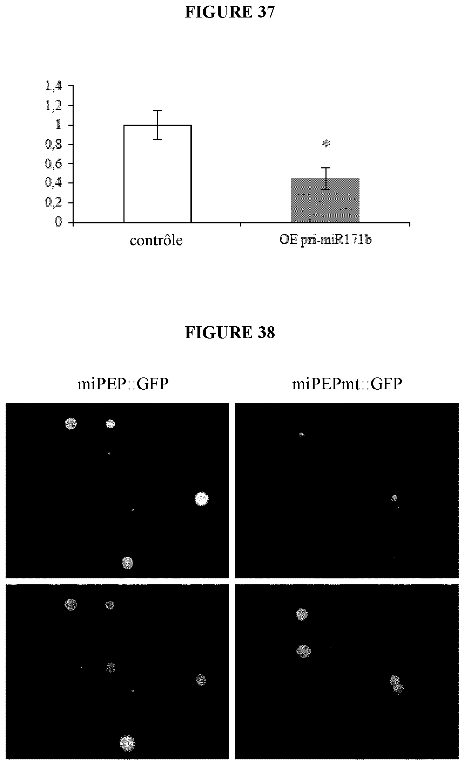

**FIGURE 37****. Effets du pri-miR171b sur le nombre de racines latérales chez M. *truncatula.***
   L'axe des ordonnées indique le nomre moyen de racines latérales observées chez une plante témoin (barre blanche) ou chez une plante dans laquelle est surexprimé le pri-miR171b (barre noire). La barre d'erreur correspond à l'erreur standard à la moyenne (nombre d'individus = 100).
   La surexpression du pri-miR171b entraîne une réduction du nombre de racines latérales.
**FIGURE 38****. Traductibilité du DmmiPEP8 dans les cellules de *Drosophila melanogaster.***
   Des cellules de *Drosphila melanogaster* ont été transfectées pour surexprimer le DmmiPEP8 (miPEP8 : :GFP) ou bien le DmmiPEP8 mt (dont les codons d'initiation de traduction ont été mutés (miPEP8 mt : :GFP)). Comme contrôle de transfection, un plasmide produisant la protéine moesin-RFP (images du bas) a été co-transfecté avec les plasmides codants les DmmiPEP8 (image de gauche) et DmmiPEP 8 mt (image de droite). On constate que les codons initiateurs présents dans la séquence DmmiPEP8 sont fonctionnels puisqu'ils permettent la synthèse de la GFP (image du haut a gauche) alors que les constructions mutées sur les ATG ne produisent quasiment plus de GFP (image du haut a droite).
   Ces résultats suggèrent donc que l'ORF du miPEP est fonctionnel.

### EXEMPLES

### A : Analyse des miPEPS chez les plantes

### EXEMPLE 1 - Caractérisation chez la plante modèle Medicago truncatula

### Exemple 1A - MtmiPEP171b1

### a) Identification et caractérisation du MtmiPEP171b1 (miPEP171b1 identifié chez Medicago truncatula)

Le microARN MtmiR171b est exprimé dans la région méristématique des racines. La surexpression de ce microARN conduit notamment à une réduction de l'expression des gènes *HAM1* (Accession n°. MtGI9- TC114268) et *HAM2* (Accession n°. MtGI9-TC120850) (Figure 1A), ainsi qu'à une réduction du nombre de racines latérales (Figures 1B). La surexpression du pri-miR171b entraîne également une réduction du nombre de racines latérales (Figure 37).

La séquence du transcrit primaire du MtmiR171b a été déterminée en utilisant la technique de RACE-PCR. L'analyse de la séquence du transcrit primaire a permis d'identifier la présence de plusieurs petits cadres ouverts de lecture (ORF) complètement inattendus. Ces ORFs ont été dénommés miORFs pour « *microRNA ORF* ». Ces miORFs codent potentiellement des peptides courts, d'environ 4 à 100 acides aminés. Aucune homologie significative concernant ces miORFS n'a été trouvée dans les bases de données.

La surexpression du premier miORF, nommé MtmiORF171b, conduit à une augmentation de l'accumulation de MtmiR171b et une réduction de l'expression des gènes *HAM1* et *HAM2* (voir Figure 2A), ainsi qu'à une réduction du nombre de racines latérales (Figure 2B), comme cela a déjà été observé lors de la surexpression du MtmiR171b.

Pour déterminer si le MtmiORF171b conduit à la production réelle d'un peptide et si la fonction de régulation observée ci-dessus est bien portée par ledit peptide, un peptide synthétique, dont la séquence est identique à celle potentiellement codée par MtmiORF171b, a été appliqué sur les racines de *Medicago truncatula.* L'application de ce peptide entraîne le phénotype déjà observé plus haut lors de la surexpression du MtmiORF171b, c'est-à-dire qu'il entraîne une augmentation de l'accumulation de MtmiR171b et une réduction de l'expression des gènes *HAM1* et *HAM2* (voir Figure 3A), ainsi qu'une réduction du nombre de racines latérales (Figure 3B).

Les résultats de ces expériences démontrent que le MtmiORF171b code un peptide capable de moduler l'accumulation du MtmiR171b, et l'expression des gènes cibles de MtmiR171b : *HAM1* et *HAM2.* Ledit peptide a été dénommé MtmiPEP171b1 (« miPEP » correspondant à *microRNA encoded PEPtide).*

Par ailleurs, le MtPEP171b1 entraîne une augmentation de l'accumulation du MtmiR171b (Figure 4A) et du pre-MtmiR171b (figure 4B).

### b) Spécificité du miPEP171b1

L'expression de différents précurseurs microARNs de *Medicago truncatula* (MtmiR171b SEQ ID NO: 319, MtmiR169 SEQ ID NO: 367, MtmiR169a SEQ ID NO: 368, MtmiR171a SEQ ID NO : 369, MtmiR171h SEQ ID NO : 370, MtmiR393a SEQ ID NO : 371, MtmiR393b SEQ ID NO : 372, MtmiR396a SEQ ID NO : 373 et MtmiR396b SEQ ID NO : 374) a été déterminée et comparée entre des plantes témoins et des plantes dans lesquelles a été surexprimé le MtmiORF171b codant le MtmiPEP171b1 (Figure 5A), ou entre des plantes témoins et des plantes cultivées sur milieu de culture contenant le MtmiPEP171b1 (Figure 5B).

Les résultats obtenus indiquent que le MtmiPEP171b1 n'entraîne une augmentation de l'accumulation que du MtmiR171b et pas des autres miRs, ce qui indique qu'un miPEP n'a d'effet que sur le microARN dont il est issu.

### c) Localisation du miPEP171b1

Par ailleurs, l'immunolocalisation du miPEP171b1 dans les racines de *M. truncatula* révèle la présence du miPEP171b1 dans les sites d'initiation des racines latérales, montrant une co-localisation entre le microARN et le miPEP correspondant (Figures 28 et 34).

### Exemple 1B - MtmiPEP169d

Concernant MtmiPEP169d, il a été démontré *in vivo* chez *M. truncatula* que l'ajout de MtmiPEP169d entraine une accumulation du MtmiR169d (Figure 32).

### Exemple 1C - MtmiPEP171e

Concernant MtmiPEP171e, il a été démontré *in vivo* chez *M. truncatula* que la surexpression de ce miPEP entraine une accumulation du MtmiR171e (Figure 33).

### EXEMPLE 2 - Caractérisation chez la plante modèle du tabac

### a) Conservation du mécanisme chez le tabac

Pour déterminer si le mécanisme de régulation des microARNs est conservé chez d'autres espèces de plantes, la régulation du MtmiR171b par le MtmiPEP171b1 a été testée dans un modèle cellulaire différent. Pour cela, le MtmiR171b et le MtmiPEP171b1 ont été introduits dans des feuilles de tabac.

L'accumulation du MtmiR171b été mesurée chez des feuilles de tabac transformées pour exprimer le MtmiR171b de *Medicago truncatula* à partir du pri-miR sauvage capable de produire le MtmiPEP171b1, ou à partir d'une version mutée du pri-miR incapable de produire le MtmiPEP171b1 (dans laquelle le codon d'initiation ATG du MtmiORF171b a été remplacé ATT) (Figure 6 et Figure 20). L'absence de traduction du MtmiPEP171b1 conduit à une forte diminution de l'accumulation du MtmiR171b.

L'accumulation de pre-MtmiR171b a été mesurée chez des feuilles de tabac transformées pour exprimer le MtmiR171b de *Medicago truncatula* seul (témoin), ou exprimant en plus le MtmiORF171b sauvage de *Medicago truncatula* (35SmiPEP171bl ATG), ou une version mutée de MtmiORF171b dans laquelle le codon d'initiation ATG a été remplacé ATT (35SmiPEP171b1 ATT) (Figure 7 et Figure 21). L'expression de MtmiORF171b entraîne une augmentation de l'accumulation du pre-miR171b, et cette accumulation du pre-miR171b dépend de la traduction du MtmiORF171b en micropeptide.

De même, chez les feuilles de tabac transformées pour exprimer le MtmiR171b de *Medicago truncatula,* traitées ou non par pulvérisation avec le MtmiPEP171b1 (0,1 µM) une première fois 12h avant prélèvement puis une seconde fois 30 minutes avant le prélèvement, il a été observé que le MtmiPEP171b1 peut être utilisé directement sous forme de peptide par le biais de pulvérisations foliaires (Figure 8).

Par ailleurs, il a été observé chez le tabac (comme chez *Medicago truncatula)* que le MtmiPEP171b1 entraîne une augmentation de l'accumulation du mtmiR171b (Figure 9A) et du pre-MtmiR171b (Figure 9B), mais diminue l'accumulation du pri-MtmiR171b (Figure 9C).

L'ensemble de ces résultats indique que le mécanisme de régulation des microARNs et de leurs gènes cibles sous le contrôle de miPEPs est conservé entre les espèces.

### b) Localisation intracellulaire du MtmiPEP171b1

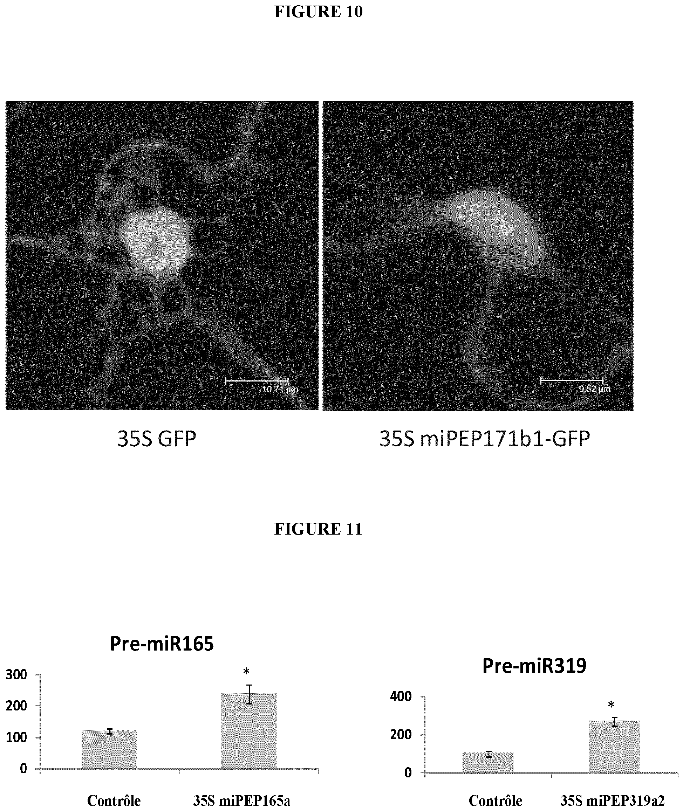

Les feuilles de tabac ont été transformées pour surexprimer le MtmiPEP171b1 de *Medicago truncatula* fusionné à une protéine fluorescente (GFP) (Figure 10). Les résultats obtenus indiquent que le miPEP est localisé dans de petits corps nucléaires.

### c) Identification de miPEPS à partir des bases de données

A partir des bases de données génomiques de plantes, il a été recherché la présence de cadres ouverts de lecture au sein des transcrits primaires de 70 miRs, nous avons identifié 101 miORFs susceptibles de coder un miPEP.

A l'heure actuelle, les AtmiPEP165a et AtmiPEP319a2, identifiés chez *Arabidopsis thaliana,* ont déjà été caractérisés. Les expériences réalisées chez la plante modèle du tabac ont permis de démontrer que la surexpression de AtmiORF165a ou de AtmiORF319a entraîne respectivement une augmentation de l'accumulation du AtmiR165a ou du AtmiR319a (Figure 11).
miR165a régule des facteurs de transcription comme *Revoluta, Phavoluta* et *Phabulosa.* Le miR319 régule des gènes de la famille TCP.

### EXEMPLE 3 - Caractérisation chez la plante modèle Arabidopsis thaliana

### Exemple 3A - AtmiPEP165a

Concernant AtmiPEP165a, il a été démontré *in vivo* chez *Arabidopsis thaliana* que le traitement par le AtmiPEP165a entraine un phénotype de croissance racinaire très accélérée (Figure 12).

Par ailleurs, le traitement des plantes avec des concentrations de plus en plus importantes de miPEP165a indique un effet dose-dépendant de l'accumulation du miR165a et de la régulation négative de ses gènes cibles (PHAVOLUTA : *PHV,* PHABOLUSA : *PHB* et REVOLUTA : *REV*) en fonction de la quantité de miPEP165A (voir figure 22).

### Exemple 3B - AtmiPEP164a

Concernant AtmiPEP164a, celui-ci a été synthétisé et a été utilisé pour rechercher une augmentation de l'accumulation du miR164a chez des racines d'*A*. *thaliana* traitées avec le peptide synthétique.

Les analyses par Northen blot indiquent que le traitement de la plante avec le peptide miPEP164a entraîne une augmentation de l'accumulation du miR164a (Figure 23).

Le même type de résultat a été obtenu par qRT-PCR (Figure 30).

Il a également été démontré *in vivo* chez *Arabidopsis thaliana* que le traitement de la plante avec le AtmiPEP164a augmente significativement la croissance de la plante (Figure 24).

### Exemple 3C - AtmiPEP165a

Concernant AtmiPEP165a, celui-ci a été synthétisé et a été utilisé pour rechercher une augmentation de l'accumulation du miR165a chez des racines d'*A*. *thaliana* traitées avec le peptide synthétique.

Les analyses par Northen blot indiquent que le traitement de la plante avec le peptide miPEP165a entraîne une augmentation de l'accumulation du miR165a (Figure 25).

Par ailleurs, pour déterminer le mode d'action des miPEPs vis-à-vis de l'accumulation de leurs propres miRs, l'expression du pri-miR165a a été analysée chez des plantes sauvages et chez des plantes transformées pour surexprimer le miPEP165a (Figure 35).

L'expression du pri-miR165a est favorisée chez les plantes surexprimant le miPEP165a (Figure 36A).

En présence de cordycepine, un inhibiteur de la synthèse d'ARN, la quantité de pri-miR165a est identique chez les plantes sauvages et chez les plantes transformées (Figure 36B), ce qui indique que le miPEP165a n'agit pas comme un stabilisateur de l'ARN, mais plutôt comme un activateur transcriptionnel du pri-miR165a.

De plus, l'expression du pri-miR165a été analysée chez des plantes sauvages (plantes Col-0) et chez des plantes mutées portant un allèle faible de la seconde grande sous-unité de l'ARN polymerase II (plantes *nrpb2-3*)*.* Les résultats obtenus indiquent que les plantes mutées ne présentent pas d'augmentation de l'accumulation du pri-miR165 en réponse au miPEP165a, contrairement aux plantes sauvages. Ceci semble indiquer que le miPEP agit comme un régulateur transcriptionnel.

### Exemple 3D - AtmiPEP319a1

Concernant AtmiPEP319a1, celui-ci a également été synthétisé et utilisé pour rechercher une augmentation de l'accumulation du miR319a chez des racines d'*A*. *thaliana* traitées avec le peptide synthétique.

Les analyses par qRT-PCRindiquent que la surexpression du AtmiPEP319a1 entraîne une augmentation de l'accumulation du miR319a (Figures 26 et 31).

Il a également été démontré *in vivo* chez *Arabidopsis thaliana* que le traitement de la plante avec le AtmiPEP319a1 augmente significativement la croissance de la plante (Figure 27).

### Exemple 3E - AtmiPEP160b1

Concernant AtmiPEP160b1, il a été démontré *in vivo* chez *A. thaliana* que la surexpression de ce miPEP entraine une accumulation du AtmiR160b (Figure 29).

### Matériels et Méthodes

### Matériel biologique

La surface des graines de *M. truncatula* a été stérilisée et celles-ci ont été mises à germer sur des plaques d'agar pendant 5 jours à 4°C dans l'obscurité. Les jeunes pousses ont ensuite été cultivées sur des plaques carrées de 12 cm remplies de milieu Fahraeus sans nitrogène et contenant du phosphate 7,5 µM (Lauresergues et al., Plant J, 72(3) :512-22, 2012). Les racines latérales ont été dénombrées chaque jour. En pots, les plantes ont été arrosées tous les deux jours avec du milieu Long Ashton modifié contenant peu de phosphore (Balzergue et al., Journal of experimental botany, (62)1049-1060, 2011).

Les peptides ont été synthétisés par Eurogentec ou Smartox-Biotech. Le MtmiPEP171b1 a été remis en suspension dans une solution d'eau 40%/ acétonitrile 50%/ acide acétique 10% (v/v/v), et les autres peptides ont été remis en suspension dans de l'eau.

L'arrosage des feuilles par pulvérisations avec les peptides a été réalisé en utilisant des solutions de peptides à différentes concentrations (0,01, 0,1, 1 µM), une première fois 12h avant prélèvement puis une deuxième fois 30min avant prélèvement

### Transcription inverse des microRNAs

L'ARN a été extrait en utilisant le réactif Tri-Reagent (MRC) selon les instructions du fabricant, à l'exception de la précipitation de l'ARN qui a été réalisée avec 3 volumes d'éthanol. La transcription inverse de l'ARN a été réalisée en utilisant l'amorce spécifique RTprimer171b tige boucle en combinaison avec des hexamères pour effectuer la transcription inverse de l'ARN de haut poids moléculaire.

En bref, 1 µg de RNA a été ajouté à l'amorce tige boucle MIR171b (0,2 µM), l'héxamère (500 ng), le tampon RT (1X), l'enzyme SuperScript Reverse transcriptase (SSIII) (une unité), les dNTPs (0,2 mM chacun), le DTT (0,8 mM) dans un mélange réactionnel final de 25 µl. Pour effectuer la transcription inverse, une réaction de transcription inverse pulsée a été réalisée (40 répétitions du cycle suivant : 16°C pendant 2 minutes, 42°C pendant une minute et 50°C pendant une seconde, suivies d'une inactivation finale de la transcription inverse à 85°C pendant 5 minutes).

### Analyses par RT-PCR quantitative (qRT-PCR)

L'ARN total des racines de *M. truncatula* ou des feuilles de tabac a été extrait en utilisant le kit d'extraction RNeasy Plant Mini Kit (Qiagen). La transcription inverse a été réalisée en utilisant la transcriptase inverse SuperScript II (Invitrogen) à partir de 500 ng d'ARN total. Trois répétitions (n=3) ont été réalisées avec deux répétitions techniques chacune. Chaque expérience a été répétée de deux à trois fois. Les amplifications par qPCR ont été réalisées en utilisant un thermocycleur LightCycler 480 System (Roche Diagnostics) selon la méthode décrite dans Lauressergues et al. (Plant J, 72(3) :512-22, 2012).

### Analyses statistiques

Les valeurs moyennes de l'expression relative des gènes ou de la production de racines latérales ont été analysées en utilisant le test de Student ou le test de Kruskal-Wallis. Les barres d'erreurs représentent l'écart type de la moyenne (SEM, Standard Error of the Mean). Les astérisques indiquent une différence significative (p<0,05).

### Constructions plasmidiques

Les fragments d'ADN d'intérêt ont été amplifiés avec la Pfu polymerase (Promega). Les fragments d'ADN ont été clonés à l'aide des enzymes XhoI etNotI dans un plasmide pPEX-DsRED pour une surexpression sous le contrôle du promoteur fort constitutif 35S, et à l'aide des enzymes KpnI-NcoI dans un plasmide pPEX GUS pour les gènes rapporteurs, selon la méthode décrite dans Combier et al. (Genes & Dev, 22 : 1549-1559, 2008*).*

Pour les miPEPs 165a et 319a, les miORFs correspondants ont été clonés dans pBIN19 selon la méthode décrite dans Combier et al. (Genes & Dev, 22 : 1549-1559, 2008*).*

### Transformation des plantes

Les plantes composites possédant des racines transformées avec *Agrobacterium Rhizogenes* ont été obtenues par la méthode décrite dans Boisson-Dernier et al. (Mol Plant Microbe Internet, 18 : 1269-1276, 2005). Les racines transformées ont été vérifiées et sélectionnées par observations du DsRED avec une loupe binoculaire à fluorescence. Les racines témoins correspondent à des racines transformées avec *A. rhizogenes* ne contenant pas le vecteur pPEX-DsRED. La transformation des feuilles de tabac a été réalisée selon la méthode décrite dans Combier et al. (Genes & Dev, 22 : 1549-1559, 2008*).*

### Northern blot

L'analyse par Northern blot a été réalisée selon le protocole décrit dans Lauressergues et al. Plant J, 72(3) :512-22, 2012.

Les échantillons biologiques ont été homogénéisés dans un tampon contenant 0,1 M de NaCl, 2% de SDS, 50 mM de Tris-HCl (pH 9), 10 mM de EDTA (PH 8) et 20 mM de mercaptoéthanol, et l'ARN a été extrait 2 fois avec un mélange phénol/chloroforme et précipité à l'éthanol.

L'ARN a été chargé sur gel PAGE 15% et transféré sur une membrane de nylon (HybondNX, Amersham). L'ARN a été hybridé avec une sonde d'oligonucléotides, radioactive marquée à son extrémité, pour détecter l'ARN U6 ou pour le miR164a.

Les hybridations ont été réalisées à 55°c. Les signaux d'hybridation ont été quantifiés en utilisant un phophorimager (Fuji) et normalisés avec le signal de la sonde spécifique de l'ARN U6.

### Stabilité de l'ARN

Pour chaque condition, des plantes agées de 2 semaines et cultivées verticalement sur milieu MS solide ont été transférées dans des plaques 6 puits contenant 1 ml de milieu MS liquide. Après 16 heures d'incubation avec 1mM de miPEP, les plantes ont été traitées avec 100 µg/ml de cordycepine ou avec de l'eau (utilisée comme contrôle), et récoltées à différents moments pour extraire et quantifier l'ARN. Chaque expérience a été réalisée 3 fois.

### Marquage histochimiques

Le marquage avec GUS a été réalisée selon la méthode décrite dans Combier et al., (Genes & Dev, 22 : 1549-1559, 2008*).* Les échantillons ont été observés avec un microscope (axiozoom).

### Immunolocalisation

Des racines ou des plantules de tissus de Medicago ont été fixés pendant 2 heures dans 4% de formol (v / v) à 50 mM de tampon phosphate (pH 7,2), puis inclus dans de l'agarose LMP 5% dans de l'eau (à bas point de fusion). Des coupes fines (100 um) ont été obtenues et placées dans du Pbi (tampon phosphate pour l'immunologie) sur des lames coatées avec du téflon, bloquées dans Pbi, 2% Tween et 1% d'albumine de sérum bovin pendant 2 heures (PbiT-BSA), puis marquées épendant une nuit (12 h) à 4 ° C avec l'anticorps primaire dilué dans du BSA-PbiT. Les coupes ont été lavées avec du PBiT et incubées à température ambiante pendant 2 h avec un anticorps secondaire dilué dans PbiT-BSA. Les lames ont ensuite été lavées dans du Pbi pendant 30 min et montées dans du citifluor (milieu de montage). Les anticorps primaires et les dilutions ont été les suivants : 1716a (1 :500, v/v). L'anticorps secondaire était un anticorps de chèvre anti-IgG de lapin couplé à la sonde fluorescente Alexa Fluor 633 (Molecular Probes), et a été utilisé à une dilution de 1 :1000 (v/v).

### Western blot

Un extrait total de protéines a été obtenu selon la méthode décrite dans Combier et al., (Genes & Dev, 22 : 1549-1559, 2008*)* et séparé par SDS-PAGE. Le transfert a été réalisé dans un tampon phosphate pendant une nuit à 4°C, à 15V, puis la membrane a été incubée pendant 45 min à température ambiante dans une solution de glutaraldehyde 0,2% (v/v). Les anticorps primaires ont été utilisés à une dilution 1 : 1 000 (v/v) et des anticorps de chèvres anti-IgG de lapin conjugués à HRP ont été utilisés comme anticorps secondaires à une dilution 1 : 40 000 (v/v).

**Table 8. Liste des amorces**

| | |
|---|---|
| AtmiR160bq5 (SEQ ID NO : 429) | TCCCCAAATTCTTGACCAAA |
| AtmiR160bq3 (SEQ ID NO : 430) | TTGAGGGGAAAACATGAACC |
| AtmiR164aq5 (SEQ ID NO : 431) | ACGAAATCCGTCTCATTTGC |
| AtmiR164aq3 (SEQ ID NO : 432) | GGGTGAAGAGCTCATGTTGG |
| AtmiR165q5 (SEQ ID NO : 433) | TGAGGGGAATGTTGTCTGG |
| AtmiR165q3 (SEQ ID NO : 434) | GAAGCCTGGTCCGAGGATA |
| AtmiR319q5 (SEQ ID NO : 435) | CGAGTCGCCAAAATTCAAAC |
| AtmiR319q3 (SEQ ID NO : 436) | GCTCCCTTCAGTCCAATCAA |
| AtPHVq5 (SEQ ID NO : 437) | GCAACTGCAGTGGAATAGCA |
| AtPHVq3 (SEQ ID NO : 438) | GCGACCTTCATGGGTTCTAA |
| AtPHBq5 (SEQ ID NO : 439) | CTCAGCATCAGCAACGTGAT |
| AtPHBq3 (SEQ ID NO : 440) | AACTCTGCTAGGGCCTCCTC |
| AtREVq5 (SEQ ID NO : 441) | TCACAACTCCTCAGCATTCG |
| AtREVq3 (SEQ ID NO : 442) | ACCCAATCAACAGCAGTTCC |
| Atactine2F (SEQ ID NO : 443) | GGTAACATTGTGCTCAGTGG |
| Atactine2R (SEQ ID NO : 444) | CTCGGCCTTGGAGATCCACA |
| miORF319acla5 (SEQ ID NO : 445) | tcatcgATGAATATACATACATACCATCAT |
| miORF319abam3 (SEQ ID NO : 446) | tccggatccTCTAAAAGCTAAAGTGATTCTA AA |
| AtmiR319cla5 (SEQ ID NO : 447) | tcatcgAGAGAGAGCTTCCTTGAGTC |
| AtmiR319bam3 (SEQ ID NO : 448) | tccggatccAGAGGGAGCTCCCTTCAGT |
| AtmiR165cla5 (SEQ ID NO : 449) | tcatcgGTTGAGGGGAATGTTGTCTG |
| AtmiR165bam3 (SEQ ID NO : 450) | tccggatccGTTGGGGGGGATGAAGCC |
| AtmiR165muorf5cla (SEQ ID NO: 451) | tcatcgATGAGGGTTAAGCTATTTCAGT |
| AtmiR165muorf3bam (SEQ ID NO : 452) | tccggatccTAATATCCTCGATCCAGACAAC |
| miR171bq5 (SEQ ID NO : 453) | CGCCTCAATTTGAATACATGG |
| miR171bq3 (SEQ ID NO : 454) | ACGCGGCTCAATCAAACTAC |
| pri-miR171bq5 (SEQ ID NO : 455) | GCACTAGCTGGTTTCATTATTCC |
| pri-miR171bq3 (SEQ ID NO : 456) | TTGCAAAATTTGGAGAGCCTA |
| miR171eq5 (SEQ ID NO : 457) | AAGCGATGTTGGTGAGGTTC |
| miR171eq3 (SEQ ID NO : 458) | CGGCTCAATCTGAGATCGTAT |
| miR169dq5 (SEQ ID NO : 459) | GGGCGGTGAGATTAACAAAA |
| miR169dq3 (SEQ ID NO : 460) | CCTGCCGGAAATGAAACTAA |
| miORF171e2Cla5 (SEQ ID NO : 461) | tcatcgATGATGGTGTTTGGGAAGCC |
| miORF171e2bam3 (SEQ ID NO : 462) | tccggatccTACATGTAAATCCGTCTTCGG |
| miORF171b5'AGG (SEQ ID NO: 463) | AGGCTTCTTCATAGGCTCTCC |
| miR171e5'Xho (SEQ ID NO : 464) | TCAGTCCGCTCGAGGAATAAGTGAATAT TATCGATATTT |
| miR171e3'Not (SEQ ID NO : 465) | AAGGAAAAAAGCGGCCGCAAAGTGATA TTGGCGCGGCT |
| 171b scramble F bis (SEQ ID NO : 466) | TAGCATGTTATTACACCGCTTAAGTAAG TTCTGTAAGATAGAGCGCGATATTGTTT ACATTAGCTAG |
| 171b scramble R bis (SEQ ID NO : 467) | CGTACTAGCTAATGTAAACAATATCGCG CTCTATCTTACAGAACTTACTTAAGCGG TGTAATAACAT |
| PromiORF171b1 ATG BsaIR (SEQ ID NO : 468) | aaaaGGTCTCgCCATAAGCATAGTGGAAT AATGAAACC |
| PromiORF171b2 ATG BsaIR (SEQ ID NO : 469) | aaaaGGTCTCgCCATTTCCATACCTGAATT TCTCCTT |
| PromiORF171b6 ATG BsaI R (SEQ ID NO : 470) | aaaaGGTCTCgCCATCTTCATCCTCAATAT CCTAATTC |
| 171b fusion GFP sans ATG BsaI R (SEQ ID NO : 471) | aGGTCTCgACACAGATATATATACTATGT CTCTTTCAATTTTGC |
| Pro171b fusionTranscript GUSpPEX BsaI R (SEQ ID NO : 472) | aaaGGTCTCgCCATGACCATTTTCCCACG AATATG |
| Pro171b fusionATG1 GUSpPEXsansATG BsaI R (SEQ ID NO : 473) | aaGGTCTCgACACAAGCATAGTGGAATA ATGAAACC |
| Pro171b fusionTraduc GUSpPEX BsaI R (SEQ ID NO : 474) | aaGGTCTCgACACAGATATATATACTATG TCTCTTTCA |
| 171b NcoI F (SEQ ID NO : 475) | Gaattcctgcccatggttttcg |
| 171b NcoI R (SEQ ID NO : 476) | Cgaaaaccatgggcaggaattc |
| 171b AflII F (SEQ ID NO : 477) | cagtcccgacttaagctcaatac |
| 171b AflII R (SEQ ID NO : 478) | gtattgagcttaagtcgggactg |
| 171b génomiq BsaI F (SEQ ID NO : 479) | aaaGGTCTC C TAGC TTGGTCAAACATACATACAGTAGCACTA G |
| 171b génomiq BsaI R (SEQ ID NO : 480) | aaaGGTCTCgCGTAAAATTGTACTGATTG AATGTAAATTGGTACAC |
| AthmiORF 160b F (SEQ ID NO : 481) | TAGCATGTTTTCCCCTCAATGA |
| AthmiORF 160b R (SEQ ID NO : 482) | cgtaTCATTGAGGGGAAAACAT |
| AthMIR160b BsaI F (SEQ ID NO : 483) | aaaGGTCTCcTAGCACTCATAACTCTCCCC AAATTC |
| AthMIR160b BsaI R (SEQ ID NO : 484) | aaaGGTCTCgCGTAGAAAGAATGTTCGAA AAACAATG |
| pro 165a BsaI F (SEQ ID NO : 485) | aGGTCTCCAAATcatgaagcaggcagtaataacct |
| 165a muORF tagHA BsaI R (SEQ ID NO : 486) | aGGTCTCgCGTActaAGCGTAATCTGGAAC ATCGTATGGGTAtaatatcctcgatccagacaac |
| 165a fusion transcript BsaI R (SEQ ID NO : 487) | aGGTCTCgccatagtggcggagacgaagatg |
| 165a fusion traduc BsaI R (SEQ ID NO : 488) | aGGTCTCgccattaatatcctcgatccagacaac |
| pro 165a BsaI F (SEQ ID NO : 489) | aGGTCTCCAAATtaaactgtcagtgcatggatgt |
| 165a fusion GTG BsaI R (SEQ ID NO : 490) | aGGTCTCgACACattcacaaatttttgttgtagagag |
| 165a fusion GTGmutGTT BsaI R (SEQ ID NO : 491) | aGGTCTCgACACattaacaaatttttgttgtagagag |
| 165a fusion CTG BsaI R (SEQ ID NO : 492) | aGGTCTCgACACtagcagattcacaaatttttgttg |
| 165a fusion ATG BsaI R (SEQ ID NO : 493) | aGGTCTCgACACcctcatgataatcgatcttagca |

### B : Analyse des miPEPs chez l'animal

### EXEMPLE 4 - Identification de candidats miPEPs chez la drosophile

### a) Identification de candidats miPEPs

Une première étude réalisée par RACE-PCR chez l'animal modèle *Drosophila melanogaster* montre l'existence de miRs qui sont exprimés au cours de l'embryogenèse, miR1 et miR8.

Comme chez les plantes, il a été identifié des miORFs dans chacun des deux miRs étudiés. Par exemple, le miR8, connu pour son rôle dans la régulation de la croissance des insectes, présente un miORF codant potentiellement le miPEP8.

Le DmmiR1 (identifié chez *Drosophila melanogaster*) présente quant à lui deux DmmiORFs codant potentiellement le DmmiPEP1a et le DmmiPEP1b.

Une analyse phylogénétique montre la conservation évolutive de la présence des miORFs à travers la douzaine d'espèces de drosophile analysées, c'est à dire depuis leur divergence il y a plus de 60 millions d'années (Figure 13).

Les miPEPs identifiés chez la drosophile présentent par ailleurs plusieurs similitudes avec les miPEPs de plante. Si leur séquence primaire et donc leur taille évoluent rapidement entre espèces, on retrouve une taille réduite (de 32 à 104 aa), ainsi qu'une forte conservation pour une charge globale basique (pHi de 9,5 à 12) (Figure 14).

L'ensemble de ces résultats indiquent donc l'existence de miPEPs régulateurs, codés par les transcrits primaires des microRNAs, à travers un large spectre d'espèces eucaryotes. Ces peptides découverts représentent un réservoir encore inexploré de molécules naturelles, susceptibles de réguler une variété de fonctions biologiques fondamentales, aussi bien chez les végétaux que chez les animaux.

### b) Expression des miPEPS

Des cellules de *Drosphila melanogaster* ont été transfectées pour surexprimer le DmmiPEP8 (à partir de la séquence SEQ ID NO : 494) ou bien le DmmiPEP8 mt dont les codons d'initiation de traduction ont été mutés (à partir de la séquence SEQ ID NO : 495). Comme contrôle de transfection, un plasmide produisant la protéine moesin-RFP a été co-transfecté avec les plasmides codants les DmmiPEP8 et DmmiPEP 8 mt. On constate que les codons initiateurs présents dans la séquence DmmiPEP8 sont fonctionnels puisqu'ils permettent la synthèse d'une protéine de fusion miPEP::GFP visualisée par la GFP alors que les constructions mutées sur les ATG ne produisent quasiment plus de GFP.

Ces résultats suggèrent donc que l'ORF du miPEP est fonctionnel (Figure 38).

### Matériels et Méthodes

### Cellules de Drosophila melanogaster

Les cellules S2 sont cultivées dans un flask T75 dans 12mL de milieu Schneider's (GIBCO), contenant 1% de pénicilline 100U/mL et streptavidine 100mg/mL (Sigma) et 10% de sérum de veau foetal décomplémenté (30' à 56°C).

Les transfections transitoires sont réalisées à l'aide du kit de transfection FuGENE^{®} HD (Roche), selon leurs recommandations. Classiquement, 1,5 millions de cellules S2, préalablement ensemmencées dans des plaques 6 puits (3ml de milieu par puit), sont transfectées avec 250 ng d'ADN plasmidique total. L'ADN est mis en contact avec le Fugene (3 µl) dans 100 µl d'OPTIMEM (GIBCO). Apres 20 minutes, le réactif de transfection formé est mis en contact avec les cellules dans le milieu de culture. L'ARN des cellules est extrait 66h après transfection.

### Clonage des fragments codant DmmiPEP8 et DmmiPEP8 muté

Les fragments d'ADN correspondant aux séquences du DmmiPEP8 (SEQ ID NO : 494) et du DmmiPEP8 muté sur les codons initiateurs (SEQ ID NO : 495) ont été amplifiés par PCR pour être clonés en phase avec la GFP dans un plasmide pUAS. Les cellules d'insecte S2 ont été co-transfectées par un mélange de plasmides (ratio 1/1/1, 300 ng au total) codant le DmmiPEP8::GFP ou sa version mutée DmmiPEP8 mt::GFP, un pACT-GAL4 permettant leur surexpression par le biais du facteur de transcription GAL4 produit sous le contrôle du promoteur actine et d'un vecteur d'expression codant la protéine moesin-RFP utilisée comme témoin de transfection, elle aussi placée sous le contrôle du promoteur actine. 48h post transfection, les cellules ont été fixées par ajout de formaldéhyde dans le milieu de culture 4% (P/V) final pendant 10min. Les cellules ont ensuite été rincées 2X par du PBS1X et montées sur lames pour être observée au microscope (Leica SPE).

**Table 9. Séquences clonées**

| | |
|---|---|
| CGCACACACACACACACACAATCACTCACACGCGGTCACAC GCACATTTCAATAAACTAATGGAGCCTGGCTTTGTTTTTGTT TTATTTCCAACCCACTTGAGCACACAGCACACACAGAGAGA AAAATCAATACTCGTTATGGGATTAAATTTACAAAGCGCAA AGCAAAGCGACAAACAAAATTCAAAAGAAAGAAAAAAAAA CACTCAAATAAACTCACAAAGAATTCCTTATCGCCAAGGGG GCCAATGTTCTAAGGTTCTTTCGCCT | SEQ ID NO: 494 |
| CGCACACACACACACACACAATCACTCACACGCGGTCACAC GCACATTTCAATAAACTAAGTGAGCCTGGCTTTGTTTTTGTT TTATTTCCAACCCACTTAAGCACACAGCACACACAGAGAGA AAAATCAATACTCGTTAGTGGATTAAATTTACAAAGCGCAA | SEQ ID NO: 495 |
| AGCAAAGCGACAAACAAAATTCAAAAGAAAGAAAAAAAAA CACTCAAATAAACTCACAAAGAATTCCTTATCGCCAAGGGG GCCAATGTTCTAAGGTTCTTTCGCCT | |

### C : Caractérisation de miPEPs chez l'homme

### EXEMPLE 5 - Caractérisation du HsmiPEP155

Les fragments d'ADN d'intérêt (le HsmiPEP155 et le miPEP muté) ont été synthétisés ou amplifiés par PCR à l'aide d'amorces spécifiques, puis clonés à l'aide des enzymes XhoI et NotI dans un plasmide pUAS permettant leur surexpression par le biais du facteur de transcription GAL4 dont l'expression est contrôlée par un promoteur fort constitutif.

Les différentes constructions ont été réalisées soit par amplification PCR sur de l'ADN génomique de cellules HeLa, soit par RT-PCR sur des ARN totaux de cellules humaines L428. Les fragments PCR amplifiés sont digérés par les enzymes de restriction HindIII/EcoRI puis clonés dans le vecteur pcDNA3.1. La souche d*'Escherichia coli* DH5α est électroporée puis cultivée sur un milieu solide (2YT+agar+Ampicilline). L'ADN plasmidique de différents clones est alors préparé et séquencé pour vérification. Les constructions sont ensuite préparées à l'aide du QIAfilter Plasmid Midi kit (QIAGEN) et conservées à -20°C.

Les cellules HeLa (lignée tumorale établie, ATCC CCL-2.2) sont cultivées en plaque 6 puits dans du milieu complet [(DMEM (1x) +Glutamax +4.5g/L glucose sans pyruvate + 1x Pénicilline/Streptomycine + 1mM Na-pyruvate + 10% sérum de veau] et placées dans un incubateur à 37°C et 5% de CO2.

Les cellules sont transfectées lorsqu'elles sont à 50% de confluence. En début d'expérience, le milieu complet contenant les antibiotiques est remplacé par du milieu complet sans antibiotique.

Pour chaque puit, un mix A [250µl d'Optimem (+Glutamax) (Gibco) + 2µg d'ADN] et un mix B [250µl d'Optimem + 4µl de Lipofectamine 2000 (Invitrogen)] est préparé, on laisse 5' à température ambiante. Puis le mix B est mélangé dans le mix A goutte à goutte, et laissé à incuber à 25' à température ambiante. Le mélange est alors déposé dans le puit, goutte à goutte. 4-5 heures après, le milieu est changé et remplacé par du milieu complet avec antibiotiques. 48 heures après transfection, les cellules sont arrêtées. Le milieu est aspiré et éliminé, les cellules sont rincées au PBS 1X. Il est alors possible de garder les cellules à - 20°C ou d'extraire directement les ARN totaux.

Pour chaque puit, les ARNs sont extraits en déposant 1ml de Tri-Reagent (Euromedex) sur les cellules. On aspire et refoule plusieurs fois le Tri-reagent afin de lyser correctement les cellules, puis on le transfère dans un tube de 1,5 ml. On rajoute 0,2 ml de chloroforme saturé en eau. On mélange par vortex, puis on laisse 2 à 3 minutes à température ambiante. On centrifuge 5 minutes à 15300rpm et à 4°C. La phase aqueuse est précipitée avec 0,5 ml d'isopropanol après une incubation de 10 minutes à température ambiante et une centrifugation de 15 minutes à 15300rpm et à 4°C. Le surnageant est éliminé et le culot est rincé avec 1ml d'Ethanol 70% et centrifugation de 5 minutes à 15300rpm à 4°C. De nouveau on élimine le surnageant et le culot est séché quelques minutes à l'air.

Afin d'éliminer au mieux l'ADN génomique potentiellement restant, les ARNs sont traités à la DNase. Pour cela, le culot est resuspendu dans 170µl d'eau ultra pure, 20µl de tampon DNase 10x et 10µl de RQ1 RNase free-DNase et placé 30 minutes à 37°C. Puis, on rajoute 20µl de SDS10% et 5µl de protéinase K (20mg/ml) durant 20 minutes à 37°C.

Une dernière extraction phénol est réalisée avec 225 µl d'un mélange Phénol/H₂O/Chloroforme, et centrifugée pendant 5 minutes à 15300 rpm à 4°C.

La phase aqueuse est alors précipitée avec 20µl de Sodium Acetate 3M et 600µl d'Ethanol 100% pendant 20 minutes à -80°C. Puis, on centrifuge 15' à 4°C à 15300rpm. On élimine le surnageant. On rince le culot dans 1ml d'Ethanol 70%, on centrifuge 5' 15300rpm 4°C, on élimine de nouveau le surnageant et on laisse quelques minutes le culot sécher à l'air.

Le culot est ensuite repris dans 15-20 µl d'eau ultra pure et les ARNs sont dosés.

10-15µg d'ARN totaux sont ensuite analysés par Northern Blot sur un gel 15% acrylamide [solution d'acrylamide/bis-acrylamide 40%, ratio 19 :1], 7M Urée en TBE 1x. La migration est réalisée à 400V, dans du TBE1x comme tampon de migration, après avoir pré-chauffé le gel. Les ARN sont ensuite électro-transférés sur une membrane de nylon Biodyne Plus 0.45µm, pendant 2heures, à 1V et 4°C dans une cuve de transfert. A la fin du transfert, la membrane est irradiée aux UV à 0,124 J/cm2. La membrane est ensuite pré-hybridée dans un tampon 5xSSPE, 1xDenhardt's, 1%SDS et 150µg/ml de d'ARNt de levure, 1heure à 50°C dans un four à hybridation. Puis, on rajoute la sonde nucléotidique marquée en 5' au ^{γ}-³²P-ATP (0.5 à 1.10⁶cpm/ml de tampon d'hybridation) et on hybride la nuit à 50°C. La membrane est alors lavée 2 fois dans du 0.1xSSPE/0.1%SDS à température ambiante et exposée dans une cassette d'autoradiographie contenant un écran BioMax HE (Kodak) et un film BioMax MS (Kodak), pour détecter un microARN, durant 24-48 heures, à -80°C.

## Revendications

1. Procédé de détection et d'identification d'un micropeptide (miPEP) codé par une séquence nucléotidique contenue dans la séquence du transcrit primaire d'un microARN (miR), comprenant :
a) une étape de détection d'un cadre ouvert de lecture de 12 à 303 nucléotides contenu dans la séquence du transcrit primaire dudit miR, puis
b) une étape de comparaison entre :
- le phénotype d'une plante exprimant ledit miR,
en présence d'un peptide codé par une séquence nucléotidique identique ou dégénérée par rapport à celle dudit cadre ouvert de lecture, ledit peptide étant présent dans la plante indépendamment de la transcription du transcrit primaire dudit miR, et
- le phénotype d'une même plante exprimant ledit miR en l'absence dudit peptide,
dans lequel, un changement de phénotype de la plante en présence dudit peptide par rapport au phénotype de la plante en absence dudit peptide indique l'existence d'un miPEP codé par ledit cadre ouvert de lecture.

2. Procédé de détection et d'identification d'un miPEP selon la revendication 1, dans lequel le changement de phénotype de ladite plante en présence dudit peptide est la conséquence d'une modulation de l'accumulation dudit miR par ledit peptide.

3. Procédé de détection et d'identification d'un miPEP selon la revendication 2, dans lequel la modulation de l'accululation dudit miRNA est une diminution ou une augmentation de l'accumulation dudit miR, en particulier une augmentation.

4. Procédé de détection et d'identification d'un miPEP selon l'une quelconque des revendications 1 à 3, dans lequel la présence du peptide dans la plante résulte :
- de l'introduction dans la plante d'un acide nucléique codant ledit peptide, ou
- de l'introduction dans la plante dudit peptide.

5. Procédé de détection et d'identification d'un miPEP selon l'une quelconque des revendications 1 à 4, dans lequel ledit cadre ouvert de lecture de l'étape a) est contenu dans la partie 5' ou 3' dudit transcrit primaire du miR, de préférence dans la partie 5'.

6. Procédé de détection et d'identification d'un miPEP selon l'une quelconque des revendications 1 à 5, dans lequel ladite plante est choisie parmi : *Arabidopsis cebennensis, Arabidopsis helleri, Arabidopsis lyrate, Arabidopsis thaliana, Brassica carinata, Brassica juncea, Brassica napus, Brassica oleracea, Brassica rapa, Capsella rubella, Carica papaya, Eucalyptus grandis, Glycine max, Gossypium raimondii, Medicago sativa, Medicago truncatula, Nicotiana benthamiana, Oryza sativa, Physcomitrella patens, Solanum lycopersicum, Solanum melongena, Solanum tuberosum, Thellungiella halophila* et *Zea mays.*

7. Procédé de détection et d'identification d'un miPEP selon l'une quelconque des revendications 1 à 6, dans lequel ledit miR dans ladite plante utilisée à l'étape b) est d'origine endogène.

8. Procédé de détection et d'identification d'un miPEP selon l'une quelconque des revendications 1 à 6, dans lequel ledit miR dans ladite plante utilisée à l'étape b) est d'origine exogène, ladite plante contenant un vecteur permettant l'expression dudit miR.

9. Procédé de détection et d'identification d'un miPEP selon l'une quelconque des revendications 1 à 8, dans lequel le phénotype observé est choisi parmi :
- la taille, la surface, le volume, la masse et le nombre de feuilles ;
- la taille et le nombre de fleurs ;
- la taille de la tige ou de la hampe florale ;
- la longueur et le nombre de racines ;
- la précocité de la germination ;
- la précocité du bourgeonnement ;
- la précocité de l'induction florale ou de la transition florale ;
- encore le nombre de cellules ;
- la résistance au stress hydrique ; et
- la résistance aux maladies infectieuses.

10. Utilisation d'une composition comprenant au moins :
- un miPEP,
- un acide nucléique codant ledit miPEP, ou
- un vecteur contenant ledit acide nucléique,
en tant qu'agent phytopharmaceutique,
- pour favoriser la croissance et/ou le développement des plantes, notamment pour la modulation des paramètres physiologiques d'une plante, en particulier la biomasse, la surface foliaire, la floraison, le calibre du fruit ; ou
- pour prévenir ou traiter les maladies des plantes, en particulier pour favoriser la résistance aux maladies infectieuses,
ledit miPEP ayant :
- une taille de 3 à 100 acides aminés, de préférence 4 à 20 acides aminés ;
- une séquence peptidique identique à celle codée par une séquence nucléotidique contenue dans le transcrit primaire d'un microARN régulant l'expression dudit gène ; et
- étant capable de moduler l'accumulation dudit miR,
ladite composition étant appliquée par pulvérisation foliaire.

11. Utilisation d'une composition selon la revendication 10, dans laquelle lesdites plantes sont choisies parmi : *Arabidopsis cebennensis, Arabidopsis helleri, Arabidopsis lyrate, Arabidopsis thaliana, Brassica carinata, Brassica juncea, Brassica napus, Brassica oleracea, Brassica rapa, Capsella rubella, Carica papaya, Eucalyptus grandis, Glycine max, Gossypium raimondii, Medicago sativa, Medicago truncatula, Nicotiana benthamiana, Oryza sativa, Physcomitrella patens, Solanum lycopersicum, Solanum melongena, Solanum tuberosum, Thellungiella halophila* et *Zea mays.*

12. Utilisation d'une composition selon la revendication 10 ou 11, dans laquelle ledit miR est choisi parmi le groupe de miR consistant en : miR155, miR156a1, miR156a2, miR156a3, miR156c1, miR156c2, miR156e1, miR156f1, miR157c, miR157d, miR159a, miR159a1, miR159b1, miR159b2, miR160a1, miR160b1, miR160b2, miR160c, miR161, miR162a1, miR162b1, miR163-1, miR163-2, miR164a1, miR164a1, miR164a2, miR164a2, miR164a3, miR164b, miR165a, miR166a, miR166b, miR166c, miR166d, miR167a, miR167b1, miR167b2, miR169a, miR169c1, miR169c2, miR169d, miR169h, miR169l1, miR169n, miR170, miR171a1, miR171b, miR171b1, miR171b2, miR171c1, miR171e, miR171h, miR172a1, miR172a3, miR172b1, miR172c1, miR172e1, miR172e2, miR172e3, miR1a, miR1b, miR319a, miR319a, miR319a1, miR319a2, miR319b1, miR394a1, miR395c1, miR395e1, miR396a, miR397b1, miR398c1, miR399b, miR399c, miR399d1, miR403, miR447a1, miR447a2, miR447b1, miR447b2, miR447c et miR8.

13. Utilisation d'une composition selon l'une quelconque des revendications 10 à 12, dans laquelle ledit miR est choisi parmi le groupe de miR consistant en :
- SEQ ID NO : 282 à SEQ ID NO : 352 ;
- SEQ ID NO : 412 à SEQ ID NO: 423 ; et
- SEQ ID NO : 427,
en particulier ledit miR étant choisi parmi le groupe de miR consistant en : SEQ ID NO : 295, SEQ ID NO : 305, SEQ ID NO : 324, SEQ ID NO : 340, SEQ ID NO : 344, SEQ ID NO : 357, SEQ ID NO : 358 et SEQ ID NO : 427.

14. Utilisation d'une composition selon l'une quelconque des revendications 10 à 13, dans laquelle ledit miPEP est choisi parmi le groupe de peptides consistant en :
- SEQ ID NO : 1 à SEQ ID NO : 101 ;
- SEQID NO : 375 à SEQID NO : 386; et
- SEQ ID NO : 424,
en particulier ledit miPEP étant choisi parmi le groupe de peptides consistant en : SEQ ID NO : 14, SEQ ID NO : 24, SEQ ID NO : 43, SEQ ID NO : 59, SEQ ID NO : 63, SEQ ID NO : 76, SEQ ID NO : 77 et SEQ ID NO : 424.

15. Utilisation d'une composition selon l'une quelconque des revendications 10 à 14, dans laquelle ledit acide nucléique codant ledit miPEP est choisi parmi le groupe d'acides nucléiques consistant en :
- SEQID NO : 105 àSEQID NO : 205 ;
- SEQID NO : 387 à SEQID NO : 399; et
- SEQ ID NO : 425,
ledit acide nucléique codant ledit miPEP étant choisi parmi le groupe d'acides nucléiques consistant en : SEQ ID NO : 118, SEQ ID NO : 128, SEQ ID NO : 147, SEQ ID NO : 163, SEQ ID NO : 167, SEQ ID NO : 180, SEQ ID NO : 181 et SEQ ID NO : 425.

## Patentansprüche

1. Verfahren zum Nachweisen und Identifizieren eines Mikropeptids (miPEP), das von einer Nukleotidsequenz kodiert wird, die in der Sequenz des Primärtranskripts einer microRNA enthalten ist, umfassend:
a) einen Schritt zum Nachweisen eines offenen Leserahmens von 12 bis 303 Nukleotiden, der in der Sequenz des Primärtranskripts der genannten microRNA enthalten ist, dann
b) einen Schritt zum Vergleichen:
- der Phänotyp einer Pflanze, die dieses miPEP exprimiert,
in Gegenwart eines Peptids exprimiert, das von einer Nukleotidsequenz kodiert wird, die in Bezug auf diejenige des offenen Leserahmens identisch oder degeneriert ist, wobei das Peptid in der Zelle unabhängig von der Transkription des Primärtranskripts der microRNA vorhanden ist, und
- der Phänotyp der gleichen Pflanze die dieses miPEP in Abwesenheit des Peptids exprimiert,
wobei eine Veränderung des Phänotyps der Pflanze in Gegenwart des Peptids in Bezug auf der Phänotyp der Pflanze in Abwesenheit des Peptids die Existenz eines miPEP anzeigt, das von dem offenen Leserahmen kodiert wird.

2. Verfahren zum Nachweisen und zur Identifizieren eines miPEP nach Anspruch 1, wobei die Veränderung des Phänotyps dieser Pflanze in Gegenwart des Peptids die Folge einer Modulation der Akkumulation dieses miR durch das Peptid ist.

3. Verfahren zum Nachweisen und Identifizieren eines miPEP nach Anspruch 2, wobei die Modulation der Akkumulation der microRNA eine Abnahme oder eine Zunahme der Akkumulation der microRNA, insbesondere eine Zunahme, ist.

4. Verfahren zum Nachweisen und Identifizieren eines miPEP nach einem der Ansprüche 1 bis 3, wobei die Gegenwart des Peptids in der Zelle aus:
- der Einführung einer Nukleinsäure, die das Peptid kodiert, in die Zelle, oder
- der Einführung dieses Peptids in die Zelle resultiert.

5. Verfahren zum Nachweisen und Identifizieren eines miPEP nach einem der Ansprüche 1 bis 4, wobei dieser offene Leserahmen aus Schritt a) in dem 5'- oder 3'-Abschnitt des Primärtranskripts des miR vorzugsweise im 5'-Abschnitt, enthalten ist.

6. Verfahren zum Nachweisen und Identifizieren eines miPEP nach einem der Ansprüche 1 bis 5, wobei die Pflanze ausgewählt wird aus: *Arabidopsis cebennensis, Arabidopsis helleri, Arabidopsis lyrate, Arabidopsis thaliana, Brassica carinata, Brassica juncea, Brassica napus, Brassica oleracea, Brassica rapa, Capsella rubella, Carica papaya, Eucalyptus grandis, Glycine max, Gossypium raimondii, Medicago sativa, Medicago truncatula, Nicotiana benthamiana, Oryza sativa, Physcomitrella patens, Solanum lycopersicum, Solanum melongena, Solanum tuberosum, Thellungiella halophila* und *Zea mays.*

7. Verfahren zum Nachweisen und Identifizieren eines miPEP nach einem der Ansprüche 1 bis 6, wobei das miR in der in Schritt b) verwendeten Pflanze endogenen Ursprungs ist.

8. Verfahren zum Nachweisen und Identifizieren eines miPEP nach einem der Ansprüche 1 bis 6, wobei das miR in der in Schritt b) verwendeten Pflanze enxogenen Ursprungs ist und die Pflanze einen Vektor enthält, der die Expression des miR ermöglicht.

9. Verfahren zum Nachweisen und Identifizieren eines miPEP nach einem der Ansprüche 1 bis 8, wobei der beobachtete Phänotyp ausgewählt wird aus:
- die Größe, die Oberfläche, das Volumen, die Masse und die Anzahl der Blätter;
- die Größe und Anzahl der Blüten;
- die Größe des Stängels oder des Blumenschaftes
- Länge und Anzahl der Wurzeln;
- die Frühzeitigkeit der Keimung;
- die Frühzeitigkeit der Knospung
- die Frühzeitigkeit der Blüteninduktion oder des Blütenübergangs;
- die Anzahl der Zellen;
- die Widerstandsfähigkeit gegen Wasserstress; und
- die Resistenz gegen Infektionskrankheiten.

10. Verwendung einer Zusammensetzung, die mindestens:
- ein miPEP,
- eine Nucleinsäure, die das genannte miPEP codiert, oder
- einen Vektor, der die genannte Nucleinsäure enthält,
als phytopharmazeutisches Mittel,
- zur Förderung des Wachstums und/oder der Entwicklung von Pflanzen, insbesondere zur Modulation der physiologischen Parameter einer Pflanze, insbesondere der Biomasse, der Blattfläche, das Blühen, der Größe der Früchte; oder
- zum Verhindern oder Behandeln von Pflanzenkrankheiten, insbesondere zur Förderung der Resistenz gegen Infektionskrankheiten,
wobei das genannte miPEP aufweist:
- eine Größe von 3 bis 100 Aminosäuren, vorzugsweise 4 bis 20 Aminosäuren;
- eine Peptidsequenz, die identisch ist mit jener, welche durch eine Nucleotidsequenz codiert wird, die in dem Primärtranskript einer microRNA enthalten ist, welche die Expression des genannten Gens reguliert, und in der Lage ist, die Akkumulation der genannten microRNA zu modulieren,
wobei die Zusammensetzung durch Blattsprühen angewendet wird.

11. Verwendung einer Zusammensetzung nach Anspruch 10, wobei die Pflanzen ausgewählt werden aus: *Arabidopsis cebennensis, Arabidopsis helleri, Arabidopsis lyrate, Arabidopsis thaliana, Brassica carinata, Brassica juncea, Brassica napus, Brassica oleracea, Brassica rapa, Capsella rubella, Carica papaya, Eucalyptus grandis, Glycine max, Gossypium raimondii, Medicago sativa, Medicago truncatula, Nicotiana benthamiana, Oryza sativa, Physcomitrella patens, Solanum lycopersicum, Solanum melongena, Solanum tuberosum, Thellungiella halophila* und *Zea mays.*

12. Verwendung einer Zusammensetzung nach Anspruch 10 oder 11, wobei das miR ausgewählt ist aus der Gruppe der miRs, bestehend aus: miR155, miR156a1, miR156a2, miR156a3, miR156c1, miR156c2, miR156e1, miR156f1, miR157c, miR157d, miR159a, miR159a1, miR159b1, miR159b2, miR160a1, miR160b1, miR160b2, miR160c, miR161, miR162a1, miR162b1, miR163-1, miR163-2, miR164a1, miR164a1, miR164a2, miR164a2, miR164a3, miR164b, miR165a, miR166a, miR166b, miR166c, miR166d, miR167a, miR167b1, miR167b2, miR169a, miR169c1, miR169c2, miR169d, miR169h, miR169l1, miR169n, miR170, miR171a1, miR171b, miR171b1, miR171b2, miR171c1, miR171e, miR171h, miR172a1, miR172a3, miR172b1, miR172c1, miR172e1, miR172e2, miR172e3, miR1a, miR1b, miR319a, miR319a, miR319a1, miR319a2, miR319b1, miR394a1, miR395c1, miR395e1, miR396a, miR397b1, miR398c1, miR399b, miR399c, miR399d1, miR403, miR447a1, miR447a2, miR447b1, miR447b2, miR447c und miR8.

13. Verwendung einer Zusammensetzung nach einem der Ansprüche 10 bis 12, wobei das miR ausgewählt ist aus der Gruppe der miRs, bestehend aus:
- SEQ ID NO: 282 to SEQ ID NO: 352;
- SEQ ID NO: 412 to SEQ ID NO: 423; und
- SEQ ID NO: 427,
wobei das miR insbesondere aus der Gruppe der miRs ausgewählt wird, die aus folgenden besteht: SEQ ID NO: 295, SEQ ID NO: 305, SEQ ID NO: 324, SEQ ID NO: 340, SEQ ID NO: 344, SEQ ID NO: 357, SEQ ID NO: 358 und SEQ ID NO: 427.

14. Verwendung einer Zusammensetzung nach einem der Ansprüche 10 bis 13, wobei das miPEP ausgewählt ist aus der Gruppe der Peptide, bestehend aus:
- SEQ ID NO: 1 to SEQ ID NO: 101;
- SEQ ID NO: 375 to SEQ ID NO: 386; und
- SEQ ID NO: 424,
wobei das miPEP insbesondere aus der Gruppe der Peptide ausgewählt wird, die aus folgenden besteht: SEQ ID NO: 14, SEQ ID NO: 24, SEQ ID NO: 43, SEQ ID NO: 59, SEQ ID NO: 63, SEQ ID NO: 76, SEQ ID NO: 77 und SEQ ID NO: 424.

15. Verwendung einer Zusammensetzung nach einem der Ansprüche 10 bis 14, wobei die Nukleinsäure, die für das miPEP kodiert, ausgewählt ist aus der Gruppe der Nukleinsäuren, bestehend aus:
- SEQ ID NO: 105 to SEQ ID NO: 205;
- SEQ ID NO: 387 to SEQ ID NO: 399; und
- SEQ ID NO: 425,
wobei die Nukleinsäure, die für das miPEP kodiert, ausgewählt ist aus der Gruppe der Nukleinsäuren, die aus folgenden besteht: SEQ ID NO: 118, SEQ ID NO: 128, SEQ ID NO: 147, SEQ ID NO: 163, SEQ ID NO: 167, SEQ ID NO: 180, SEQ ID NO: 181 und SEQ ID NO: 425.

## Claims

1. Process for detecting and identifying a micropeptide (miPEP) encoded by a nucleotide sequence contained in the sequence of the primary transcript of a microRNA, comprising:
a) a step of detecting an open reading frame from 12 to 303 nucleotides contained in the sequence of the primary transcript of said microRNA, then
b) a step of comparison between:
- the phenotype of a plant expressing said miPEP, in the presence of a peptide encoded by a nucleotide sequence that is identical or degenerate with respect to that of said open reading frame, said peptide being present in the plant independently of transcription of the primary transcript of said microRNA, and
- the phenotype of the same plant le phénotype expressing said miPEP in the absence of said peptide,
wherein, a change in the phenotype of the plant in the presence of said peptide compared to the phenotype of the plant in the absence of said peptide indicates the existence of a miPEP encoded by said open reading frame.

2. Process for detecting and identifying a miPEP according to claim 1, wherein the change in phenotype of said plant in the presence of said peptide is the consequence of a modulation of the accumulation of said miR by said peptide.

3. Process for detecting and identifying a miPEP according to claim 2, wherein the modulation of the accumulation of said microRNA is a decrease or an increase in the accumulation of said microRNA, in particular an increase.

4. Process for detecting and identifying a miPEP according to any one of claims 1 to 3, wherein the presence of the peptide in the cell results:
- from the introduction into the cell of a nucleic acid encoding said peptide, or
- from the introduction into the cell of said peptide.

5. Process for detecting and identifying a miPEP according to any one of claims 1 to 4, wherein said open reading frame in step a) is contained in the 5' or 3' portion of said primary transcript of the microRNA, preferably in the 5' portion.

6. Process for detecting and identifying a miPEP according to any one of claims 1 to 5, wherein said plant is chosen among: *Arabidopsis cebennensis, Arabidopsis helleri, Arabidopsis lyrate, Arabidopsis thaliana, Brassica carinata, Brassica juncea, Brassica napus, Brassica oleracea, Brassica rapa, Capsella rubella, Carica papaya, Eucalyptus grandis, Glycine max, Gossypium raimondii, Medicago sativa, Medicago truncatula, Nicotiana benthamiana, Oryza sativa, Physcomitrella patens, Solanum lycopersicum, Solanum melongena, Solanum tuberosum, Thellungiella halophila* and *Zea mays.*

7. Process for detecting and identifying a miPEP according to any one of claims 1 to 6, wherein said miR in said plant used in step b) is of endogenous origin.

8. Process for detecting and identifying a miPEP according to any one of claims 1 to 6, wherein said miR in said plant used in step b) is of enxogenous origin, said plant containing a vector enabling expression of said miR.

9. Process for detecting and identifying a miPEP according to any one of claims 1 to 8, wherein the observed phenotype is chosen among:
- the size, surface area, volume, mass and the number of leaves;
- the size and number of flowers;
- the size of the stem or spike;
- the length and number of roots;
- the earliness of germination;
- the earliness of budding;
- the earliness of floral induction or floral transition;
- the number of cells;
- the resistance to water stress; and
- the resistance to infectious diseases.

10. Use of a composition comprising at least:
- a miPEP,
- a nucleic acid encoding said miPEP, or
- a vector containing said nucleic acid,
as a phytopharmaceutical agent,
- for promoting the growth and/or development of plants, in particular for modulating the physiological parameters of a plant, in particular the biomass, foliar surface area, flowering, fruit size; or
- for preventing or treating plant diseases, in particular for promoting resistance to infectious diseases,
said miPEP having:
- a size from 3 to 100 amino acids, preferably 4 to 20 amino acids;
- a peptide sequence identical to that encoded by a nucleotide sequence contained in the primary transcript of a microRNA regulating the expression of said gene, and
- being capable of modulating the accumulation of said microRNA,
said composition being applied by foliar spraying.

11. Use of a composition according to claim 10, wherein said plants are chosen among: *Arabidopsis cebennensis, Arabidopsis helleri, Arabidopsis lyrate, Arabidopsis thaliana, Brassica carinata, Brassica juncea, Brassica napus, Brassica oleracea, Brassica rapa, Capsella rubella, Carica papaya, Eucalyptus grandis, Glycine max, Gossypium raimondii, Medicago sativa, Medicago truncatula, Nicotiana benthamiana, Oryza sativa, Physcomitrella patens, Solanum lycopersicum, Solanum melongena, Solanum tuberosum, Thellungiella halophila* and *Zea mays.*

12. Use of a composition according to claim 10 or 11, wherein said miR is selected from the group of miRs consisting of: miR155, miR156a1, miR156a2, miR156a3, miR156c1, miR156c2, miR156e1, miR156f1, miR157c, miR157d, miR159a, miR159a1, miR159b1, miR159b2, miR160a1, miR160b1, miR160b2, miR160c, miR161, miR162a1, miR162b1, miR163-1, miR163-2, miR164a1, miR164a1, miR164a2, miR164a2, miR164a3, miR164b, miR165a, miR166a, miR166b, miR166c, miR166d, miR167a, miR167b1, miR167b2, miR169a, miR169c1, miR169c2, miR169d, miR169h, miR169l1, miR169n, miR170, miR171a1, miR171b, miR171b1, miR171b2, miR171c1, miR171e, miR171h, miR172a1, miR172a3, miR172b1, miR172c1, miR172e1, miR172e2, miR172e3, mirla, miR1b, miR319a, miR319a, miR319a1, miR319a2, miR319b1, miR394a1, miR395c1, miR395e1, miR396a, miR397b1, miR398c1, miR399b, miR399c, miR399d1, miR403, miR447a1, miR447a2, miR447b1, miR447b2, miR447c and miR8.

13. Use of a composition according to any one of claims 10 to 13, wherein said miR is selected from the group of miRs consisting of:
- SEQ ID NO: 282 to SEQ ID NO: 352;
- SEQ ID NO: 412 to SEQ ID NO: 423; and
- SEQ ID NO: 427,
in particular said miR being selected from the group of miRs consisting of: SEQ ID NO: 295, SEQ ID NO: 305, SEQ ID NO: 324, SEQ ID NO: 340, SEQ ID NO: 344, SEQ ID NO: 357, SEQ ID NO: 358 and SEQ ID NO: 427.

14. Use of a composition according to any one of claims 10 to 14, wherein said miPEP is selected from the group of peptides consisting of:
- SEQ ID NO: 1 to SEQ ID NO: 101;
- SEQ ID NO: 375 to SEQ ID NO: 386; and
- SEQ ID NO: 424,
in particular said miPEP being selected from the group of peptides consisting of: SEQ ID NO: 14, SEQ ID NO: 24, SEQ ID NO: 43, SEQ ID NO: 59, SEQ ID NO: 63, SEQ ID NO: 76, SEQ ID NO: 77 and SEQ ID NO: 424.

15. Use of a composition according to any one of claims 10 to 15, wherein said nucleic acid encoding said miPEP is selected from the group of nucleic acids consisting of:
- SEQ ID NO: 105 to SEQ ID NO: 205;
- SEQ ID NO: 387 to SEQ ID NO: 399; and
- SEQ ID NO: 425,
said nucleic acid encoding said miPEP being selected from the group of nucleic acids consisting of: SEQ ID NO: 118, SEQ ID NO: 128, SEQ ID NO: 147, SEQ ID NO: 163, SEQ ID NO: 167, SEQ ID NO: 180, SEQ ID NO: 181 and SEQ ID NO: 425.
